# EUROPÄISCHE PATENTSCHRIFT

(11) **EP 3 027 614 B1**
(45) Veröffentlichungstag und Bekanntmachung des Hinweises auf die Patenterteilung: **23.08.2017**
(21) Anmeldenummer: 14744029.1
(22) Anmeldetag: 21.07.2014
(51) Int. Cl.: C07D 471/04, A61K 31/4985, A61P 35/00

(54) **SUBSTITUIERTE DIHYDROPYRIDO[3,4-B]PYRAZINONE ALS DUALE INHIBITOREN VON BET-PROTEINEN UND POLO-LIKE KINASEN**
SUBSTITUTED DIHYDROPYRIDO[3,4-B]PYRAZINONES AS DUAL INHIBITORS OF BET-PROTEINS AND POLO-LIKE KINASES
DIHYDROPYRIDO[3,4-B]PYRAZINONES SUBSTITUÉES COMME DOUBLE INHIBITEURS DE LA PROTÉINES BET ET POLO-LIKE KINASES

(30) Priorität: 23.07.2013 EP 13177539
(43) Veröffentlichungstag der Anmeldung: 08.06.2016
(73) Patentinhaber: Bayer Pharma Aktiengesellschaft, 13353 Berlin (DE)
(72) Erfinder: SCHMEES, Norbert, 13469 Berlin (DE); BADER, Benjamin, 13467 Berlin (DE); HAENDLER, Bernard, 13465 Berlin (DE); SCHULZE, Volker, 16562 Hohen Neuendorf (DE); HARTUNG, Ingo, 13158 Berlin (DE); BÖHNKE, Niels, 10713 Berlin (DE); PÜHLER, Florian, Wellesley, Massachusetts 02148 (US)
(74) Vertreter: BIP Patents
(86) Internationale Anmeldenummer: PCT/EP2014/065605
(87) Internationale Veröffentlichungsnummer: WO 2015/011084

(56) Entgegenhaltungen:
- WO-A1-2006/021548
- WO-A1-2012/075456
- US-A1- 2006 009 457

## Beschreibung

Die vorliegende Erfindung betrifft substituerte Dihydropyrido[3,4-*b*]pyrazinone als duale Inhibitoren von BET-Proteinen, insbesondere BRD4-Proteinen und Polo-like Kinasen, insbesondere Plk-1-Proteine, Intermediate zur Herstellung der erfindungsgemäßen Verbindungen, pharmazeutische Mittel enthaltend die erfindungsgemäßen Verbindungen sowie deren prophylaktische und therapeutische Verwendung bei hyper-proliferativen Erkrankungen, insbesondere bei Tumorerkrankungen. Desweiteren betrifft diese Erfindung die Verwendung der erfindungsgemäßen Dihydropyrido[3,4-*b*]pyrazinone in viralen Infektionen, in neurodegenerativen Erkrankungen, in inflammatorischen Krankheiten, in atherosklerotischen Erkrankungen und in der männlichen Fertilitätskontrolle.

Die humane BET-Familie (bromodomain and extra C-terminal domain family) hat vier Mitglieder (BRD2, BRD3, BRD4 und BRDT), die zwei verwandte Bromodomänen und eine extraterminale Domäne enthalten (Wu und Chiang, J. Biol. Chem., 2007, 282:13141-13145). Die Bromodomänen sind Proteinregionen, die acetylierte Lysinreste erkennen. Solche acetylierten Lysine findet man oft am N-terminalen Ende von Histonen (z. B. Histon 3 oder Histon 4) und sind Merkmale für eine offene Chromatin-Struktur und aktive Gentranskription (Kuo und Allis, Bioessays, 1998, 20:615-626). Die verschiedenen Acetylierungsmuster, die durch BET Proteine in Histonen erkannt werden, wurden genau untersucht (Umehara et al., J. Biol. Chem., 2010, 285:7610-7618; Filippakopoulos et al., Cell, 2012, 149:214-231). Zusätzlich können Bromodomänen weitere acetylierte Proteine erkennen. Zum Beispiel bindet BRD4 an RelA, was zur Stimulierung von NF-κB und transkriptioneller Aktivität von inflammatorischen Genen führt (Huang et al., Mol. Cell. Biol., 2009, 29:1375-1387; Zhang et al., J. Biol. Chem., 2012, 287: 28840-28851; Zou et al., Oncogene, 2013, doi:10.1038/onc.2013.179). BRD4 bindet auch an Cyclin T1 und bildet einen aktiven Komplex, der für die Transkriptionselongation wichtig ist (Schröder et al., J. Biol. Chem., 2012, 287:1090-1099). Die extraterminale Domäne von BRD2, BRD3 und BRD4 interagiert mit mehreren Proteinen, die eine Rolle in der Chromatinmodulierung und der Regulation der Genexpression haben (Rahman et al., Mol. Cell. Biol., 2011, 31:2641-2652).
Mechanistisch spielen BET-Proteine eine wichtige Rolle im Zellwachstum und im Zellzyklus. Sie sind mit mitotischen Chromosomen assoziiert, was eine Rolle im epigenetischen Gedächtnis nahelegt (Dey et al., Mol. Biol. Cell, 2009, 20:4899-4909; Yang et al., Mol. Cell. Biol., 2008, 28:967-976). Eine Rolle von BRD4 in der post-mitotischen Reaktivierung der Gentranskription wurde nachgewiesen (Zhao et al., Nat. Cell. Biol., 2011, 13:1295-1304). BRD4 ist essentiell für die Transkriptionselongation und rekrutiert den Elongationskomplex P-TEFb, der aus CDK9 und Cyclin T1 besteht, was zur Aktivierung der RNA Polymerase II führt (Yang et al., Mol. Cell, 2005, 19:535-545; Schröder et al., J. Biol. Chem., 2012, 287:1090-1099). Folglich wird die Expression von Genen stimuliert, die in der Zellproliferation involviert sind, wie zum Beispiel c-Myc, Cyclin D1 und Aurora B (You et al., Mol. Cell. Biol., 2009, 29:5094-5103; Zuber et al., Nature, 2011, doi:10.1038). BRD2 ist in der Regulation von Targetgene des Androgenrezeptors beteiligt (Draker et al., PLOS Genetics, 2012, 8, e1003047). BRD2 und BRD3 binden an transkribierte Gene in hyperacetylierten Chromatinbereichen und fördern die Transkription durch RNA Polymerase II (LeRoy et al., Mol. Cell, 2008, 30:51-60).

Der Knock-down von BRD4 bzw. die Hemmung der Interaktion mit acetylierten Histonen in verschiedenen Zelllinien führt zu einem G1-Arrest (Mochizuki et al., J. Biol. Chem., 2008, 283:9040-9048; Mertz et al., Proc. Natl. Acad. Sci. USA, 2011, 108:16669-16674). Es wurde auch gezeigt, dass BRD4 an Promotorregionen von mehreren Genen, die in der G1-Phase aktiviert werden wie zum Beispiel Cyclin D1 und D2, bindet (Mochizuki et al., J. Biol. Chem., 2008, 283:9040-9048). Zusätzlich wurde eine Hemmung der Expression von c-Myc, ein essentieller Faktor in der Zellproliferation, nach BRD4-Inhibition nachgewiesen (Dawson et al., Nature, 2011, 478:529-533; Delmore et al., Cell, 2011, 146:1-14; Mertz et al., Proc. Natl. Acad. Sci. USA, 2011, 108:16669-16674). Eine Hemmung der Expression von androgenregulierten Genen und eine Bindung von BRD2 an entsprechende regulatorischen Regionen wurde auch nachgewiesen (Draker et al., PLOS Genetics, 2012, 8, e1003047).

BRD2 und BRD4 Knockout-Mäuse sterben früh während der Embryogenese (Gyuris et al., Biochim. Biophys. Acta, 2009, 1789:413-421; Houzelstein et al., Mol. Cell. Biol., 2002, 22:3794-3802). Heterozygote BRD4 Mäuse haben verschiedene Wachstumsdefekte, die auf eine reduzierte Zellproliferation zurückzuführen sind (Houzelstein et al., Mol. Cell. Biol., 2002, 22:3794-3802). BET-Proteine spielen eine wichtige Rolle in verschiedenen Tumorarten. Die Fusion zwischen den BET-Proteinen BRD3 oder BRD4 und NUT, einem Protein, das normalerweise nur im Hoden exprimiert wird, führt zu einer aggressiven Form des Plattenepithelkarzinoms, genannt NUT midline carcinoma (French, Cancer Genet. Cytogenet., 2010, 203:16-20). Das Fusionsprotein verhindert Zelldifferenzierung und fördert Proliferation (Yan et al., J. Biol. Chem., 2011, 286:27663-27675, Grayson et al., 2013, doi:10-1038/onc.2013.126). Das Wachstum von davon abgeleiteten *in vivo* Modellen wird durch einen BRD4-Inhibitor gehemmt (Filippakopoulos et al., Nature, 2010, 468:1067-1073). Ein Screening für therapeutische Targets in einer akuten myeloiden Leukämiezelllinie (AML) zeigte, dass BRD4 eine wichtige Rolle in diesem Tumor spielt (Zuber et al., Nature, 2011, 478, 524-528). Die Reduktion der BRD4-Expression führt zu einem selektiven Arrest des Zellzyklus und zur Apoptose. Die Behandlung mit einem BRD4-Hemmer verhindert die Proliferation eines AML-Xenografts *in vivo.* Weitere Versuche mit einem BRD4-Hemmer zeigen, dass BRD4 eine Rolle in verschiedenen hämatologischen Tumoren spielt, wie zum Beispiel Multiples Myelom (Delmore et al., Cell, 2011, 146, 904-917) und Burkitt's Lymphom (Mertz et al., Proc. Natl. Acad. Sci. USA, 2011, 108, 16669-16674). Auch in soliden Tumoren, wie zum Beispiel Lungenkrebs spielt BRD4 eine wichtige Rolle (Lockwood et al., Proc. Natl. Acad. Sci. USA, 2012, 109, 19408-19413). Eine erhöhte Expression von BRD4 wurde im Multiplen Myelom festgestellt, und auch eine Amplifizierung des BRD4-Gens wurde in Patienten mit Multiplem Myelom festgestellt (Delmore et al., Cell, 2011, 146, 904-917). Eine Amplifizierung der DNA-Region, die das BRD4-Gen enthält, wurde in primären Brusttumoren nachgewiesen (Kadota et al., Cancer Res, 2009, 69:7357-7365). Auch für BRD2 gibt es Daten bezüglich einer Rolle in Tumoren. Eine transgene Maus, die BRD2 selektiv in B-Zellen hochexprimiert, entwickelt B-Zell Lymphome und Leukämien (Greenwall et al., Blood, 2005, 103:1475-1484).

BET-Proteine sind auch an viralen Infektionen beteiligt. BRD4 bindet an das E2 Protein von verschiedenen Papillomaviren und ist wichtig für das Überleben der Viren in latent infizierten Zellen (Wu et al., Genes Dev., 2006, 20:2383-2396; Vosa et al., J. Virol., 2006, 80:8909-8919). Auch das Herpesvirus, das für das Kaposi-Sarkom verantwortlich ist, interagiert mit verschiedenen BET-Proteinen, was für die Krankheitsbeständigkeit wichtig ist (Viejo-Borbolla et al., J. Virol., 2005, 79:13618-13629; You et al., J. Virol., 2006, 80:8909-8919). Durch Bindung an P-TEFb spielt BRD4 auch eine wichtige Rolle in der Replikation von HIV-1 (Bisgrove et al., Proc. Natl Acad. Sci. USA, 2007, 104:13690-13695). Die Behandlung mit einem BRD4-Hemmer führt zu einer Stimulierung des ruhenden, nicht behandelbaren Reservoirs von HIV-1 Viren in T-Zellen (Banerjee et al., J. Leukoc. Biol., 2012, 92, 1147-1154). Diese Reaktivierung könnte neue Therapiewege für AIDS-Behandlung ermöglichen (Zinchenko et al., J. Leukoc. Biol., 2012, 92, 1127-1129). Eine kritische Rolle von BRD4 in der DNA Replikation von Polyomaviren wurde auch berichtet (Wang et al., PLoS Pathog., 2012, 8, doi:10.1371).

BET-Proteine sind zusätzlich an Inflammationsprozessen beteiligt. BRD2-hypomorphe Mäuse zeigen eine reduzierte Inflammation im Fettgewebe (Wang et al., Biochem. J., 2009, 425:71-83). Auch die Infiltration von Makrophagen in weißem Fettgewebe ist in BRD2-defizienten Mäusen reduziert (Wang et al., Biochem. J., 2009, 425:71-83). Es wurde auch gezeigt, dass BRD4 eine Reihe von Genen reguliert, die in der Inflammation involviert sind. In LPS-stimulierten Makrophagen verhindert ein BRD4-Inhibitor die Expression von inflammatorischen Genen, wie zum Beispiel IL-1 oder IL-6 (Nicodeme et al., Nature, 2010, 468:1119-1123).

BET-Proteine sind auch in der Regulierung des ApoA1-Gens involviert (Mirguet et al., Bioorg. Med. Chem. Lett., 2012, 22:2963-2967). Das entsprechende Protein ist Bestandteil des Lipoproteins höherer Dichte (HDL), das bei Atherosklerose eine wichtige Rolle spielt (Smith, Arterioscler. Thromb. Vasc. Biol., 2010, 30:151-155). Durch die Stimulierung der ApoA1-Expression, können BET-Proteininhibitoren die Konzentrationen an Cholesterin HDL erhöhen und somit für die Behandlung von Atherosklerose potentiell nützlich sein (Mirguet el al., Bioorg. Med. Chem. Lett., 2012, 22:2963-2967).

Das BET-Protein BRDT spielt eine wesentliche Rolle in der Spermatogenese durch die Regulierung der Expression mehreren Genen, die während und nach der Meiose wichtig sind (Shang et al., Development, 2007, 134:3507-3515; Matzuk et al., Cell, 2012, 150:673-684). Desweiteren ist BRDT in der post-meiotischen Organisation des Chromatins involviert (Dhar et al., J. Biol. Chem., 2012, 287:6387-6405). *In vivo* Versuche in der Maus zeigen, dass die Behandlung mit einem BET-Hemmer, der auch BRDT inhibiert, zu einer Abnahme der Spermienproduktion und Infertilität führt (Matzuk et al., Cell, 2012, 150:673-684).

Alle diese Untersuchungen zeigen, dass die BET-Proteine eine essentielle Rolle in verschiedenen Pathologien und auch in der männlichen Fertilität spielen. Es wäre deshalb wünschenswert, potente und selektive Inhibitoren zu finden, die die Interaktion zwischen den BET-Proteinen und acetylierten Proteinen verhindern. Diese neuen Inhibitoren sollten auch geeignete pharmakokinetische Eigenschaften haben, die es erlauben *in vivo,* also im Patienten, diese Interaktionen zu hemmen.

Tumorzellen zeichnen sich weiterhin durch einen ungehemmten Zell-Zyklus-Prozess aus. Dies beruht einerseits auf dem Verlust von Kontroll-Proteinen wie RB, p16, p21, p53 *etc.* sowie der Aktivierung von sog. Beschleunigern des Zell-Zyklus-Prozesses, den cyclin-abhängigen Kinasen (CDKs). Die CDKs sind ein in der Pharmazie anerkanntes Anti-Tumor Ziel-Protein. Neben den CDKs wurden neue Zell-Zyklus regulierende Serin/Threonin-Kinasen, sogenannte 'Polo-like Kinasen' beschrieben, die nicht nur bei der Regulation des Zell-Zyklus sondern auch an der Koordination mit anderen Vorgängen während der Mitose und Zytokinese (Ausbildung des Spindelapparates, Chromosomentrennung) beteiligt sind. Daher stellt diese Klasse von Proteinen einen interessanten Angriffspunkt zur therapeutischen Intervention proliferativer Krankheiten wie Krebs dar (Descombes und Nigg. Embo J, 17; 1328ff, 1998; Glover et al. Genes Dev 12, 3777ff, 1998).

Eine hohe Expressionsrate von Plk-1 wurde im 'non-small cell lung'-Krebs (Wolf et al. Oncogene, 14, 543ff, 1997), in Melanomen (Strebhardt et al. JAMA, 283, 479ff, 2000), in 'squamous cell carcinomas' (Knecht et al. Cancer Res, 59, 2794ff, 1999) und in 'esophageal carcinomas '(Tokumitsu et al. Int J Oncol 15, 687ff, 1999) gefunden.
Eine Korrelation von hoher Expressionsrate in Tumorpatienten mit schlechter Prognose wurde für verschiedenste Tumore gezeigt (Strebhardt et al. JAMA, 283, 479ff, 2000, Knecht et al. Cancer Res, 59, 2794ff, 1999 und Tokumitsu et al. Int J Oncol 15, 687ff, 1999).

Konstitutive Expression von Plk-1 in NIH-3T3-Zellen führte zu einer malignen Transformation (erhöhte Proliferation, Wachstum in Soft-Agar, Kolonie-Bildung und Tumor-Entwicklung in Nacktmäusen (Smith et al. Biochem Biophys Res Comm, 234, 397ff., 1997).

Mikroinjektionen von Plk-1-Antikörpern in HeLa-Zellen führte zu fehlerhafter Mitose (Lane et al.; Journal Cell Biol, 135, 1701ff, 1996).

Mit einem '20-mer' Antisense Oligo konnte die Expression von Plk-1 in A549-Zellen inhibiert und deren Überlebensfähigkeit gestoppt werden. Ebenso konnte eine deutliche Anti-Tumor-Wirkung in Nacktmäusen gezeigt werden (Mundt et al., Biochem Biophys Res Comm, 269, 377ff., 2000).

Die Mikroinjektion von Anti-Plk-1-Antikörpern in nichtimmortalisierte humane Hs68-Zellen, zeigte im Vergleich zu HeLa-Zellen eine deutlich höhere Fraktion von Zellen, welche in einer Wachstumsarretierung an G2 verblieben waren und weit weniger Anzeichen von fehlerhafter Mitose zeigten (Lane et al.; Journal Cell Biol, 135, 1701ff, 1996).

Im Gegensatz zu dem Wachstum von Tumor-Zellen inhibierten Antisense-Oligo-Moleküle das Wachstum und die Viabilität von primären humanen mesangialen Zellen nicht (Mundt et al., Biochem Biophys Res Comm, 269, 377ff., 2000).

In Säugern wurden bislang neben der Plk-1 drei weitere Polo-Kinasen beschrieben, die als mitogene Antwort induziert werden und ihre Funktion in der G1-Phase des Zell-Zykluses ausüben. Dies sind zum einen die sog Prk/Plk-3 (das humane Homolog der Maus-Fnk= Fibroblast growth factor induced kinase; Wiest et al., Genes, Chromosomes & Cancer, 32: 384ff, 2001), Snk/Plk-2 (Serum induced kinase, Liby et al., DNA Sequeuce, 11, 527-33, 2001) und Sak/Plk4 (Fode et al., Proc.Natl.Acad.Sci. U.S.A, 91, 6388ff; 1994).

Die Sequenzidentität innerhalb der Plk-Domänen der Polo-Familie liegt zwischen 40 und 60 %, sodass zum Teil Wechselwirkung von Inhibitoren einer Kinase mit einer oder mehrerer anderen Kinasen dieser Familie auftreten.

Es besteht nach wie vor ein großes Bedürfnis nach wirksamen Verbindungen zur Prophylaxe und Therapie von Erkrankungen, insbesondere von hyperproliferativen Erkrankungen, und ganz besonders von Tumorerkrankungen.

Es wäre deshalb wünschenswert, geeignete Verbindungen mit dualer inhibitorischer Wirkung zur Verfügung zu haben, die sowohl BET-Proteine, als auch Plk-Proteine inhibieren.

Es wurde nun überraschenderweise gefunden, dass substituierte Pyridopyrazinone die erwünschten Eigenschaften aufweisen, d.h. eine BET-inhibitorische, insbesondere eine BRD4 inhibitorische und gleichzeitig eine Plk-inhibitorische, insbesondere Plk-1 inhibitorische Wirkung zeigen.

Die erfindungsgemäßen Verbindungen stellen somit wertvolle Wirkstoffe zur prophylaktischen und therapeutischen Verwendung bei hyper-proliferativen Erkrankungen, insbesondere bei Tumorerkrankungen dar. Desweiteren können die erfindungsgemäßen Verbindungen bei viralen Infektionen, bei neurodegenerativen Erkrankungen, bei inflammatorischen Krankheiten, bei atherosklerotischen Erkrankungen und in der männlichen Fertilitätskontrolle zur Anwendung kommen.

Die erfindungsgemäßen Verbindungen inhibieren sowohl die BET-Proteine, als auch die Polo Like Kinasen, worauf auch deren Wirkung zum Beispiel gegen Krebs, wie solide Tumoren und Leukämie, Autoimmunerkrankungen wie Psoriasis, Alopezie, und Multiple Sklerose, Chemotherapeutika-induzierte Alopezie und Mukositis, kardiovaskulare Erkrankungen, wie Stenosen, Arteriosklerosen und Restenosen, infektiöse Erkrankungen, wie z. B. durch unizellulare Parasiten, wie Trypanosoma, Toxoplasma oder Plasmodium, oder durch Pilze hervorgerufen, nephrologische Erkrankungen, wie z. B. Glomerulonephritis, chronische neurodegenerative Erkrankungen, wie Huntington's Erkrankung, amyotropisch laterale Sklerose, Parkinsonsche Erkrankung, AIDS Dementia und Alzheimersche Erkrankung, akute neurodegenerative Erkrankungen, wie Ischämien des Gehirns und Neurotraumata, virale Infektionen, wie z. B. Cytomegalus-Infektionen, Herpes, Hepatitis B und C, und HIV Erkrankungen basiert.

### Stand der Technik

Die bei der Betrachtung des Standes der Technik angewandte Nomenklatur (abgeleitet aus der Nomenklatursoftware ACD Name batch, Version 12.01, von Advanced Chemical Development, Inc.) wird durch die nachfolgenden Abbildungen verdeutlicht:

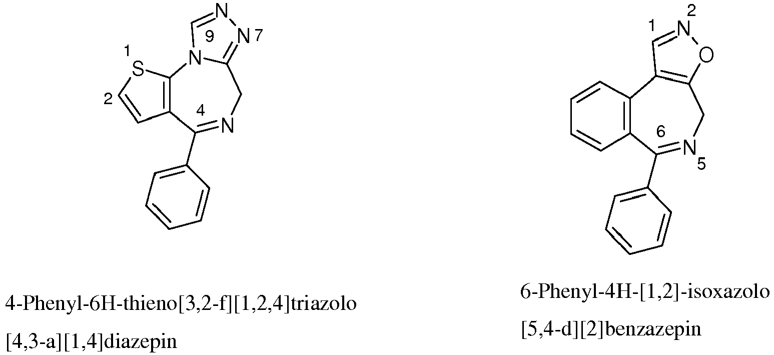

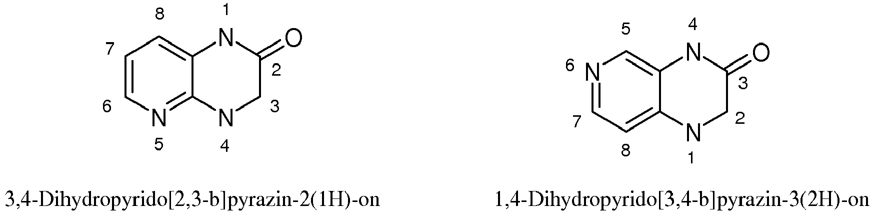

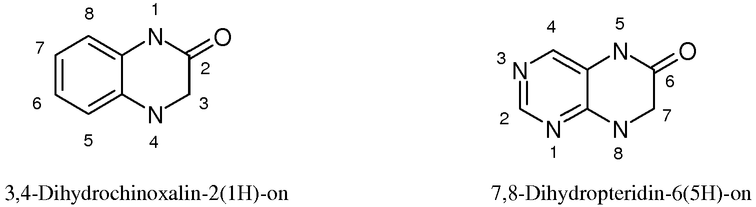

Bezogen auf die chemische Struktur wurden bisher nur sehr wenige Typen von BRD4-Inhibitoren beschrieben (Chun-Wa Chung et al., Progress in Medicinal Chemistry 2012, 51, 1-55).
Die ersten publizierten BRD4-Inhibitoren waren Diazepine. So werden z. B. Phenyl-thieno-triazolo-1,4-diazepine (4-Phenyl-6*H*-thieno[3,2-*f*][1,2,4]triazolo[4,3*-a*][1,4]diazepine) in WO2009/084693 (Mitsubishi Tanabe Pharma Corporation) und als Verbindung JQ1 in WO2011/143669 (Dana Farber Cancer Institute) beschrieben.

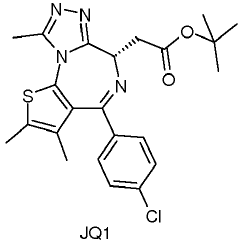

Der Ersatz der Thieno- durch eine Benzo-Einheit führt ebenfalls zu aktiven Inhibitoren (J. Med. Chem. 2011, 54, 3827 - 3838; E. Nicodeme et al., Nature 2010, 468, 1119). Weitere 4-Phenyl-6*H-*thieno[3,2-*f*][1,2,4]triazolo[4,3-*a*][1,4]diazepine und verwandte Verbindungen mit alternativen Ringen als Fusionspartner anstelle der Benzo-Einheit werden generisch beansprucht oder explizit beschrieben in WO2012/075456 (Constellation Pharmaceuticals).
Azepine als BRD4-Inhibitoren werden in der WO2012/075383 (Constellation Pharmaceuticals) beschrieben. Diese Anmeldung betrifft 6-substituierte 4*H*-Isoxazolo[5,4-*d*][2]benzazepine und 4H-Isoxazolo[3,4-*d*][2]benzazepine einschließlich solcher Verbindungen, die an Position 6 optional substituiertes Phenyl aufweisen und auch Analoga mit alternativen heterocyclischen Fusionspartnern anstelle der Benzo-Einheit, wie z.B. Thieno- oder Pyridoazepine. Als eine andere strukturelle Klasse von BRD4-Inhibitoren werden 7-Isoxazolochinoline und verwandte Chinolon-Derivate beschrieben (Bioorganic & Medicinal Chemistry Letters 22 (2012) 2963-2967). In WO2011/054845 (GlaxoSmithKline) werden weitere Benzodiazepine als BRD4-Inhibitoren beschrieben.

Einige Schriften offenbaren strukturell ähnliche Verbindungen, die teils als Hemmer von Zellzyklus-Kinasen, beispielsweise von Plk-1, beschrieben sind, teils aber auch auf völlig andere Wirkmechanismen und teilweise auch auf andere Indikationen gerichtet sind. Dihydropyridopyrazinone sowie verwandte bicyclische Systeme sind in einer Reihe von Patentanmeldungen beschrieben.

WO 2006/005510 bzw. US 2006/009457 (Boehringer Ingelheim) beschreibt 1,4-Dihydropyrido[3,4-b]pyrazin-3(2H)-on-Derivate als Inhibitoren von Plk-1 zur Behandlung hyperproliferativer Erkrankungen. Die beanspruchten Substanzen sind durch eine anilinische Gruppe gekennzeichnet, die via -NH- an C-7 des Dihydropyridopyrazinon-Gerüstes gebunden ist, und seinerseits in *para*-Position mit einem Carboxamid substituiert ist. Im Unterschied dazu weisen die Verbindungen der vorliegenden Erfindung ein substituiertes Aminopyridin an der Stelle der oben genannten anilinischen Gruppe auf.

WO 2013/071217 (OSI Pharmaceuticals) offenbart vor allem 7,8-Dihydropteridin-6(5H)-one, aber auch 1,4-Dihydropyrido[3,4-b]pyrazin-3(2H)-on-Derivate als Hemmer von Kinasen, insbesondere von RSK-1 und RSK-2, als Arzneimittel unter anderem zur Behandlung verschiedener Krebserkrankungen. Die dort offenbarten Verbindungen unterscheiden sich jedoch von den erfindungsgemäßen Verbindungen unter anderem durch die obligat aromatische Substitution an dem der Oxo-Gruppe unmittelbar benachbarten Stickstoff-Atom (N-5 in den Dihydropteridonen, beziehungsweise N-4 in den Dihydropyrido[3,4-b]pyrazinonen).

WO 2010/085570 (Takeda Pharmaceutical Company) beschreibt Hemmer der Poly-ADP-Ribose-Polymerase (PARP), die aus einer Reihe bi- und tricyclischer Gerüste abgeleitet sind, und welche 3,4-Dihydropyrido[2,3-b]pyrazin-2(1H)-on-Derivate einschließen, als Arzneimittel zur Behandlung verschiedener Krankheiten. Die darin offenbarten Beispielverbindungen unterscheiden sich von den erfindungsgemäßen Verbindungen durch die Position des Stickstoffes im Pyridinteil des Pyridopyridazin-Gerüstes, sowie durch Art und Position der dort vorhandenen Substitution.

WO 2011/031965 (Gilead Sciences) beschreibt 3-Deazapteridinon-Derivate (entspricht 1,4-Dihydropyrido[3,4-b]pyrazin-3(2H)-on-Derivaten) als Modulatoren von Toll-like-Rezeptoren zur Behandlung verschiedener Krankheiten. Die dort offenbarten Substanzen unterscheiden sich von den erfindungsgemäßen Verbindungen unter anderem durch die obligate Aminosubstitution an C-5 sowie durch die fehlende Substitution an N-4.

WO 2003/020722 und WO 2004/076454 (Boehringer Ingelheim) offenbaren 7,8-Dihydropteridin-6(5H)-one als Hemmer spezifischer Zellcyclus-Kinasen zur Therapie hyperproliferativer Erkrankungen.

WO 2006/018182 (Boehringer Ingelheim) beschreibt pharmazeutische Zubereitungen von 7,8-Dihydropteridin-6(5H)-onen in Kombination unter anderem mit verschiedenen Zytostatika zur Therapie von Tumorerkrankungen.

WO 2006/018185 (Boehringer Ingelheim) beschreibt die Verwendung von 7,8-Dihydropteridin-6(5H)-onen zur Therapie verschiedener Tumorerkrankungen.

WO 2011/101369 (Boehringer Ingelheim), WO 2011/113293 (Jiangsu Hengrui Medicine), WO 2009/141575 (Chroma Therapeutics) WO 2009/071480 (Nerviano Medical Sciences), sowie WO 2006/021378, WO 2006/021379 und WO 2006/021548 (ebenfalls Boehringer Ingelheim) offenbaren weitere 7,8-Dihydropteridin-6(5H)-on-Derivate als Hemmer von Plk-1 zur Behandlung hyperproliferativer Erkrankungen.

WO 2012/085176 (Hoffmann-La Roche AG) offenbart tricyclische Pyrazinon-Derivate als Hemmer von Janus-Kinasen (JNK) zur Behandlung verschiedener Krankheiten.

WO 2008/117061 (Sterix Ltd) beschreibt eine Reihe bicyclischer Chemotypen, darunter 3,4-Dihydrochinoxalin-2(1H)-on-Derivate, als Inhibitoren der Steroid-Sulfatase, unter anderem zur Verwendung zur Hemmung des Wachstums von Tumoren.

WO 2006/050054, WO 2007/134169 und US 2009/0264384 (Nuada LLC) beschreiben eine Reihe bicyclischer Chemotypen, darunter 3,4-Dihydrochinoxalin-2(1H)-on-Derivate, als Hemmer von Tumor-Nekrose-Faktor alpha (TNF-α) sowie verschiedener Isoformen der Phosphodiesterase zur Behandlung unter anderem von entzündlichen Erkrankungen.

US 2006/0019961 (P. E. Mahaney et al.) beschreibt substituierte 3,4-Dihydrochinoxalin-2(1H)-on-Derivate als Modulatoren des Estrogen-Rezeptors zur Behandlung verschiedener entzündlicher, kardiovaskulärer, sowie Autoimmun-Erkrankungen.

Bei den erfindungsgemäßen Verbindungen handelt es sich hingegen um substituierte 1,4-Dihydropyrido[3,4-b]pyrazin-3(2H)-on-Derivate, die sich strukturell in vielfältiger Form von den oben diskutierten Chemotypen von BRD4- und Plk1-Inhibitoren unterscheiden. Aufgrund der wesentlichen Strukturunterschiede, aber auch im Hinblick auf die Strukturen selbst, war nicht davon auszugehen, dass die hier beanspruchten Verbindungen eine duale Aktivität aufweisen, d.h. dass sie sowohl BRD4-inhibitorisch, als auch Plk-inhibitorisch wirksam sind. Es ist deshalb überraschend, dass die erfindungsgemäßen Verbindungen trotz der erheblichen Strukturunterschiede eine duale Wirkungsweise und somit eine gute inhibitorische Wirkung aufweisen.

Es wurde nun gefunden, dass Verbindungen der allgemeinen Formel (I) 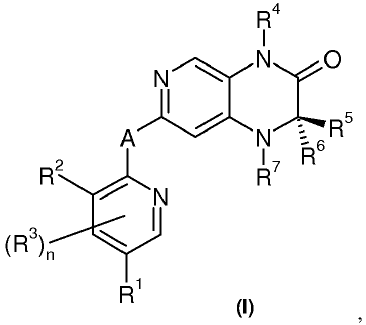 in der
- A: für -NH- oder -O- steht,
- R¹: für eine Gruppe -C(=O)NR⁸R⁹ oder -S(=O)ₐNR⁸R⁹ steht,
oder
für Oxazolin-2-yl-, das gegebenenfalls ein- oder zweifach substituiert sein kann mit C₁-C₃-Alkyl-,
oder
für 5-gliedriges monocyclisches Heteroaryl- steht, das gegebenenfalls ein-, zwei- oder dreifach, gleich oder verschieden substituiert sein kann mit Halogen, Cyano, C₁-C₄-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio-, Halogen-C₁-C₄-alkylthio-, -NR¹⁰R¹¹, -C(=O)OR¹², -C(=O)N¹⁰R¹¹, -C(=O)R¹², -S(=O)₂R¹², -S(=O)₂NR¹⁰R¹¹,
- R²: für Wasserstoff, Halogen, Cyano, C₁-C₄-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio- oder Halogen-C₁-C₄-alkylthio- steht,
- R³: für Halogen, C₁-C₃-Alkyl-, C₁-C₃-Alkoxy- oder Cyano steht,
- R⁴: für Methyl- oder Ethyl- steht,
- R⁵: für Wasserstoff oder C₁-C₃-Alkyl- steht,
- R⁶: für Wasserstoff oder C₁-C₃-Alkyl steht, oder
- R⁵ und R⁶: gemeinsam mit dem Kohlenstoffatom, an das sie gebunden sind, für C₃-C₆-Cycloalkyl stehen,
- R⁷: für C₁-C₆-Alkyl- steht, das gegebenenfalls einfach substituiert sein kann mit Phenyl-, C₃-C₈-Cycloalkyl-, oder 4-bis 8-gliedrigem Heterocycloalkyl-,
worin Phenyl- seinerseits gegebenenfalls ein-, zwei- oder dreifach, gleich oder verschieden substituiert sein kann mit: Halogen, Cyano, C₁-C₄-Alkyl-, C₂-C₄-Alkenyl-, C₂-C₄-Alkinyl-, C₁-C₄-Alkoxy-, Halogen-C₁-C₄-Alkyl-, Halogen-C₁-C₄-Alkoxy-,
und
worin C₃-C₈-Cycloalkyl- und 4-bis 8-gliedriges Heterocycloalkyl- ihrerseits gegebenenfalls ein- oder zweifach substituiert sein können mit C₁-C₃-Alkyl-,
oder
für C₃-C₈-Cycloalkyl- oder 4-bis 8-gliedriges Heterocycloalkyl- steht, die gegebenenfalls ein- oder zweifach substituiert sein können mit C₁-C₃-Alkyl-,
- R⁸: für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₄-Alkoxy-, Halogen-C₁-C₄-alkoxy-, -NR¹⁰R¹¹, C₃-C₈-Cycloalkyl-, C₄-C₈-Cycloalkenyl-, 4- bis 8-gliedrigem Heterocycloalkyl, 4- bis 8-gliedrigem Heterocycloalkenyl-, C₅-C₁₁-Spirocycloalkyl-, C₅-C₁₁-Heterospirocycloalkyl-, verbrücktem C₆-C₁₂-Cycloalkyl-, verbrücktem C₆-C₁₂-Heterocycloalkyl-, C₆-C₁₂-Bicycloalkyl-, C₆-C₁₂-Heterobicycloalkyl-, Phenyl- oder 5- bis 6-gliedrigem Heteroaryl-,
worin C₃-C₈-Cycloalkyl-, C₄-C₈-Cycloalkenyl-, 4- bis 8-gliedriges Heterocycloalkyl-, 4- bis 8-gliedriges Heterocycloalkenyl-, C₅-C₁₁-Spirocycloalkyl-, C₅-C₁₁-Heterospirocycloalkyl-, verbrücktes C₆-C₁₂-Cycloalkyl-, verbrücktes C₆-C₁₂-Heterocycloalkyl-, C₆-C₁₂-Bicycloalkyl-, C₆-C₁₂-Heterobicycloalkyl- gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR¹⁰R¹¹,
und
worin Phenyl- und 5- bis 6-gliedriges Heteroaryl- gegebenenfalls ein- oder zweifach, gleich oder verschieden substituiert sein können mit: Halogen, Cyano, Trifluormethyl-, C₁-C₃-Alkyl-, C₁-C₃-Alkoxy-,
oder für C₃-C₆-Alkenyl- oder C₃-C₆-Alkinyl- steht,
oder für Fluor-C₁-C₃-Alkyl- steht, das gegebenenfalls einfach substituiert sein kann durch Cyano oder Hydroxy,
oder für C₃-C₈-Cycloalkyl- C₄-C₈-Cycloalkenyl-, C₅-C₁₁-Spirocycloalkyl-, verbrücktes C₆-C₁₂-Cycloalkyl- oder C₆-C₁₂-Bicycloalkyl- steht, die gegebenenfalls ein- oder zweifach, gleich oder verschieden substituiert sein können mit: Hydroxy, Oxo, Cyano, Fluor, C₁-C₃-Alkyl, C₁-C₃-Alkoxy, Trifluormethyl, -NR¹⁰R¹¹,
oder für 4- bis 8-gliedriges Heterocycloalkyl-, 4- bis 8-gliedriges
Heterocycloalkenyl-, C₅-C₁₁-Heterospirocycloalkyl-, verbrücktem C₆-C₁₂-Heterocycloalkyl- oder C₆-C₁₂-Heterobicycloalkyl- steht, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR¹⁰R¹¹,
- R⁹: für Wasserstoff oder für gegebenenfalls ein- oder zweifach, gleich oder verschieden mit Hydroxy, Oxo, C₁-C₃-Alkoxy- substituiertes C₁-C₃-Alkyl-, oder
für Fluor-C₁-C₃-Alkyl steht,
oder
- R⁸ und R⁹: gemeinsam mit dem Stickstoffatom, an welches sie gebunden sind, für 4-bis 8-gliedriges Heterocycloalkyl, 4-bis 8-gliedriges Heterocycloalkenyl-, C₅-C₁₁-Heterospirocycloalkyl-, verbrücktes C₆-C₁₂-Heterocycloalkyl- oder C₆-C₁₂-Heterobicycloalkyl- stehen, die gegebenenfalls ein- oder zweifach, gleich oder
verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR¹⁰R¹¹,
- R¹⁰ und R¹¹: unabhängig voneinander für Wasserstoff oder für gegebenenfalls ein- oder zweifach, gleich oder verschieden mit Hydroxy, Oxo, C₁-C₃-Alkoxy- substituiertes C₁-C₃-Alkyl, oder für Fluor-C₁-C₃-Alkyl stehen,
oder
- R¹⁰ und R¹¹: gemeinsam mit dem Stickstoffatom, an das sie gebunden sind, für 4- bis 8-gliedriges Heterocycloalkyl- stehen, das gegebenenfalls ein- oder zweifach, gleich oder verschieden substituiert sein kann mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-oder C₁-C₄-Alkoxycarbonyl-,
- R¹²: für C₁-C₆-Alkyl- oder Phenyl-C₁-C₃-Alkyl- steht, und
- n: für 0 oder 1 steht,
sowie deren Diastereomere, Racemate, Metaboliten, Polymorphe und physiologisch verträglichen Salze, überraschenderweise die Interaktion zwischen BET-Proteinen, insbesondere BRD4 und einem acetylierten Histon 4-Peptid und die Kinase Plk-1 inhibieren und somit über den genannten dualen Mechanismus die oben beschriebenen Eigenschaften aufweisen und insbesondere das Wachstum von Krebszellen hemmen.

Bevorzugt sind solche Verbindungen der allgemeinen Formel (I),
in der
- A: für -NH- steht,
- R¹: für eine Gruppe -C(=O)NR⁸R⁹ oder -S(=O)ₐNR⁸R⁹ steht,
oder für Oxazolin-2-yl- steht, das gegebenenfalls ein- oder zweifach substituiert sein kann mit C₁-C₃-Alkyl-,
oder
für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluormethoxy- oder -NR¹⁰R¹¹,
- R²: für Wasserstoff, Fluor, Chlor, Cyano, Methyl-, Ethyl-, Methoxy- oder Ethoxy-steht,
- R³: für Fluor, Chlor oder Methyl- steht,
- R⁴: für Methyl- steht,
- R⁵: für Wasserstoff, Methyl- oder Ethyl- steht,
- R⁶: für Wasserstoff, Methyl- oder Ethyl- steht,
- R⁷: für C₃-C₅-Alkyl- steht,
oder
für Methyl- oder Ethyl- steht, die einfach substituiert sein können mit Phenyl- oder 4-bis 8-gliedrigem Heterocycloalkyl-,
worin Phenyl- seinerseits gegebenenfalls ein- oder zweifach, gleich oder verschieden substituiert sein kann mit: Fluor, Chlor, Brom, Cyano, C₁-C₃-Alkyl-, C₁-C₃-Alkoxy-, Trifluoromethyl-,
und
worin 4-bis 8-gliedriges Heterocycloalkyl- seinerseits gegebenenfalls ein- oder zweifach substituiert sein kann mit Methyl-,
oder
für C₃-C₆-Cycloalkyl- oder 4-bis 8-gliedriges Heterocycloalkyl- steht, die gegebenenfalls ein- oder zweifach substituiert sein können mit Methyl-,
- R⁸: für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₃-Alkoxy-, Fluor-C₁-C₃-alkoxy-, -NR¹⁰R¹¹, 4- bis 8-gliedrigem Heterocycloalkyl-, Phenyl- oder 5- bis 6-gliedrigem Heteroaryl-,
worin das 4- bis 8-gliedrige Heterocycloalkyl- gegebenenfalls einfach substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
und worin Phenyl- und 5- bis 6-gliedriges Heteroaryl- gegebenenfalls ein- oder zweifach, gleich oder verschieden substituiert sein können mit: Fluor, Chlor,
Cyano, Trifluormethyl-, Methyl-, Methoxy-,
oder für Fluor-C₁-C₃-Alkyl- steht,
oder für C₃-C₆-Cycloalkyl- steht, das gegebenenfalls ein- oder zweifach, gleich oder verschieden substituiert sein kann mit: Hydroxy, Oxo, Cyano, Fluor, -NR¹⁰R¹¹,
oder für 4- bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- steht, die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
- R⁹: für Wasserstoff oder C₁-C₃-Alkyl- steht,
oder
- R⁸ und R⁹: gemeinsam mit dem Stickstoffatom, an welches sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- stehen,
die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
- R¹⁰ und R¹¹: unabhängig voneinander für Wasserstoff oder für gegebenenfalls einfach mit Hydroxy oder Oxo substituiertes C₁-C₃-Alkyl- oder für Trifluormethyl- stehen, oder
- R¹⁰ und R¹¹: gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert-*Butoxycarbonyl-, und
- n: für 0 oder 1 steht,
sowie deren Diastereomere, Racemate, Polymorphe und physiologisch verträglichen Salze.

Besonders bevorzugt sind solche Verbindungen der allgemeinen Formel (I), in der
- A: für -NH- steht,
- R¹: für eine Gruppe -C(=O)NR⁸R⁹ oder -S(=O)ₐNR⁸R⁹ steht,
oder
für Oxazolin-2-yl- steht, das gegebenenfalls ein- oder zweifach substituiert sein kann mit C₁-C₃-Alkyl-,
- R²: für Wasserstoff, Methyl-, Ethyl- oder Methoxy- steht,
- R⁴: für Methyl- steht,
- R⁵: für Methyl- oder Ethyl- steht,
- R⁶: für Wasserstoff steht,
- R⁷: für C₃-C₅-Alkyl- steht,
oder
für Methyl- steht, das einfach substituiert ist mit Phenyl- oder 4-bis 6-gliedrigem Heterocycloalkyl-, worin Phenyl- seinerseits gegebenenfalls ein-, oder zweifach, gleich oder verschieden substituiert sein kann mit: Fluor, Chlor, Cyano, Methyl-, Methoxy-,
und
worin 4-bis 6-gliedriges Heterocycloalkyl- seinerseits gegebenenfalls einfach substituiert sein kann mit Methyl-,
oder
für C₃-C₆-Cycloalkyl- oder für 4-bis 6-gliedriges Heterocycloalkyl- steht,
- R⁸: für C₁-C₄-Alkyl- steht, das gegebenenfalls ein- oder zweifach substituiert sein kann mit Hydroxy, C₁-C₃-Alkoxy-, -NR¹⁰R¹¹, 4- bis 8-gliedrigem Heterocycloalkyl-, Phenyl- oder 5- bis 6-gliedrigem Heteroaryl-,
worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-,
und worin Phenyl- und 5- bis 6-gliedriges Heteroaryl- gegebenenfalls ein- oder zweifach, gleich oder verschieden substituiert sein können mit: Fluor, Chlor,
Cyano, Trifluormethyl-, Methyl- oder Methoxy-,
oder für Fluor-C₁-C₃-Alkyl- steht,
oder für C₃-C₆-Cycloalkyl- steht, das gegebenenfalls einfach substituiert sein kann mit Hydroxy, Fluor oder -NR¹⁰R¹¹,
oder für 4- bis 8-gliedriges Heterocycloalkyl-, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
- R⁹: für Wasserstoff oder Methyl- steht, oder
- R⁸ und R⁹: gemeinsam mit dem Stickstoffatom, an welches sie gebunden sind, für 5-bis 6-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
- R¹⁰ und R¹¹: unabhängig voneinander für Wasserstoff oder für C₁-C₃-Alkyl- stehen, oder
- R¹⁰ und R¹¹: gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-, und
- n: für 0 steht,
sowie deren Diastereomere, Racemate, Polymorphe und physiologisch verträglichen Salze.

Ganz besonders bevorzugt sind solche Verbindungen der allgemeinen Formel (I), in der
- A: für -NH- steht,
- R¹: für eine Gruppe -C(=O)NR⁸R⁹ steht,
oder
für Oxazolin-2-yl- steht, das gegebenenfalls ein- oder zweifach substituiert sein kann mit C₁-C₃-Alkyl-,
- R²: für Methyl-, Ethyl- oder Methoxy- steht,
- R⁴: für Methyl- steht,
- R⁵: für Methyl- oder Ethyl- steht,
- R⁶: für Wasserstoff steht,
- R⁷: für C₃-C₅-Alkyl- steht,
oder
für C₃-C₆-Cycloalkyl- steht,
- R⁸: für C₁-C₃-Alkyl- steht, das gegebenenfalls einfach substituiert sein kann mit Hydroxy, C₁-C₃-Alkoxy-, Phenyl- oder 5- bis 6-gliedrigem Heteroaryl-, worin Phenyl- und 5- bis 6-gliedriges Heteroaryl- gegebenenfalls ein- oder zweifach, gleich oder verschieden substituiert sein können mit: Fluor, Chlor, Methyl- oder Methoxy-,
oder für Fluor-C₁-C₃-Alkyl- steht,
oder für C₃-C₆-Cycloalkyl- steht,
oder für 4- bis 8-gliedriges Heterocycloalkyl-, das gegebenenfalls ein- oder zweifach, gleich oder verschieden substituiert sein kann mit: Oxo oder C₁-C₃-Alkyl-,
- R⁹: für Wasserstoff steht,
- n: für 0 steht,
sowie deren Diastereomere, Racemate, Polymorphe und physiologisch verträglichen Salze.

Überaus bevorzugt sind solche Verbindungen der allgemeinen Formel (I),
in der
- A: für -NH- steht,
- R¹: für eine Gruppe -C(=O)NR⁸R⁹ steht,
oder
für Oxazolin-2-yl- steht, das gegebenenfalls ein- oder zweifach substituiert sein kann mit Methyl-,
- R²: für Methyl-, Ethyl- oder Methoxy- steht,
- R⁴: für Methyl- steht,
- R⁵: für Methyl- oder Ethyl- steht,
- R⁶: für Wasserstoff steht,
- R⁷: für Cyclopentyl- steht,
- R⁸: für C₁-C₄-Alkyl- steht, das gegebenenfalls einfach substituiert sein kann mit Hydroxy, Methoxy- oder Pyridinyl-,
oder für Fluor-C₁-C₂-Alkyl- steht,
oder für C₃-C₆-Cycloalkyl- steht,
oder für 4- bis 8-gliedriges Heterocycloalkyl-, das gegebenenfalls ein- oder zweifach, gleich oder verschieden substituiert sein kann mit: Oxo oder C₁-C₃-Alkyl-,
- R⁹: für Wasserstoff steht,
- n: für 0 steht,
sowie deren Diastereomere, Racemate, Polymorphe und physiologisch verträglichen Salze.

Noch mehr bevorzugt sind solche Verbindungen der allgemeinen Formel (I), in der
- A: für -NH- steht,
- R¹: für eine Gruppe -C(=O)NR⁸R⁹ steht,
oder
für Oxazolin-2-yl- steht, das zweifach substituiert ist mit Methyl-,
- R²: für Methyl-, Ethyl- oder Methoxy- steht,
- R⁴: für Methyl- steht,
- R⁵: für Methyl- oder Ethyl- steht,
- R⁶: für Wasserstoff steht,
- R⁷: für Cyclopentyl- steht,
- R⁸: für C₁-C₄-Alkyl- steht, das gegebenenfalls einfach substituiert sein kann mit Hydroxy, Methoxy- oder Pyridinyl-,
oder für 2,2,2-Trifluorethyl- steht,
oder für Cyclopropyl- oder Cyclohexyl- steht,
oder für Piperidinyl, Azepanyl oder Tetrahydropyranyl, die gegebenenfalls ein- oder zweifach, gleich oder verschieden substituiert sein können mit: Oxo oder Methyl,
- R⁹: für Wasserstoff steht,
- n: für 0 steht,
sowie deren Diastereomere, Racemate, Polymorphe und physiologisch verträglichen Salze.

Bevorzugt sind Verbindungen der allgemeinen Formel (I), in denen A für -NH- steht.

Bevorzugt sind Verbindungen der allgemeinen Formel (I), in denen A für -O- steht.

Bevorzugt sind Verbindungen der allgemeinen Formel (I), in denen R¹ für -C(=O)NR⁸R⁹ steht.

Bevorzugt sind Verbindungen der allgemeinen Formel (I), in denen R¹ für -S(=O)ₐNR⁸R⁹ steht.

Bevorzugt sind Verbindungen der allgemeinen Formel (I), in denen R¹ für Oxazolin-2-yl- steht, das gegebenenfalls ein- oder zweifach, gleich oder verschieden substituiert sein kann mit C₁-C₃-Alkyl-,

In der allgemeinen Formel (I), kann R¹ für 5-gliedriges monocyclisches Heteroaryl-, das gegebenenfalls ein-, zwei- oder dreifach, gleich oder verschieden substituiert sein kann mit Halogen, Cyano, C₁-C₄-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio-, Halogen-C₁-C₄-alkylthio-, -NR¹⁰R¹¹, -C(=O)OR¹², -C(=O)N¹⁰R¹¹, -C(=O)R¹², -S(=O)₂R¹², -S(=O)ₐNR¹⁰R¹¹, stehen.

Bevorzugt sind Verbindungen der allgemeinen Formel (I), in denen R¹ für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluormethoxy- oder -NR¹⁰R¹¹.

Bevorzugt sind Verbindungen der allgemeinen Formel (I), in denen R² für Wasserstoff, Fluor, Chlor, Cyano, Methyl-, Ethyl-, Methoxy- oder Ethoxy- steht.

Bevorzugt sind Verbindungen der allgemeinen Formel (I), in denen R² für Wasserstoff, Methyl-, Ethyl- oder Methoxy- steht.

Bevorzugt sind Verbindungen der allgemeinen Formel (I), in denen R² für Wasserstoff steht.

Besonders bevorzugt sind Verbindungen der allgemeinen Formel (I), in denen R² für Methyl-, Ethyl- oder Methoxy- steht.

Besonders bevorzugt sind Verbindungen der allgemeinen Formel (I), in denen R² für Methyl- steht.

Besonders bevorzugt sind Verbindungen der allgemeinen Formel (I), in denen R² für Ethyl- steht.

Besonders bevorzugt sind Verbindungen der allgemeinen Formel (I), in denen R² für Methoxysteht.

Bevorzugt sind Verbindungen der allgemeinen Formel (I), in denen R³ für Fluor, Chlor oder Methyl- steht.

Bevorzugt sind Verbindungen der allgemeinen Formel (I), in denen R⁴ für Wasserstoff, Methyl- oder Ethyl- steht.

Bevorzugt sind Verbindungen der allgemeinen Formel (I), in denen R⁴ für Methyl- oder Ethylsteht.

Bevorzugt sind Verbindungen der allgemeinen Formel (I), in denen R⁴ für Ethyl- steht.

Besonders bevorzugt sind Verbindungen der allgemeinen Formel (I), in denen R⁴ für Methyl- steht.

Bevorzugt sind Verbindungen der allgemeinen Formel (I), in denen R⁵ für Wasserstoff, Methyl- oder Ethyl- steht.

Bevorzugt sind Verbindungen der allgemeinen Formel (I), in denen R⁵ für Methyl- oder Ethyl-steht.

Bevorzugt sind Verbindungen der allgemeinen Formel (I), in denen R⁵ für Ethyl- steht.

Bevorzugt sind Verbindungen der allgemeinen Formel (I), in denen R⁵ für Methyl- steht.

Bevorzugt sind Verbindungen der allgemeinen Formel (I), in denen je ein Substituent aus R⁵ und R⁶ für Methyl- und einer für Wasserstoff steht, so dass bezüglich des aus R⁵, R⁶ und dem an R⁵ und R⁶ gebundenen Kohlenstoffatom gebildeten Stereozentrums ein Racemat resultiert.

Bevorzugt sind Verbindungen der allgemeinen Formel (I), in denen je ein Substituent aus R⁵ und R⁵ für Methyl- und einer für Wasserstoff steht, so dass bezüglich des aus R⁵, R⁶ und dem an R⁵ und R⁶ gebundenen Kohlenstoffatom gebildeten Stereozentrums ein Isomerengemisch resultiert, in welchem die (R)-Form überwiegt.

Besonders bevorzugt sind Verbindungen der allgemeinen Formel (I), in denen R⁵ für Methyl- und R⁶ für Wasserstoff steht.

Besonders bevorzugt sind Verbindungen der allgemeinen Formel (I), in denen R⁵ für Ethyl- und R⁶ für Wasserstoff steht.

Bevorzugt sind Verbindungen der allgemeinen Formel (I), in denen R⁷ für unsubstituiertes C₃-C₅-Alkyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-,
und
worin 4-bis 6-gliedriges Heterocycloalkyl- seinerseits gegebenenfalls einfach substituiert sein kann mit Methyl-,
oder
für C₃-C₆-Cycloalkyl- steht, oder für 4-bis 6-gliedriges Heterocycloalkyl-,

Bevorzugt sind Verbindungen der allgemeinen Formel (I), in denen R⁷ für unsubstituiertes C₃-C₅-Alkyl steht.

Bevorzugt sind Verbindungen der allgemeinen Formel (I), in denen R⁷ für C₃-C₅-Alkyl steht.

Bevorzugt sind Verbindungen der allgemeinen Formel (I), in denen R⁷ für Methyl- steht, das einfach substituiert ist mit Phenyl-,
worin Phenyl- seinerseits gegebenenfalls ein-, oder zweifach, gleich oder verschieden substituiert sein kann mit: Fluor, Chlor, Cyano, Methyl-, Methoxy-.

Bevorzugt sind Verbindungen der allgemeinen Formel (I), in denen R⁷ für Methyl- steht, das einfach substituiert ist mit 4-bis 6-gliedrigem Heterocycloalkyl-,
worin 4-bis 6-gliedriges Heterocycloalkyl- seinerseits gegebenenfalls einfach substituiert sein kann mit Methyl-.

Bevorzugt sind Verbindungen der allgemeinen Formel (I), in denen R⁷ für C₃-C₆-Cycloalkyl- steht.

Bevorzugt sind Verbindungen der allgemeinen Formel (I), in denen R⁷ für 4-bis 6-gliedriges Heterocycloalkyl- steht.

Besonders bevorzugt sind Verbindungen der allgemeinen Formel (I), in denen R⁷ für Cyclopentylsteht,

Bevorzugt sind Verbindungen der allgemeinen Formel (I), in denen R⁸ für C₁-C₄-Alkyl- steht, das gegebenenfalls einfach substituiert sein kann mit Hydroxy, C₁-C₃-Alkoxy-, -NR¹⁰R¹¹, 4- bis 8-gliedrigem Heterocycloalkyl-, Phenyl- oder 5- bis 6-gliedrigem Heteroaryl-,
worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-,
und worin Phenyl- und 5- bis 6-gliedriges Heteroaryl- gegebenenfalls ein- oder zweifach, gleich oder verschieden substituiert sein können mit: Fluor, Chlor, Cyano, Trifluormethyl-, Methyl- oder Methoxy-,
oder für Fluor-C₁-C₃-Alkyl- steht,
oder für C₃-C₆-Cycloalkyl- steht, das gegebenenfalls einfach substituiert sein kann mit Hydroxy, Fluor oder -NR¹⁰R¹¹,
oder für 4- bis 8-gliedriges Heterocycloalkyl-, das gegebenenfalls ein- oder zweifach, gleich oder verschieden substituiert sein kann mit: Oxo, C₁-C₃-Alkyl, Fluor-C₁-C₃-Alkyl-, Cyclopropyl- oder Cyclopropylmethyl-.

Bevorzugt sind Verbindungen der allgemeinen Formel (I), in denen R⁸ für C₁-C₄-Alkyl- steht, das gegebenenfalls einfach substituiert sein kann mit Hydroxy, C₁-C₃-Alkoxy-, -NR¹⁰R¹¹, 4- bis 8-gliedrigem Heterocycloalkyl-, Phenyl- oder 5- bis 6-gliedrigem Heteroaryl-,
worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-,
und worin Phenyl- und 5- bis 6-gliedriges Heteroaryl- gegebenenfalls ein- oder zweifach, gleich oder verschieden substituiert sein können mit: Fluor, Chlor, Cyano, Trifluormethyl-, Methyl- oder Methoxy-.

Bevorzugt sind Verbindungen der allgemeinen Formel (I), in denen R⁸ für C₃-C₆-Cycloalkyl- steht, das gegebenenfalls einfach substituiert sein kann mit Hydroxy, Fluor oder -NR¹⁰R¹¹.

Bevorzugt sind Verbindungen der allgemeinen Formel (I), in denen R⁸ für 4- bis 8-gliedriges Heterocycloalkyl-, das gegebenenfalls ein- oder zweifach, gleich oder verschieden substituiert sein kann mit: Oxo, C₁-C₃-Alkyl, Fluor-C₁-C₃-Alkyl-, Cyclopropyl- oder Cyclopropylmethyl-.

Besonders bevorzugt sind Verbindungen der allgemeinen Formel (I), in denen R⁸ für C₁-C₃-Alkyl-steht, das gegebenenfalls einfach substituiert sein kann mit -NR¹⁰R¹¹, Hydroxy, C₁-C₃-Alkoxy-, Phenyl- oder 5- bis 6-gliedrigem Heteroaryl-,
worin Phenyl- und 5- bis 6-gliedriges Heteroaryl- gegebenenfalls ein- oder zweifach, gleich oder verschieden substituiert sein können mit: Fluor, Chlor, Methyl- oder Methoxy-,
oder für Fluor-C₁-C₃-Alkyl- steht,
oder für C₃-C₆-Cycloalkyl- steht,
oder für 4- bis 6-gliedriges Heterocycloalkyl-, das gegebenenfalls einfach substituiert sein kann mit: Oxo oder Methyl-.

Besonders bevorzugt sind Verbindungen der allgemeinen Formel (I), in denen R⁸ für für C₁-C₃-Alkyl- steht, das gegebenenfalls einfach substituiert sein kann mit -NR¹⁰R¹¹, Hydroxy, C₁-C₃-Alkoxy-, Phenyl- oder 5- bis 6-gliedrigem Heteroaryl-,
worin Phenyl- und 5- bis 6-gliedriges Heteroaryl- gegebenenfalls ein- oder zweifach, gleich oder verschieden substituiert sein können mit: Fluor, Chlor, Methyl- oder Methoxy-.

Besonders bevorzugt sind Verbindungen der allgemeinen Formel (I), in denen R⁸ für Fluor-C₁-C₃-Alkyl steht.

Besonders bevorzugt sind Verbindungen der allgemeinen Formel (I), in denen R⁸ für C₃-C₆-Cycloalkyl- steht.

Besonders bevorzugt sind Verbindungen der allgemeinen Formel (I), in denen R⁸ für 4- bis 6-gliedriges Heterocycloalkyl- steht, das gegebenenfalls einfach substituiert sein kann mit: Oxo oder C₁-C₃-Alkyl-.

Bevorzugt sind Verbindungen der allgemeinen Formel (I), in denen R⁹ für Wasserstoff oder Methyl- steht.

Bevorzugt sind Verbindungen der allgemeinen Formel (I), in denen R⁹ für Methyl- steht.

Besonders bevorzugt sind Verbindungen der allgemeinen Formel (I), in denen R⁹ für Wasserstoff steht.

Bevorzugt sind Verbindungen der allgemeinen Formel (I), in denen R⁸ und R⁹, gemeinsam mit dem Stickstoffatom, an das sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl-stehen,
die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-.

Bevorzugt sind Verbindungen der allgemeinen Formel (I), in denen R⁸ und R⁹, gemeinsam mit dem Stickstoffatom, an das sie gebunden sind, für 4-bis 8-gliedriges Heterocycloalkyl- stehen,
das gegebenenfalls ein- oder zweifach, gleich oder verschieden substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert-*Butoxycarbonyl-.

Besonders bevorzugt sind Verbindungen der allgemeinen Formel (I), in denen R⁸ und R⁹, gemeinsam mit dem Stickstoffatom, an das sie gebunden sind, für 5-bis 6-gliedriges Heterocycloalkyl- oder C₆-C₈-Heterospirocycloalkyl- stehen, die gegebenenfalls ein- oder zweifach, gleich oder verschieden substituiert sein können mit: Oxo, C₁-C₃-Alkyl, Fluor-C₁-C₃-Alkyl-, Cyclopropyl- oder Cyclopropylmethyl-.

Besonders bevorzugt sind Verbindungen der allgemeinen Formel (I), in denen R⁸ und R⁹, gemeinsam mit dem Stickstoffatom, an das sie gebunden sind, für 5-bis 6-gliedriges Heterocycloalkyl- stehen, das gegebenenfalls ein- oder zweifach, gleich oder verschieden substituiert sein kann mit: Oxo, C₁-C₃-Alkyl, Fluor-C₁-C₃-Alkyl-, Cyclopropyl- oder Cyclopropylmethyl-.

Bevorzugt sind Verbindungen der allgemeinen Formel (I), in denen R¹⁰ und R¹¹ unabhängig voneinander für Wasserstoff oder für gegebenenfalls einfach mit Hydroxy oder Oxo substituiertes C₁-C₃-Alkyl- oder für Trifluormethyl- stehen.

Besonders bevorzugt sind Verbindungen der allgemeinen Formel (I), in denen R¹⁰ und R¹¹ unabhängig voneinander für Wasserstoff oder für C₁-C₃-Alkyl- stehen.

Bevorzugt sind Verbindungen der allgemeinen Formel (I), in denen R¹⁰ und R¹¹, gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-.

Bevorzugt sind Verbindungen der allgemeinen Formel (I), in denen n für die Zahl 1 steht.

Bevorzugt sind Verbindungen der allgemeinen Formel (I), in denen n für die Zahl 0 oder die Zahl 1 steht.

Besonders bevorzugt sind Verbindungen der allgemeinen Formel (I), in denen n für die Zahl 0 steht.

Besonders bevorzugt sind Verbindungen der allgemeinen Formel (I), in denen n für die Zahl 0 steht, und in denen A für -NH- steht, R⁴ für Methyl- steht, R⁵ für Methyl- oder Ethyl- steht und R⁶ für Wasserstoff steht.

Die in den jeweiligen Kombinationen bzw. bevorzugten Kombinationen von Resten im Einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Ganz besonders bevorzugt sind die nachfolgenden Verbindungen der allgemeinen Formel (I):
6-{[1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid,
6-{[(2*R*)-1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid,
6-{[(2*R*)-1-Cyclopentyl-2-ethyl-4-methyl-3-oxo-1,2,3,4-tetrahydropyrido[3,4-b]pyrazin-7-yl]amino}-N-cyclopropyl-5-ethylpyridin-3-carboxamid,
6-[(1-Cyclopentyl-2-ethyl-1,2,3,4-tetrahydro-4-methyl-3-oxopyrido[3,4-b]pyrazin-7-yl)amino]-N-cyclopropyl-5-methoxy-3-pyridincarboxamid,
6-[[(2*R*)-1-Cyclopentyl-2-ethyl-1,2,3,4-tetrahydro-4-methyl-3-oxopyrido[3,4-b]pyrazin-7-yl)amino]-N-cyclopropyl-5-methoxy-3-pyridincarboxamid,
6-[(1-Cyclopentyl-2,4-dimethyl-3-oxo-1,2,3,4-tetrahydropyrido[3,4-b]pyrazin-7-yl)amino]-5-methoxy-N-(1-methylpiperidin-4-yl)pyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(2,2,2-trifluoroethyl)-3-pyridincarboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(2-methoxyethyl)-3-pyridincarboxamid,
6-{[(2*R*)-1-Cyclopentyl-2-ethyl-4-methyl-3-oxo-1,2,3,4-tetrahydropyrido[3,4-b]pyrazin-7-yl]amino}-5-ethyl-*N*-[(3R)-2-oxoazepan-3-yl]pyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(tetrahydro-2H-pyran-4-yl)-3-pyridincarboxamid,
6-[[(2*R*)-1-Cyclopentyl-2-ethyl-1,2,3,4-tetrahydro-4-methyl-3-oxopyrido[3,4-b]pyrazin-7-yl]amino]-*N*-(2-hydroxy-1,1-dimethylethyl)-5-methoxy-3-pyridincarboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(3-pyridinylmethyl)-3-pyridincarboxamid,
6-{[(2*R*)-1-Cyclopentyl-2-ethyl-4-methyl-3-oxo-1,2,3,4-tetrahydropyrido[3,4-b]pyrazin-7-yl]amino}-*N*-cyclopropyl-5-methylpyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(1-methyl-4-piperidinyl)-3-pyridincarboxamid,
6-{[(2*R*)-1-Cyclopentyl-2-ethyl-4-methyl-3-oxo-1,2,3,4-tetrahydropyrido[3,4-b]pyrazin-7-yl]amino}-5-methylpyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-*N*-[(3*R*)-hexahydro-2-oxo-1H-azepin-3-yl]-5-methoxy-3-pyridincarboxamid,
6-{[(2*R*)-1-Cyclopentyl-2-ethyl-4-methyl-3-oxo-1,2,3,4-tetrahydropyrido[3,4-b]pyrazin-7-yl]amino}-5-methyl-*N*-(tetrahydro-2H-pyran-4-yl)pyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-5-methyl-*N*-(1-methyl-4-piperidinyl)pyridin-3-carboxamid,
1*N*-Cyclopentyl-7-[[5-(4,5-dihydro-4,4-dimethyl-2-oxazolyl)-3-methoxy-2-pyridinyl]amino]-(2*R*)-ethyl-4*N*-methyl-1,4-dihydro-pyrido[3,4-b]pyrazin-3(2H)-on,
*N*-Cyclohexyl-6-[[(2*R*)-1-cyclopentyl-2-ethyl-1,2,3,4-tetrahydro-4-methyl-3-oxopyrido[3,4-b]pyrazin-7-yl]amino]-5-methoxy-pyridin-3-carboxamid.

### Definitionen:

Unter C₁-C₆-Alkyl-, bzw. einer C₁-C₆-Alkyl-Gruppe ist ein linearer oder verzweigter, gesättigter, monovalenter Kohlenwasserstoffrest zu verstehen, wie z.B. ein Methyl-, Ethyl-, Propyl-, Butyl-, Pentyl-, Hexyl-, *iso*-Propyl-, *iso*-Butyl-, *sec*-Butyl, *tert*-Butyl-*, iso*-Pentyl-, 2-Methylbutyl-, 1-Methylbutyl-, 1-Ethylpropyl-, 1,2-Dimethylpropyl-, *neo*-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- oder 1,2-Dimethylbutyl-Rest.
Vorzugsweise ist unter C₁-C₆-Alkyl- bzw. einer C₁-C₆-Alkyl-Gruppe C₁-C₄-Alkyl-beziehungsweise C₂-C₅-Alkyl-, besonders bevorzugt C₁-C₃-Alkyl- beziehungsweise ein Methyl-, Ethyl-, Propyl- oder Isopropyl-Rest zu verstehen.

Unter C₂-C₄-Alkenyl-, bzw. einer C₂-C₄-Alkenyl-Gruppe ist ein linearer oder verzweigter, monovalenter Kohlenwasserstoffrest mit einer oder zwei C=C-Doppelbindungen zu verstehen, wie z.B. ein Ethenyl-, (*E*)-Prop-2-enyl-, (Z)-Prop-2-enyl-, Allyl- (Prop-1-enyl-), Allenyl- Buten-1-yl-, oder Buta-1,3-dienyl-Rest. Bevorzugt sind Ethenyl- und Allyl-.

Unter C₂-C₄-Alkinyl-, bzw. einer C₂-C₄-Alkinyl-Gruppe ist ein linearer oder verzweigter, monovalenter Kohlenwasserstoffrest mit einer C=C-Dreifachbindung zu verstehen, wie z.B. ein Ethinyl-, Propargyl- (Prop-1-inyl-), oder Butin-1-yl-Rest. Bevorzugt sind Ethinyl und Propargyl.

Unter C₁-C₄-Alkoxy-, bzw. einer C₁-C₄-Alkoxy-Gruppe ist ein linearer oder verzweigter, gesättigter Alkyletherrest -O-Alkyl zu verstehen, wie z.B. ein Methoxy-, Ethoxy-, n-Propoxy-, Isopropoxy- oder *tert*.-Butoxy-Rest.
Vorzugsweise ist unter C₁-C₄-Alkoxy-, bzw. einer C₁-C₄-Alkoxy-Gruppe C₁-C₃-Alkoxy-, besonders bevorzugt ein Methoxy- oder Ethoxy-Rest zu verstehen.

Unter C₁-C₄-Alkylthio-, bzw. einer C₁-C₄-Alkylthio-Gruppe ist ein linearer oder verzweigter, gesättigter Alkylthioetherrest -S-Alkyl zu verstehen, wie z.B. ein Methylthio-, Ethylthio-, n-Propylthio-, Isopropylthio-, oder tert.-Butylthio-Rest.
Vorzugsweise ist unter C₁-C₄-Alkylthio-, bzw. einer C₁-C₄-Alkylthio-Gruppe C₁-C₃-Alkylthio-, besonders bevorzugt ein ein Methylthio- oder Ethylthio-Rest zu verstehen.

Unter einem Heteroatom ist zu verstehen -O-, NH-, =N- oder -S-. Das Heteroatom -NH- kann gegebenenfalls substituiert sein durch C₁-C₃-Alkyl, C₁-C₃-Alkylcarbonyl, C₁-C₄-Alkoxycarbonyl, oder -S(=O)₂-C₁-C₃-Alkyl.
Bevorzugt sind ein Sauerstoff- oder ein Stickstoffatom.

Unter Oxo, beziehungsweise einem Oxo-Substituenten ist ein doppelt gebundenes Sauerstoff-Atom =O zu verstehen. Oxo kann an Atome geeigneter Valenz gebunden sein, beispielsweise an ein gesättigtes Kohlenstoff-Atom oder an Schwefel.
Bevorzugt ist die Bindung an Kohlenstoff unter Bildung einer Carbonyl-Gruppe -(C=O)-. Bevorzugt ist weiterhin die Bindung zweier doppelt gebundener Sauerstoffatome an Schwefel unter Bildung einer Sulfonyl-Gruppe -(S=O)₂-.

Unter Halogen ist Fluor, Chlor Brom oder Iod zu verstehen.

Unter einem Halogen-C₁-C₄-Alkylrest beziehungsweise Halogen-C₁-C₄-Alkyl- ist ein C₁-C₄-Alkylrest, substituiert mit mindestens einem Halogensubstituenten, vorzugsweise mit mindestens einem Fluorsubstituenten, zu verstehen.
Bevorzugt sind Fluor-C₁-C₃-Alkyl-Reste, beispielsweise Difluormethyl-, Trifluormethyl-, 2,2,2-Trifluorethyl- oder Pentafluorethyl-.
Besonders bevorzugt sind perfluorierte Alkylreste wie Trifluormethyl- oder Pentafluorethyl-.

Unter Phenyl-C₁-C₃-alkyl- ist eine Gruppe zu verstehen, die zusammengesetzt ist aus einem gegebenenfalls substituierten Phenylrest und einer C₁-C₃-Alkyl-Gruppe, und die über die C₁-C₃-AlkylGruppe an den Rest des Moleküls gebunden ist.

Unter einem Halogen-C₁-C₄-Alkoxyrest beziehungsweise Halogen-C₁-C₄-Alkoxy- ist ein C₁-C₄-Alkoxyrest, substituiert mit mindestens einem Halogensubstituenten, vorzugsweise mit mindestens einem Fluorsubstituenten, zu verstehen.
Bevorzugt sind Fluor-C₁-C₃-Alkoxy-Reste, beispielsweise Difluormethoxy-, Trifluormethoxy- oder 2,2,2-Trifluorethoxy-.

Unter einem Halogen-C₁-C₄-Alkylthio-Rest beziehungsweise Halogen-C₁-C₄-Alkylthio- ist ein C₁-C₄-Alkylthio-Rest substituiert mit mindestens einem Halogensubstituenten, vorzugsweise mit mindestens einem Fluorsubstituenten, zu verstehen.
Bevorzugt sind Fluor-C₁-C₃-Alkylthio-Reste, insbesondere Trifluormethylthio-.

Unter einem C₁-C₄-Alkylcarbonylrest ist eine C₁-C₄-Alkyl-C(=O)-Gruppe zu verstehen. Bevorzugt ist Acetyl- oder Propanoyl-.

Unter einem C₁-C₄-Alkoxycarbonylrest ist eine C₁-C₄-Alkoxy-C(=O)-Gruppe zu verstehen. Bevorzugt ist Methoxycarbonyl-, Ethoxycarbonyl-, oder *tert*.-Butoxycarbonyl-.

Unter einem C₁-C₄-Alkoxy-C₁-C₄-Alkylrest ist ein mit C₁-C₄-Alkoxy substituierter C₁-C₄-Alkylrest zu verstehen, wie z. B.Methoxymethyl-, Methoxyethyl-, Ethoxymethyl- und Ethoxyethyl-.

Unter Aryl ist ein aus Kohlenstoffatomen aufgebautes ungesättigtes vollständig konjugiertes System zu verstehen, welches über 3, 5 oder 7 konjugierte Doppelbindungen verfügt, wie z.B. Phenyl-, Naphthyl- oder Phenantryl-. Bevorzugt ist Phenyl.

Unter Heteroaryl- sind Ringsysteme zu verstehen, die über ein aromatisch konjugiertes Ringsystem verfügen sowie mindestens ein und bis zu fünf Heteroatomen wie voranstehend definiert enthalten, enthalten. Diese Ringsysteme können über 5, 6 oder 7 Ringatome verfügen, oder im Fall von kondensierten beziehungsweise benzokondensierten Ringsystemen auch über Kombinationen aus 5- und 6-gliedrigen Ringsystemen, 5- und 5-gliedrigen Ringsystemen oder auch aus 6- und 6-gliedrigen Ringsystemen verfügen. Als Beispiel seien aufgeführt Ringsysteme wie Pyrrolyl-, Pyrazolyl-, Imidazolyl-, Triazolyl-, Tetrazolyl-, Furanyl-, Thienyl-, Oxazolyl-, Thiazolyl-, Isoxazolyl-, Oxadiazolyl-, Thiadiazolyl-, Pyridinyl-, Pyrimidinyl-, Pyrazinyl-, Triazinyl-, Oxazinyl-, Indolyl-, Benzimidazolyl-, Indazolyl-, Benzotriazolyl-, Benzothiazolyl-, Benzoxazolyl-, Benzofuranyl-, Benzothienyl-, Chinolinyl-, Isochinolinyl-, Cinnolinyl-, Chinazolinyl-, Chinoxalinyl-, Imidazopyridylyl- oder auch Benzoxazinyl-.
Bevorzugt ist 5- bis 6-gliedriges, monocyclisches Heteroaryl-, beispielsweise Pyrrolyl-, Pyrazolyl-, Imidazolyl-, Triazolyl-, Tetrazolyl-, Furanyl-, Thienyl-, Oxazolyl-, Thiazolyl-, Isoxazolyl-, Oxadiazolyl-, Thiadiazolyl-, Pyridinyl-, Pyrimidinyl-, Pyrazinyl-, Triazinyl-.

Unter C₃-C₆-Cycloalkyl, C₃-C₈-Cycloalkyl, bzw. C₅-C₈-Cycloalkyl ist ein monocyclisches, ausschließlich aus Kohlenstoffatomen aufgebautes, gesättigtes Ringsystem mit 3 bis 6, 3 bis 8 Atomen, bzw. 5 bis 8 Atomen zu verstehen. Beispiele sind Cyclopropyl-, Cyclobutyl-, Cyclopentyl-, Cyclohexyl-, Cycloheptyl- oder Cyclooctyl-.

Unter C₄-C₆-Cycloalkenyl, C₄-C₈-Cycloalkenyl, bzw. C₅-C₈-Cycloalkenyl ist ein monocyclisches, ausschließlich aus Kohlenstoffatomen aufgebautes, ein- oder mehrfach ungesättigtes, nicht-aromatisches Ringsystem mit 3 bis 6, 3 bis 8 Atomen, bzw. 5 bis 8 Atomen zu verstehen. Beispiele sind Cyclobuten-1-yl-, Cyclopenten-1-yl-, Cyclohexen-2-yl-, Cyclohexen-1-yl- oder Cycloocta-2,5-dienyl-.

Unter Heterocycloalkyl- ist ein 4- bis 8-gliedriges monocyclisches, gesättigtes Ringsystem zu verstehen, welches über 1 bis 3 Heteroatome wie voranstehend definiert, in beliebiger Kombination verfügt. Bevorzugt sind 4- bis 7-gliedrige Heterocycloalkyl-Gruppen, besonders bevorzugt sind 5-bis 6-gliedrige Heterocycloalkyl-Gruppen. Beispielhaft zu nennen sind Pyrrolidinyl-, Piperidinyl-, Tetrahydrofuranyl-, Tetrahydropyranyl-, Oxetanyl-, Azetidinyl-, Azepanyl-, Morpholinyl-, Thiomorpholinyl- oder Piperazinyl-.

Unter Heterocycloalkenyl ist ein 4- bis 8-gliedriges monocyclisches, ein- oder mehrfach ungesättigtes, nicht-aromatisches Ringsystem zu verstehen, welches über 1 bis 3 Heteroatome wie voranstehend definiert, in beliebiger Kombination verfügt. Bevorzugt sind 4- bis 7-gliedrige Heterocycloalkenyl-Gruppen, besonders bevorzugt sind 5- bis 6-gliedrige Heterocycloalkenyl-Gruppen. Beispielhaft zu nennen sind 4H-Pyranyl-, 2H-Pyranyl-, 2,5-Dihydro-1H-pyrrolyl-, [1,3]Dioxolyl, 4H-[1,3,4]Thiadiazinyl, 2,5-Dihydrofuranyl, 2,3-Dihydrofuranyl, 2,5-Dihydrothiophenyl-, 2,3-Dihydrothiophenyl-, 4,5-Dihydrooxazolyl-, or 4H-[1,4]Thiazinyl-.

Unter C₅-C₁₁ -Spirocycloalkyl bzw. C₅-C₁₁-Heterospirocycloalkyl mit einem Ersatz von 1 bis 4 Kohlenstoffatomen durch Heteroatome wie voranstehend definiert in beliebiger Kombination, ist eine Fusion aus zwei gesättigten Ringsystemen zu verstehen, die sich ein gemeinsames Atom teilen. Beispiele sind Spiro[2.2]pentyl-, Spiro[2.3]hexyl-, Azaspiro[2.3]hexyl-, Spiro[3.3]heptyl-, Azaspiro[3.3]heptyl-, Oxaazaspiro[3.3]heptyl-, Thiaazaspiro[3.3]heptyl-, Oxaspiro[3.3]heptyl-, Oxazaspiro[5.3]nonyl-, Oxazaspiro[4.3]octyl-, Oxazaspiro[5.5]undecyl-, Diazaspiro[3.3]heptyl-, Thiazaspiro[3.3]heptyl-, Thiazaspiro[4.3]octyl-, Azaspiro[5.5]decyl-, sowie die weiteren homologen Spiro[3.4]-, Spiro[4.4]-, Spiro[5.5]-, Spiro[6.6]-, Spiro[2.4]-, Spiro[2.5]-, Spiro[2.6]-, Spiro[3.5]-, Spiro[3.6]-, Spiro[4.5]-, Spiro[4.6]- und Spiro[5.6]-Systeme inklusive der durch Heteroatome modifizierten Varianten gemäß Definition. Bevorzugt ist C₆-C₈-Heterospirocycloalkyl.

Unter C₆-C₁₂-Bicycloalkyl bzw. C₆-C₁₂-Heterobicycloalkyl mit einem Ersatz von 1 bis 4 Kohlenstoffatomen durch Heteroatome wie voranstehend definiert in beliebiger Kombination, ist eine Fusion aus zwei gesättigten Ringsystemen zu verstehen, die sich gemeinsam zwei direkt benachbarte Atome teilen. Beispiele sind Bicyclo[2.2.0]hexyl-, Bicyclo[3.3.0]octyl-, Bicyclo[4.4.0]decyl-, Bicyclo[5.4.0]undecyl-, Bicyclo[3.2.0]heptyl-, Bicyclo[4.2.0]octyl-, Bicyclo[5.2.0]nonyl-, Bicyclo[6.2.0]decyl-, Bicyclo[4.3.0]nonyl-, Bicyclo[5.3.0]decyl-, Bicyclo[6.3.0]undecyl- und Bicyclo[5.4.0]undecyl-, inklusive der durch Heteroatome modifizierten Varianten wie z.B. Azabicyclo[3.3.0]octyl-, Azabicyclo[4.3.0]nonyl-, Diazabicyclo[4.3.0]nonyl-, Oxazabicyclo[4.3.0]nonyl-, Thiazabicyclo[4.3.0]nonyl- oder Azabicyclo[4.4.0]decyl- sowie die weiteren möglichen Kombinationen gemäß Definition. Bevorzugt ist C₆-C₁₀-Heterobicycloalkyl.

Unter einem verbrückten C₆-C₁₂-Ringsystem wie verbrücktes C₆-C₁₂-Cycloalkyl oder verbrücktes C₆-C₁₂-Heterocycloalkyl ist eine Fusion aus mindestens zwei gesättigten Ringen zu verstehen, die sich zwei Atome teilen, die nicht direkt benachbart zueinander sind. Dabei kann sowohl ein verbrücktes Carbocyclus (verbrücktes Cycloalkyl) entstehen als auch ein verbrückter Heterocyclus (verbrücktes Heterocycloalkyl) mit einem Ersatz von 1 bis 4 Kohlenstoffatomen durch Heteroatome wie voranstehend definiert in beliebiger Kombination. Beispiele sind Bicyclo[2.2.1]heptyl-, Azabicyclo[2.2.1]heptyl-, Oxazabicyclo[2.2.1]heptyl-, Thiazabicyclo[2.2.1]heptyl-, Diazabicyclo[2.2.1]heptyl-, Bicyclo[2.2.2]octyl-, Azabicyclo[2.2.2]octyl-, Diazabicyclo[2.2.2]octyl-, Oxazabicyclo[2.2.2]octyl-, Thiazabicyclo[2.2.2]octyl-, Bicyclo[3.2.1]octyl-, Azabicyclo[3.2.1]octyl-, Diazabicyclo[3.2.1]octyl-,
Oxazabicyclo[3.2.1]octyl-, Thiazabicyclo[3.2.1]octyl-, Bicyclo[3.3.1]nonyl-, Azabicyclo[3.3.1]nonyl-, Diazabicyclo[3.3.1]nonyl-, Oxazabicyclo[3.3.1]nonyl-, Thiazabicyclo[3.3.1]nonyl-, Bicyclo[4.2.1]nonyl-, Azabicyclo[4.2.1]nonyl-, Diazabicyclo[4.2.1]nonyl-, Oxazabicyclo[4.2.1]nonyl-, Thiazabicyclo[4.2.1]nonyl-, Bicyclo[3.3.2]decyl-, Azabicyclo[3.3.2]decyl-, Diazabicyclo[3.3.2]decyl-, Oxazabicyclo[3.3.2]decyl-, Thiazabicyclo[3.3.2]decyl- oder Azabicyclo[4.2.2]decyl- sowie die weiteren möglichen Kombinationen gemäß Definition. Bevorzugt ist verbrücktes C₆-C₁₀-Heterocycloalkyl.

Erfindungsgemäße Verbindungen sind die Verbindungen der allgemeinen Formel (I) und deren Salze, Solvate und Solvate der Salze, die von der allgemeinen Formel (I) umfassten Verbindungen der nachfolgend genannten Formeln und deren Salze, Solvate und Solvate der Salze sowie die von der allgemeinen Formel (I) umfassten, nachfolgend als Ausführungsbeispiele genannten Verbindungen und deren Salze, Solvate und Solvate der Salze, soweit es sich bei den von der allgemeinen Formel (I) umfassten, nachfolgend genannten Verbindungen nicht bereits um Salze, Solvate und Solvate der Salze handelt.

Ebenfalls als von der vorliegenden Erfindung als umfasst anzusehen ist die Verwendung der Salze der erfindungsgemäßen Verbindungen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Die vorliegende Erfindung betrifft auch Arzneimittel enthaltend die erfindungsgemäßen Verbindungen zusammen mit mindestens einem oder mehreren weiteren Wirkstoffen, insbesondere zur Prophylaxe und/oder Therapie von Tumorerkrankungen.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere. Die erfindungsgemäßen Verbindungen können am Kohlenstoffatom, an welches R⁵ und R⁶ gebunden sind, ein Asymmetriezentrum aufweisen. Sie können daher als reine Enantiomere, Racemate aber auch als Diastereomere oder deren Gemische vorliegen, wenn einer oder mehrere der in der Formel (I) beschriebenen Substituenten ein weiteres Asymmetrieelement enthält, beispielsweise ein chirales Kohlenstoffatom. Die vorliegende Erfindung umfasst deshalb auch Diastereomere und ihre jeweiligen Mischungen. Aus solchen Mischungen lassen sich die reinen Stereoisomere in bekannter Weise isolieren; vorzugsweise werden hierfür chromatographische Verfahren verwendet, insbesondere die HPLC-Chromatographie an chiraler bzw. achiraler Phase.

In der Regel inhibieren die erfindungsgemäßen Enantiomere unterschiedlich stark die Targetproteine und sind unterschiedlich aktiv in den untersuchten Krebszelllinien. Das aktivere Enantiomer ist bevorzugt, welches oft dasjenige ist, an dem das durch das an R⁵ und R⁶ gebundene Kohlenstoffatom repräsentierte Asymmetriezentrum (R)-konfiguriert ist.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Die erfindungsgemäßen Verbindungen können systemisch und/oder lokal wirken. Zu diesem Zweck kann sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.
Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die die erfindungsgemäßen Verbindungen, üblicherweise zusammen mit einem oder mehreren inerten, nichttoxischen, pharmazeutisch geeigneten Hilfsstoffen enthalten, sowie deren Verwendung zu den zuvor genannten Zwecken.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können in fester Form, zum Beispiel als Tabletten, Dragees, Pillen, Suppositorien, Kapseln, transdermale Systeme oder in halbfester Form, zum Beispiel als Salben, Cremes, Gele, Suppositorien, Emulsionen oder in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

Die erfindungsgemäßen Verbindungen eignen sich zur Prophylaxe und/oder Therapie von hyper-proliferativen Erkrankungen wie beispielsweise Psoriasis, Keloide und andere Hyperplasien, die die Haut betreffen, gutartige Prostathyperplasien (BPH), solide Tumore und hämatologische Tumore.

Als solide Tumore sind erfindungsgemäß beispielsweise Tumore behandelbar der Brust, des Respirationstraktes, des Gehirns , der Fortpflanzungsorgane, des Magen-Darmtraktes, des Urogenitaltraktes, des Auges, der Leber, der Haut, des Kopfes und des Halses, der Schilddrüse, der Nebenschilddrüse, der Knochen sowie des Bindegewebes und Metastasen dieser Tumore.

Als hämatologischeTumore sind beispielsweise behandelbar multiple Myelome, Lymphome oder Leukämien.

Als Brusttumore sind beispielsweise behandelbar Mammakarzinome mit positivem Hormonrezeptorstatus, Mammakarzinome mit negativem Hormonrezeptorstatus, Her-2 positive Mammakarzinome, Hormonrezeptor- und Her-2 negative Mammakarzinome, BRCA -assoziierte Mammakarzinome und entzündliches Mammakarzinom.

Als Tumore des Respirationstraktes sind beispielsweise behandelbar nicht-kleinzellige Bronchialkarzinome und kleinzellige Bronchialkarzinome.

Als Tumore des Gehirns sind beispielsweise behandelbar Gliome, Glioblastome, Astrozytome, Meningiome und Medulloblastome.

Als Tumore der männlichen Fortpflanzungsorgane sind beispielsweise behandelbar Prostatakarzinome, Maligne Nebenhodentumore, Maligne Hodentumore und Peniskarzinome.

Als Tumore der weiblichen Fortpflanzungsorgane sind beispielsweise behandelbar Endometriumkarzinome, Zervixkarzinome, Ovarialkarzinome, Vaginalkarzinome und Vulvarkarzinome.

Als Tumore des Magen- Darm-Traktes sind beispielsweise behandelbar Kolorektale Karzinome, Analkarzinome, Magenkarzinome, Pankreaskarzinome, Ösophagukarzinome, Gallenblasenkarzinome, Dünndarmkarzinome, Speicheldrüsenkarzinome, Neuroendokrine Tumore und Gastrointestinale Stromatumore.

Als Tumore des Urogenital-Traktes sind beispielsweise behandelbar Harnblasenkarzinome, Nierenzellkarzinome, und Karzinome des Nierenbeckens und der ableitenden Harnwege.

Als Tumore des Auges sind beispielsweise behandelbar Retinoblastome und Intraokulare Melanome.

Als Tumore der Leber sind beispielsweise behandelbar Hepatozelluläre Karzinome und Cholangiozelluläre Karzinome.

Als Tumore der Haut sind beispielsweise behandelbar Maligne Melanome, Basaliome, Spinaliome, Kaposi-Sarkome und Merkelzellkarzinome.

Als Tumore des Kopfes und Halses sind beispielsweise behandelbar Larynxkarzinome und Karzinome des Pharynx und der Mundhöhle.

Als Sarkome sind beispielsweise behandelbar Weichteilsarkome und Osteosarkome.

Als Lymphome sind beispielsweise behandelbar Non-Hodgkin-Lymphome, Hodgkin-Lymphome, Kutane Lymphome, Lymphome des zentralen Nervensystems und AIDS-assoziierte Lymphome.

Als Leukämien sind beispielsweise behandelbar Akute myeloische Leukämien, Chronische myeloische Leukämien, Akute lymphatische Leukämien, Chronische lymphatische Leukämien und Haarzellleukämien.

Vorteilhaft können die erfindungsgemäßen Verbindungen verwendet werden zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Besonders vorteilhaft können die erfindungsgemäßen Verbindungen verwendet werden zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Die erfindungsgemäßen Verbindungen eignen sich auch zur Prophylaxe und/oder Therapie von benignen hyperproliferativen Krankheiten wie zum Beispiel Endometriose, Leiomyom und benigne Prostatahyperplasie.

Die erfindungsgemäßen Verbindungen eignen sich auch zur Prophylaxe und/oder Therapie von systemischen inflammatorischen Krankheiten, insbesondere LPS-induzierter endotoxischer Schock und/oder Bakterien-induzierte Sepsis.

Die erfindungsgemäßen Verbindungen eignen sich auch zur Prophylaxe und/oder Therapie von inflammatorischen oder Autoimmunerkrankungen wie zum Beispiel:
- Lungenerkrankungen, die mit entzündlichen, allergischen und/oder proliferativen Prozessen einhergehen: chronisch obstruktive Lungenerkrankungen jeglicher Genese, vor allem Asthma bronchiale; Bronchitis unterschiedlicher Genese; alle Formen der restriktiven Lungenerkrankungen, vor allem allergische Alveolitis; alle Formen des Lungenödems, vor allem toxisches Lungenödem; Sarkoidosen und Granulomatosen, insbesondere Morbus Boeck
- Rheumatische Erkrankungen/Autoimmunerkrankungen/Gelenkerkrankungen, die mit entzündlichen, allergischen und/oder proliferativen Prozessen einhergehen: alle Formen rheumatischer Erkrankungen, insbesondere rheumatoide Arthritis, akutes rheumatisches Fieber, Polymyalgia rheumatica; reaktive Arthritis; entzündliche Weichteilerkrankungen sonstiger Genese; arthritische Symptome bei degenerativen Gelenkerkankungen (Arthrosen); traumatische Arthritiden; Kollagenosen jeglicher Genese, z.B. systemischer Lupus erythematodes, Sklerodermie, Polymyositis, Dermatomyositis, Sjögren-Syndrom, Still-Syndrom, Felty-Syndrom
- Allergien, die mit entzündlichen und/oder proliferativen Prozessen einhergehen: alle Formen allergischer Reaktionen, z.B. Quincke Ödem, Heuschnupfen, Insektenstich, allergische Reaktionen auf Arzneimittel, Blutderivate, Kontrastmittel etc., anaphylaktischer Schock, Urtikaria, Kontaktdermatitis
- Gefäßentzündugen (Vaskulitiden): Panarterilitis nodosa, Arterilitis temporalis, Erythema nodosum
- Dermatologische Erkrankungen, die mit entzündlichen, allergischen und/oder proliferativen Prozessen einhergehen: atopische Dermatitis; Psoriasis; Pityriasis rubra pilaris; erythematöse Erkrankungen, ausgelöst durch unterschiedliche Noxen, z.B. Strahlen, Chemikalien, Verbrennungen etc.; bullöse Dermatosen; Erkrankungen des lichenoiden Formenkreises; Pruritus; Seborrhoisches Ekzem; Rosacea; Pemphigus vulgaris; Erythema exsudativum multiforme; Balanitis; Vulvitis; Haarausfall wie Alopecia areata; kutane T-Zell Lymphome
- Nierenerkrankungen, die mit entzündlichen, allergischen und/oder proliferativen Prozessen einhergehen: nephrotisches Syndrom; alle Nephritiden
- Lebererkrankungen, die mit entzündlichen, allergischen und/oder proliferativen Prozessen einhergehen: akuter Leberzellzerfall; akute Hepatitis unterschiedlicher Genese, z.B. viral, toxisch, arneimittelinduziert; chronisch aggressive und/oder chronisch intermittierende Hepatitis
- Gastrointestinale Erkrankungen, die mit entzündlichen, allergischen und/oder proliferativen Prozessen einhergehen: regionale Enteritis (Morbus Crohn); Colitis ulcerosa; Gastritis; Refluxoesophagitis; Gastroenteritiden anderer Genese, z.B. einheimische Sprue
- Proktologische Erkrankungen, die mit entzündlichen, allergischen und/oder proliferativen Prozessen einhergehen: Analekzem; Fissuren; Hämorrhoiden; idiopatische Proktitis
- Augenerkrankungen, die mit entzündlichen, allergischen und/oder proliferativen Prozessen einhergehen: allergische Keratitis, Uveitis, Iritis; Konjuktivitis; Blepharitis; Neuritis nervi optici; Chlorioditis; Opthalmia sympathica
- Erkrankungen des Hals-Nasen-Ohren-Bereiches, die mit entzündlichen, allergischen und/oder proliferativen Prozessen einhergehen: allergische Rhinitis, Heuschnupfen; Otitis externa, z.B. bedingt durch Kontaktexem, Infektion etc.; Otitis media
- Neurologische Erkrankungen, die mit entzündlichen, allergischen und/oder proliferativen Prozessen einhergehen: Hirnödem, vor allem Tumor-bedingtes Hirnödem; Multiple Sklerose; akute Encephalomyelitis; Meningitis; verschiedene Formen von Krampfanfällen, z.B. BNS-Krämpfe
- Bluterkrankungen, die mit entzündlichen, allergischen und/oder proliferativen Prozessen einhergehen: erworbene hämolytische Anämie; idiopathische Thrombozytopenie
- Tumorerkrankungen, die mit entzündlichen, allergischen und/oder proliferativen Prozessen einhergehen: akute lymphatische Leukämie; maligne Lymphome; Lymphogranulomatosen; Lymphosarkome; ausgedehnte Metastasierungen, vor allem bei Mamma-, Bronchial- und Prostatakarzinom
- Endokrine Erkrankungen, die mit entzündlichen, allergischen und/oder proliferativen Prozessen einhergehen: endokrine Orbitopathie; thyreotoxische Krise; Thyreoditis de Quervain; Hashimoto Thyreoditis; Morbus Basedow
- Organ- und Gewebstransplantationen, Graft-versus-Host disease
- Schwere Schockzuständen, z.B. anaphylaktischer Schock, systemic inflammatory response syndrome (SIRS)
- Substitutionstherapie bei: angeborene primäre Nebenniereninsuffizienz, z.B. kongenitales adrenogenitales Syndrom; erworbene primäre Nebenniereninsuffizienz, z.B. Morbus Addison, autoimmune Adrenalitis, postinfektiös, Tumoren, Metastasen, etc; angeborene sekundäre Nebenniereninsuffizienz, z.B. kongenitaler Hypopituitarismus; erworbene sekundäre Nebenniereninsuffizenz, z.B. postinfektiös, Tumoren, etc
- Emesis, die mit entzündlichen, allergischen und/oder proliferativen Prozessen einhergehen, z.B. in Kombination mit einem 5-HT3-Antagonisten bei Zytostatika-bedingten Erbrechen
- Schmerzen bei entzündlicher Genese, z.B. Lumbago.

Die erfindungsgemäßen Verbindungen eignen sich auch für die Behandlung von viralen Erkrankungen, wie zum Beispiel Infektionen die verursacht sind durch Papilloma-Viren, Herpes-Viren, Epstein-Barr-Viren, Hepatitis B- oder C-Viren, und humane Immunschwäche-Viren.

Die erfindungsgemäßen Verbindungen eignen sich auch für die Behandlung von Atherosklerose, Dyslipidemie, Hypercholesterolemie, Hypertriglyceridämie, perifere Gefäßerkrankungen, kardiovaskuläre Erkrankungen, Angina, pectoris, Ischemie, Schlaganfall, Myokardinfarkt, angioplastische Restenose, Bluthochdruck, Thrombose, Adipositas, Endotoxemie.

Die erfindungsgemäßen Verbindungen eignen sich auch für die Behandlung von neurodegenerativen Krankheiten wie zum Beispiel multiple Sklerose, Alzheimer's Krankheit und Parkinson's Krankheit.

Diese Erkrankungen sind gut charakterisiert im Menschen, existieren aber auch bei anderen Säugetieren.

Ein weiterer Gegenstand der vorliegenden Anmeldung sind die erfindungsgemäßen Verbindungen zur Verwendung als Arzneimittel, insbesondere zur Prophylaxe und/oder Therapie von Tumorerkrankungen.

Ein weiterer Gegenstand der vorliegenden Anmeldung sind die erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung sind die erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der Erfindung ist die Verwendung der erfindungsgemäßen Verbindungen zur Herstellung eines Arzneimittels.

Ein weiterer Gegenstand der vorliegenden Anmeldung ist die Verwendung der erfindungsgemäßen Verbindungen zur Herstellung eines Arzneimittels zur Prophylaxe und/oder Therapie von Tumorerkrankungen.

Ein weiterer Gegenstand der vorliegenden Anmeldung ist die Verwendung der erfindungsgemäßen Verbindungen zur Herstellung eines Arzneimittels zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung ist die Verwendung der erfindungsgemäßen Verbindungen zur Herstellung eines Arzneimittels zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der vorliegenden Anmeldung ist die Verwendung der erfindungsgemäßen Verbindungen zur Prophylaxe und/oder Therapie von Tumorerkrankungen.

Ein weiterer Gegenstand der vorliegenden Anmeldung ist die Verwendung der erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung ist die Verwendung der erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der vorliegenden Anmeldung sind pharmazeutische Formulierungen in Form von Tabletten enthaltend eine der erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung sind pharmazeutische Formulierungen in Form von Tabletten enthaltend eine der erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der Erfindung ist die Verwendung der erfindungsgemäßen Verbindungen zur Behandlung von Erkrankungen, die mit proliferativen Prozessen einhergehen.

Ein weiterer Gegenstand der Erfindung ist die Verwendung der erfindungsgemäßen Verbindungen zur Behandlung von benignen Hyperplasien, inflammatorischen Erkrankungen, autoimmunen Erkrankungen, Sepsis, viralen Infektionen, Gefäßerkrankungen und neurodegenerativen Erkrankungen.

Die erfindungsgemäßen Verbindungen können allein oder bei Bedarf in Kombination mit einer oder mehreren weiteren pharmakologisch wirksamen Substanzen eingesetzt werden, solange diese Kombination nicht zu unerwünschten und inakzeptablen Nebenwirkungen führt. Ein weiterer Gegenstand der vorliegenden Erfindung sind daher Arzneimittel, enthaltend eine erfindungsgemäße Verbindung und einen oder mehrere weitere Wirkstoffe, insbesondere zur Prophylaxe und/oder Therapie der zuvor genannten Erkrankungen.

Beispielsweise können die erfindungsgemäßen Verbindungen mit bekannten anti-hyper-proliferativen, zytostatischen oder zytotoxischen chemischen und biologischen Substanzen zur Behandlung von Krebserkrankungen kombiniert werden. Die Kombination der erfindungsgemäßen Verbindungen mit anderen für die Krebstherapie gebräuchlichen Substanzen oder auch mit der Strahlentherapie ist besonders angezeigt.

Als geeignete Kombinationswirkstoffe seien beispielhaft genannt, ohne dass diese Aufzählung abschließend wäre:
Abiraterone acetate, Abraxane, Acolbifen, Actimmun, Actinomycin D (Dactinomycin), Afatinib, Affinitak,Afinitor, Aldesleukin, Alendronsäure, Alfaferon, Alitretinoin, Allopurinol, Aloprim, Aloxi, Alpharadin, Altretamin, Amino¬glutethimid, Aminopterin, Amifostin, Amrubicin, Amsacrin, Anastrozol, Anzmet, Apatinib, Aranesp, Arglabin, Arsen-trioxid, Aromasin, Arzoxifen, Asoprisnil, L-Asparaginase, Atamestan, Atrasentan, Avastin, Axitinib, 5-Azacytidin, Azathioprin, BCG oder tice-BCG, Bendamustin, Bestatin, Beta-methason-Acetat, Betamethason-Natriumphosphat, Bexaroten, Bicalutamid, Bleomycin-Sulfat, Broxuridin, Bortezomib, Bosutinib, Busulfan, Cabazitaxel, Calcitonin, Campath, Camptothecin, Capecitabin, Carboplatin, Carfilzomib, Carmustin, Casodex,CCI-779, CDC-501, Cediranib, Cefeson, Celebrex, Celmoleukin, Cerubidin, Cediranib, Chlorambucil, Cisplatin, Cladribin, Clodronsäure, Clofarabin, Colaspase, Corixa, Crisnatol, Crizotinib, Cyclophosphamid, Cyproterone-Acetat, Cytarabin, Dacarbazin, Dactinomycin, Dasatinib, Daunorubicin, DaunoXome, Decadron, Decadron-Phosphat, Decitabin, Degarelix, Delestrogen, Denileukin Diftitox, Depomedrol, Deslorelin, Dexrazoxan, Diethylstilbestrol, Diflucan, 2',2'-Difluordeoxycytidin, DN-101, Docetaxel, Doxifluridin, Doxo¬rubicin (Adriamycin), Dronabinol, dSLIM, Dutasterid, DW-166HC, Edotecarin, Eflornithin, Eligard, Elitek, Ellence, Emend, Enzalutamide, Epirubicin, Epoetin-alfa, Epogen, Epothilon und seine Derivate, Eptaplatin, Ergamisol, Erlotinib, Erythro-Hydroxynonyladenin, Estrace, Estradiol, Estramustin-Natriumphosphat, Ethinylestradiol, Ethyol, Etidronsäure, Etopophos, Etoposid, Everolimus, Exatecan, Exemestan, Fadrozol, Farston, Fenretinid, Filgrastim, Finasterid, Fligrastim, Floxuridin, Fluconazol, Fludarabin, 5-Fluordeoxyuridin-Monophosphat, 5-Fluoruracil (5-FU), Flu¬oxy¬mesteron, Flutamid, Folotin, Formestan, Fosteabin, Fotemustin, Fulvestrant, Gammagard, Gefitinib, Gemcitabin, Gemtuzumab, Gleevec, Gliadel, Goserelin, Gossypol, Granisetron-Hydrochlorid, Hexamethylmelamin, Histamin-Dihydrochlorid, Histrelin, Holmium-166-DOTPM, Hycamtin, Hydrocorton, erythro-Hydroxynonyladenin, Hydroxyharnstoff, Hydroxyprogesteronecaproat, Ibandronsäure, Ibritumomab Tiuxetan, Idarubicin, Ifosfamid, Imatinib, Iniparib, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interleukin-2, Intron A, Iressa, Irinotecan, Ixabepilon, Keyhole limpet Hemocyanin, Kytril, Lanreotid, Lapatinib, Lasofoxifen, Lenalidomid, Lentinan-Sulfat, Lestaurtinib, Letrozol, Leucovorin, Leuprolid, Leuprolid-Acetat, Levamisol, Levofolinsäure-Calciumsalz, Levothroid, Levoxyl, Libra, liposomales MTP-PE, Lomustin, Lonafarnib, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Minodronat, Miproxifen, Mitomycin C, Mitotan, Mitoxantron, Modrenal, MS-209, MX-6, Myocet, Nafarelin, Nedaplatin, Nelarabine, Nemorubicin, Neovastat, Neratinib, Neulasta, Neumega, Neupogen, Nilotimib, Nilutamid, Nimustin, Nolatrexed, Nolvadex, NSC-631570, Obatoclax, Oblimersen, OCT-43, Octreotid, Olaparib, Ondansetron-Hydrochlorid, Onko-TCS, Orapred, Osidem, Oxaliplatin, Paclitaxel, Pamidronat-Dinatrium, Pazopanib, Pediapred, Pegaspargase, Pegasys, Pemetrexed, Pentostatin, N-Phosphono-acetyl-L-Aspartat, Picibanil, Pilocarpin-Hydrochlorid, Pirarubicin, Plerixafor, Plicamycin, PN-401, Porfimer-Natrium, Prednimustin, Prednisolon, Prednison, Premarin, Procarbazin, Procrit, QS-21, Quazepam, R-1589, Raloxifene, Raltitrexed, Ranpirnas, RDEA119, Rebif, Regorafenib, 13-cis-Retinsäure, Rhenium-186-Etidronat, Rituximab, Roferon-A, Romidepsin, Romurtid, Ruxolitinib, Salagen, Salinomycin, Sandostatin, Sargramostim, Satraplatin, Semaxatinib, Semustin, Seocalcitol, Sipuleucel-T, Sizofiran, Sobuzoxan, Solu-Medrol, Sorafenib, Streptozocin, Strontium-89-chlorid, Sunitinib, Synthroid, T-138067, Tamoxifen, Tamsulosin, Tarceva, Tasonermin, Tastolacton, Taxoprexin, Taxoter, Teceleukin, Temozolomid, Temsirolimus, Teniposid, Testosteron-Propionat, Testred, Thalidomid, Thymosin-alpha-1, Thioguanin, Thiotepa, Thyrotropin, Tiazorufin, Tiludronsäure, Tipifarnib, Tirapazamin, TLK-286, Toceranib, Topotecan, Toremifen, Tositumomab, Tastuzumab, Teosulfan, TransMID-107R, Tretinoin, Trexall, Trimethylmelamin, Trimetrexat, Triptorelin-Acetat, Triptorelin-Pamoat, Trofosfamid, UFT, Uridin, Valrubicin, Valspodar, Vandetanib, Vapreotid, Vatalanib, Vemurafinib, Verte-porfin, Vesnarinon, Vinblastin, Vincristin, Vindesin, Vinflumin, Vinorelbin, Virulizin, Vismodegib, Xeloda, Z-100, Zinecard, Zinostatin-Stimalamer, Zofran, Zoledronsäure
   Insbesondere lassen sich die erfindungsgemäßen Verbindungen mit Antikörpern wie z.B. Aflibercept, Alemtuzumab, Bevacizumab, Brentuximumab, Catumaxomab, Cetuximab, Denosumab, Edrecolomab, Gemtuzumab, Ibritumomab, Ipilimumab, Ofatumumab, Panitumumab, Pertuzumab, Rituximab, Tositumumab oder Trastuzumabsowie mit rekombinanten Proteinen kombinieren.

Insbesondere können die erfindungsgemäßen Verbindungen in Kombination mit gegen die Angiogenese gerichtete Therapien wie z.B. Bevacizumab, Axitinib, Regorafenib, Cediranib, Sorafenib, Sunitinib, Lenalidomid oder Thalidomid zum Einsatz kommen.

Kombinationen mit Antihormonen und steroidalen metabolischen Enzyminhibitoren sind wegen ihres günstigen Nebenwirkungsprofils besonders geeignet.

Kombinationen mit P-TEFb- und CDK9-Inhibitoren sind wegen der möglichen synergistischen Effekte ebenfalls besonders geeignet.

Generell können mit der Kombination der erfindungsgemäßen Verbindungen mit anderen, zytostatisch oder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Verbindung mit einer Strahlentherapie und/oder einer chirurgischen Intervention eingesetzt werden.

### Herstellung der erfindungsgemäßen Verbindungen:

In der vorliegenden Beschreibung bedeuten:
NMR-Signale werden mit ihrer jeweils erkennbaren Multiplizität bzw. deren Kombinationen angegeben. Dabei bedeutet s = Singulett, d = Dublett, t = Triplett, q = Quartett, qi = Quintett, sp = Septett, m = Multiplett, b = breites Signal. Signale mit kombinierter Multiplizität werden beispielsweise angegeben als dd = Dublett vom Dublett.
   - CDCl₃: Deuterochloroform
   - dba: Dibenzylidenaceton
   - DMF: *N,N*-Dimethylformamid
   - DMSO-d6: deuteriertes Dimethylsulfoxid
   - DMSO: Dimethylsulfoxid
   - HATU: (7-Aza-1 H-benzotriazole-1-yl)-1,1, 3,3-tetramethyluronium hexafluorophosphat
   - RP-HPLC: Reversed Phase Hochdruckflüssigkeitschromatographie
   - RT: Raumtemperatur
   - THF: Tetrahydrofuran
   - HBTU: O-Benzotriazol-N,N,N',N' -tetramethyl-uronium-hexafluor-phosphat
   - PyBOB: (Benzotriazol-1-yl)-oxytripyrrolidinophosphonium hexafluorophosphat)
   - T3P: 2,4,6-Tripropyl-1,3,5,2,4,6-trioxatriphosphorinan-2,4,6-trioxid
   - LCMS: Flüssigchromatographie gekoppelt mit Massenspektronmetrie
   - CHAPS: 3-{Dimethyl[3-(4-{5,9,16-trihydroxy-2,15-dimethyltetracyclo [8.7.0.0^{2,7}.0^{11,15}]heptadecan-14-yl}pentanamido)propyl]-azaniumyl}propan-1-sulfonat
   - (+)-BINAP: (R)-(+)-2,2'-Bis(diphenylphosphino)-1,1'-binaphthyl
   - (±)-BINAP: 2,2'-Bis(diphenylphosphino)-1,1'-binaphthyl (racemisch)
   - TBTU: (Benzotriazol-1-yloxy)bisdimethylaminomethyliumfluoroborat
   - DCC: Dicyclohexylcarbodiimid

### Allgemeine Beschreibung der Herstellung der erfindungsgemäßen Verbindungen der allgemeinen Formel(I)

Die in Schema 1 gezeigten erfindungsgemäßen Verbindungen der Formeln (Ia), (Ib) und (Ic) lassen sich herstellen über Synthesewege, die im Folgenden beschrieben werden. Die genannten Formeln stellen verschiedene Teilmengen der allgemeinen Formel (I) dar, in denen A, R², R³, R⁴, R⁵, R⁶, R⁷, R⁸, R⁹ und n definiert sind wie für die allgemeine Formel (I). In Carboxamiden der Formel (Ia) steht eine Gruppe -C(=O)NR⁸R⁹ an der Stelle von R¹; in Sulfonamiden der Formel (Ib)
steht -S(=O)ₐNR⁸R⁹ an der Stelle von R¹, und in Verbindungen (Ic) schließlich steht HetAr, das 5-gliedriges, monocyclisches Heteroaryl- wie in Formel (I) für R¹ definiert bedeutet, an der Stelle von R¹.

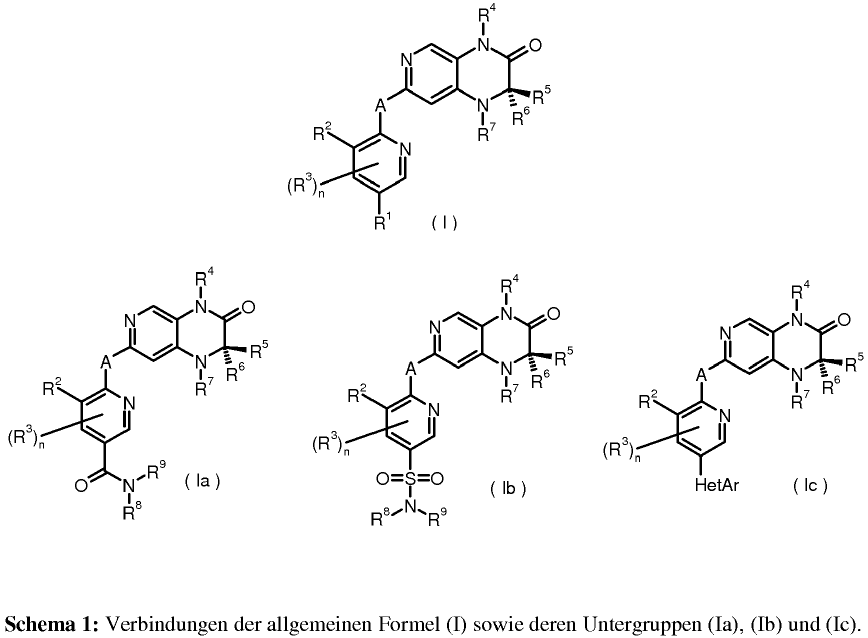

Zusätzlich zu den nachfolgend besprochenen Synthesequenzen können, entsprechend den allgemeinen Kenntnissen des Fachmannes in der Organischen Chemie, auch weitere Synthesewege für die Synthese von erfindungsgemäßen Verbindungen der allgemeinen Formel (I) beschritten werden. Die Reihenfolge der in den nachfolgenden Schemata gezeigten Syntheseschritte ist nicht bindend, und Syntheseschritte aus verschiedenen der nachfolgend gezeigten Schemata können gegebenenfalls zu neuen Sequenzen kombiniert werden. Zusätzlich können Interkonversionen der Substituenten R², R³, R⁴, R⁵, R⁶, R⁷, R⁸, R⁹ vor oder nach den gezeigten Synthesestufen durchgeführt werden. Beispiele für solche Umwandlungen sind die Einführung oder Abspaltung von Schutzgruppen, Reduktion oder Oxidation funktioneller Gruppen, Halogenierung, Metallierung, metallkatalysierte Kupplungsreaktionen, Substitutionsreaktionen oder weitere dem Fachmann bekannte Umsetzungen. Diese Reaktionen schließen Umsetzungen ein, welche eine funktionelle Gruppe einführen, die weitere Umwandlung von Substituenten ermöglicht. Geeignete Schutzgruppen sowie Methoden zu ihrer Einführung und Abspaltung sind dem Fachmann bekannt (siehe z.B. T.W. Greene und P.G.M. Wuts in: Protective Groups in Organic Synthesis, 3. Auflage, Wiley 1999). Weiterhin ist die Zusammenfassung zweier oder mehrerer Reaktionsschritte ohne zwischenzeitliche Aufarbeitung in einer dem Fachmann bekannten Weise möglich (z.B. in sogenannten "Eintopf "-Reaktionen).

Schema 2 illustriert den Aufbau von Amiden der Formel (V) aus einfachen Pyridinderivaten wie 5-Amino-2,4-dichloropyridin ((II), CAS-Nr. 7321-93-9). Für die Herstellung von (III) aus (II) kann eine Vielzahl an Methoden zur Herstellung von Amiden ausgehend von den Azido-Carbonsäuren der Formel (IIa), in denen R⁵ und R⁶ definiert sind wie für die allgemeine Formel (I), verwendet werden. So können für den Fachmann bekannte Kopplungsreagenzien wie TBTU, HATU oder DCC eingesetzt werden. Ebenfalls eignet sich die Umsetzung der zum Einsatz kommenden Azido-Carbonsäuren mit einem anorganischen Säurechlorid wie Thionylchlorid, Phosphoroxychlorid oder Oxalylchlorid und anschließender Zugabe des Pyridinamins. Die Herstellung der benötigten Azido-Carbonsäuren ist in der Literatur beschrieben (Chem Eur J (2010), 16, p7572 ff, D. Tietze et al.; J Org Chem (2010), 75, p6532ff, Katritzky et al.). Die Handhabung der Carbonsäureazide ist mit größter Vorsicht durchzuführen, da diese sich explosionsartig zersetzten können. Ebenso sollte man auf Lagerung der zur Azid-Einführung benötigten Reagenzien verzichtet werden. Diese Aspekte werden bei Katritzky et al. diskutiert.
Zur Reduktion der Azidogruppe in (III), die zu Aminen der Formel (IV) führt, kann man die Reaktion mit Trialkyl- oder Triarylphosphinen nach Staudinger durchführen (Tetrahedron (2012), 68, p697ff, Laschat et al.). Geeignet ist z.B. Trimethylphosphin. Die Isolation der Amine (IV) kann als freie Base oder, vorteilhaft, in Salzform, etwa als Hydrochlorid, erfolgen. Dazu wird das rohe Amin der Formel (IV) in einem unpolaren Lösungsmittel, beispielsweise Diethylether gelöst, und durch Zugabe einer Säure, beispielsweise von Chlorwasserstoff, als Salz ausgefällt. Die weitere Umsetzung zu Verbindungen der Formel (V) unter Einführung des Restes R⁷, der definiert ist wie für die allgemeine Formel (I), kann bevorzugt durch die für den Fachmann bekannte reduktive Aminierung durchgeführt werden (für repräsentative Vorschriften siehe z.B. US2010/105906 A1). Dabei wird das primäre Amin (IV), als freie Base oder in Salzform, mit einem für die Einführung von R⁷ geeigneten Aldehyd oder Keton *in situ* zu einem Imin umgesetzt und dieses anschließend durch Zugabe eines geeigneten Reduktionsmittels wie Natriumtriacetoxyborhydrid zum sekundären Amin der Formel (V) transformiert.

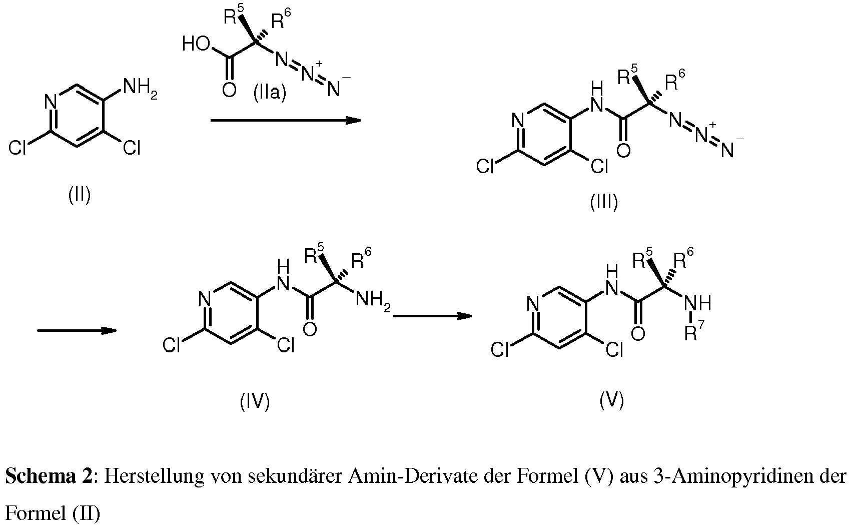

Einen alternativen Zugang zu Zwischenprodukten der Formel (V), in denen R⁵, R⁶ und R⁷ definiert sind wie in der allgemeinen Formel (I), zeigt Schema 2a. Hierin werden einfache Pyridinderivate wie 5-Amino-2,4-dichloropyridin ((II), CAS-Nr. 7321-93-9) mit Bromocarbonsäurehalogeniden der Formel (IIb), in denen LG für Halogen, bevorzugt Chlor oder Brom, steht, und R⁵ und R⁶ definiert sind wie in der allgemeinen Formel (I), in dem Fachmann geläufiger Weise zur Umsetzung gebracht. Die resultierenden alpha-Brom-Carboxamide der Formel (IIIa) werden nachfolgend mit primären Aminen R⁷-NH₂, in denen R⁷ definiert ist wie in der allgemeinen Formel (I), und die im Regelfall käuflich sind oder dem Fachmann bekannt sind, in einer nucleophilen Substitutionsreaktion zu den Zwischenprodukten der Formel V umgesetzt.

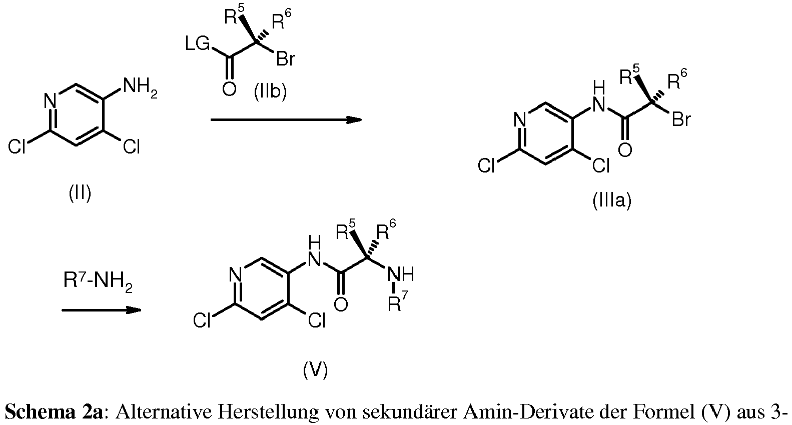

### Aminopyridinen der Formel (II)

Wie in Schema 3 gezeigt, lassen sich die sekundären Amine der Formel (V) durch Cyclisierung zu Dihydropyridopyrazinonen der Formel (VI) umsetzen (für weitere Zugänge zu Intermediaten der Formel (VI) sei auch verwiesen auf US 2006/009457). Dazu kann man Verbindungen der Formel (V) in Anwesenheit einer geeigneten Base unter erhöhter Temperatur umsetzen (siehe dazu auch WO2010/96426 A2, Example 16). Die nachfolgende Alkylierung zu Verbindungen (VII) kann erfolgen durch Umsetzung mit R⁴-LG, worin R⁴ definiert ist wie in der allgemeinen Formel (I) und LG für eine Abgangsgruppe, bevorzugt Iodid, steht, in Gegenwart einer geeigneten Base wie Natriumhydrid, nach für den Fachmann bekannten Bedingungen. Die weitere Umsetzung der resultierenden Verbindungen der Formel (VII) zu den Esterderivaten (VIII) kann erfolgen durch Umsetzung mit Aminopyridinen der Formel (VIIa), in denen A, R², R³ und n definiert sind wie in der allgemeinen Formel I, und in denen R^{E} für C₁-C₆-Alkyl steht, in einer Palladium-katalysierten Kupplungsreaktion nach Buchwald und Hartwig (siehe beispielsweise J. Organomet. Chem. (1999), 576, p125ff). Als Palladiumquelle eignen sich hier z.B. Palladiumacetat oder Palladium-(dba)-Komplexe, wie zum Beispiel Pd₂(dba)₃ (CAS-Nr. 51364-51-3 bzw. 52409-22-0). Der Umsatz hängt dabei stark von den verwendeten Liganden ab. Die im Experimentalteil aufgeführten Beispiele ließen sich so z.B. durch die Verwendung von (+)-BINAP erhalten (vgl. auch US2006/009457 A1). Die Aminopyridine der Formel (VIIa) sind teilweise käuflich oder können unter Verwendung dem Fachmann bekannter Methoden hergestellt werden.

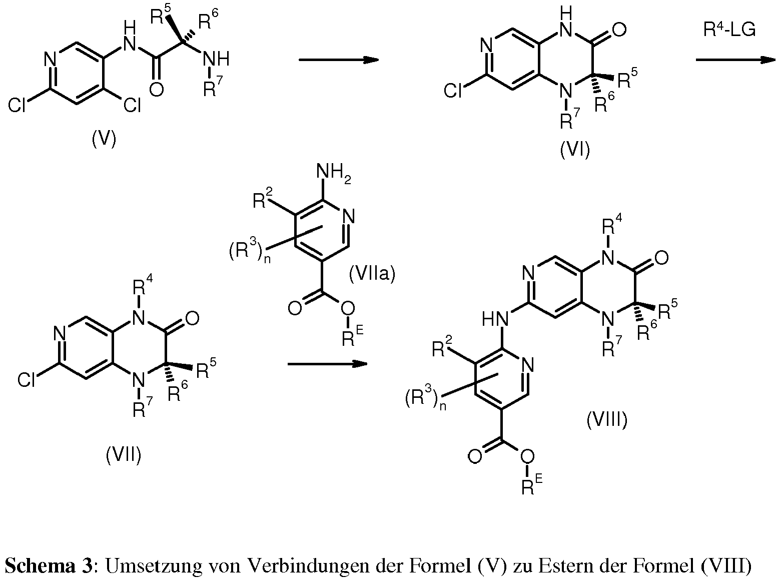

Die Herstellung von Carboxamiden der allgemeinen Formel (Ia) kann entsprechend Schema 4 mittels Hydrolyse der jeweiligen Ester der Formel (VIII) zu den entsprechenden Carbonsäuren der Formel (IX) nach für den Fachmann bekannten Methoden erfolgen. Diese Umsetzungen lassen sich bevorzugt unter Verwendung von Alkalihydroxiden wie Lithiumhydroxid, Natriumhydroxid oder Kaliumhydroxid in wässrigen alkoholischen Lösungen durchführen.
Die so erhaltenen Carbonsäuren (IX) lassen sich in die erfindungsgemäßen Carboxamide der allgemeinen Formel (Ia) überführen durch Umsetzung mit den in der Regel kommerziell erhältlichen, beispielsweise mit den in den Ausführungsbeispielen angegebenen, Aminen der Formel R⁸R⁹NH, in denen R⁸ und R⁹ definiert sind wie für die allgemeine Formel (I), unter zusätzlicher Aktivierung mit einer Methode, wie sie dem Fachmann allgemein bekannt ist. Als mögliche Methoden seien hier genannt die Verwendung von HATU, HBTU, PyBOB oder T3P unter Zusatz einer geeigneten Base. Die Umwandlung der Carbonsäuren in ihre Amide wird allgemein beschrieben in Referenzbüchern wie "Compendium of Organic Synthetic Methods", Band I-VI (Wiley Interscience) oder "The Practice of Peptide Synthesis", Bodansky (Springer Verlag).

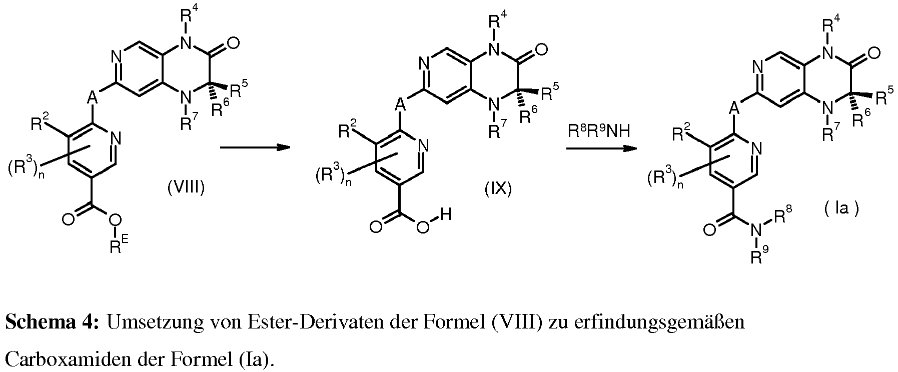

Die Herstellung der erfindungsgemäßen Verbindungen gemäß Formel (Ib) mit einer Sulfonamid-Gruppe an der Stelle von R¹ kann erfolgen gemäß Schema 5. Hierbei können Verbindungen der Formel (VII), in denen das Chlor auch durch Brom oder eine andere Abgangsgruppe ersetzt sein kann, in ähnlicher Weise wie in Schema 3 für die Umsetzung von (VII) zu (VIII) diskutiert, mit Verbindungen der Formel (X), in denen A, R², R³, R⁸, R⁹ und n definiert sind wie in der allgemeinen Formel (I), in einer Palladium-katalysierten Kupplungsreaktion nach Buchwald und Hartwig direkt zu den erfindungsgemäßen Verbindungen der Formel (Ib) umgesetzt werden. (siehe z.B. J. Med. Chem. (1996), 39, p904ff., T. R. Jones et al.). Verbindungen der Formel (X) sind kommerziell erhältlich oder können über für den Fachmann bekannte Methoden hergestellt werden. 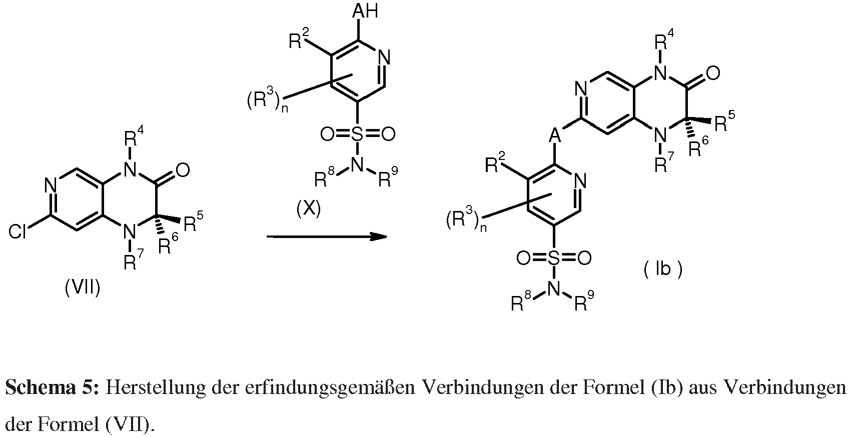 In analoger Weise kann diese Methode, wie in Schema 6 gezeigt, auch als alternative Methode für die Herstellung von Carboxamiden der allgemeinen Formel (Ia) verwendet werden, indem man die Sulfonamid-Intermediate (X) durch die analogen Carboxamide (XI) ersetzt, in denen A, R², R³, R⁸, R⁹ und n definiert sind wie in der allgemeinen Formel (I).

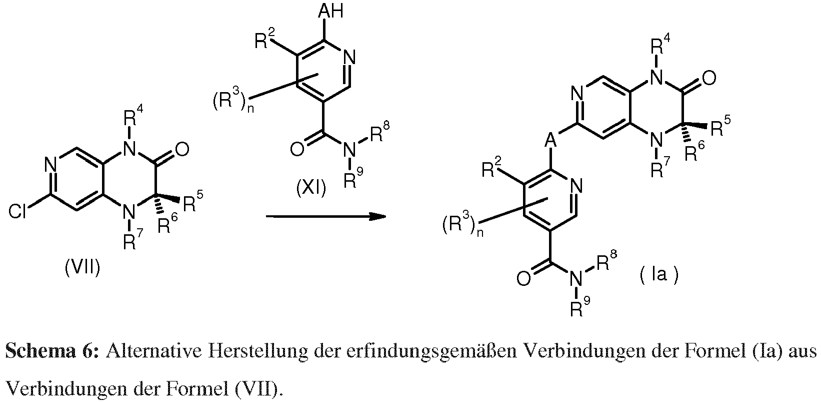

Weiterhin können in ebenfalls analoger Weise aus den halogenierten Zwischenprodukten wie (VII) durch Umsetzung mit Verbindungen der Formel (XII), in denen A, R², R³ und n definiert sind wie in der allgemeinen Formel (I), und in denen HetAr für 5-gliedriges monocyclisches Heteroraryl-, wie in Formel (I) für R¹ definiert, steht, erfindungsgemäße Verbindungen der Formel (Ic) erhalten werden, wie in Schema 7 gezeigt:

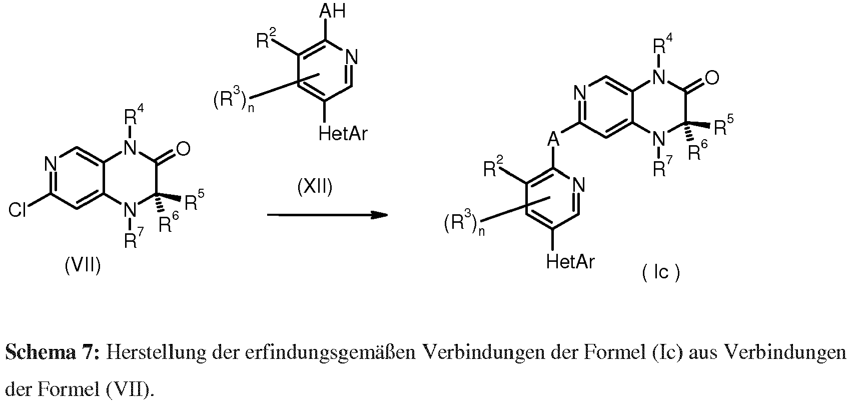

Verbindungen der Formel (XII) sind gegebenenfalls käuflich beziehungsweise dem Fachmann bekannt. Erfindungsgemäße Verbindungen der Formel (Ic) sind weiterhin zugänglich, indem man, wie in Schema 8 gezeigt, Zwischenprodukte der Formel (XIII), die mit den voranstehend beschriebenen Methoden hergestellt werden können und in denen A, R², R³, R⁴, R⁵, R⁶, R⁷ und n definiert sind wie in der allgemeinen Formel (I), und in denen R^{Hal} für ein Halogen, vorzugsweise Brom oder Iod steht, in einer dem Fachmann geläufigen Suzuki-Kupplung mit einer heteroaromatischen Boronsäure oder einem entsprechendem Boronsäureester, worin HetAr für 5-gliedriges monocyclisches Heteroraryl-, wie in Formel (I) für R¹ definiert, steht, und R für Wasserstoff oder C₁-C₄-Alkyl- steht, oder -B(OR)₂ für einen Pinacolylboronsäureester steht, zu den erfindungsgemäßen Verbindungen der Formel (Ic) umsetzt (siehe auch D.G. Hall, Boronic Acids, 2005 WILEY-VCH Verlag GmbH & Co. KGaA, Weinheim, ISBN 3-527-30991-8, und darin zitierte Literatur).

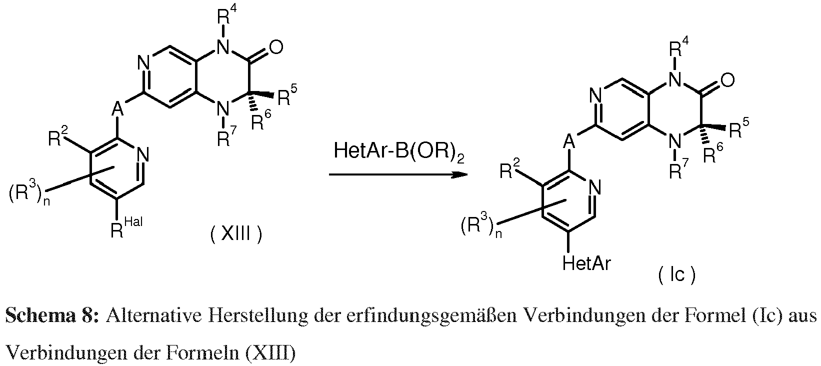

Ferner können die erfindungsgemäßen Verbindungen der Formel (Ic) auch aus den in Schema 4 gezeigten Ester-Intermediaten der Formel (VIII) sowie Carbonsäuren der Formel (IX) in dem Fachmann bekannter Weise aufgebaut werden.

Die beschriebenen Reaktionswege erlauben, dass bei dem Einsatz einer enantiomerenreinen Azidocarbonsäure der Formel (IIa) zu Beginn der Sequenz eine Epimerisierung oder Racemisierung des stereogenen Zentrums am Kohlenstoffatom, welches an R⁵ und R⁶ gebunden ist, weitestgehend unterdrückt werden kann.

Ebenfalls Gegenstand der vorliegenden Erfindung sind die Intermediate der allgemeinen Formel (VIII), 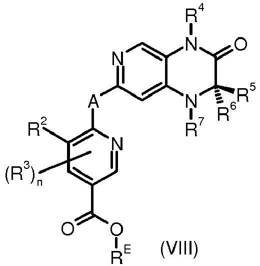 in der A, R², R³, R⁴, R⁵, R⁶, R⁷ und n die in der allgemeinen Formel (I) angegebenen Bedeutungen haben und R^{E} für C₁-C₆-Alkyl steht, die bevorzugt zur Herstellung der erfindungsgemäßen Verbindungen der allgemeinen Formel (I) verwendet werden können.

Ein weiterer Gegenstand der vorliegenden Erfindung sind die Intermediate der allgemeinen Formel (IX), 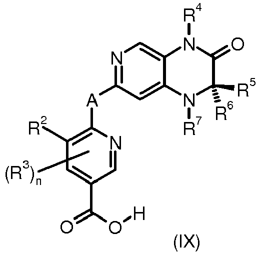 in der A, R², R³, R⁴, R⁵, R⁶, R⁷ und n die in der allgemeinen Formel (I) angegebenen Bedeutungen haben, die bevorzugt zur Herstellung der erfindungsgemäßen Verbindungen der allgemeinen Formel (I) verwendet werden können.

### Ausführungsbeispiele

Die nachfolgenden Beispiele erläutern die Herstellung der erfindungsgemäßen Verbindungen, ohne die Erfindung auf diese Beispiele zu beschränken.

Zunächst wird die Herstellung der Intermediate beschrieben, die schließlich zur Herstellung der erfindungsgemäßen Verbindungen bevorzugt zur Anwendung kommen.

IUPAC-Namen wurden erstellt mit Hilfe der Nomenklatursoftware ACD Name batch, Version 12.01, von Advanced Chemical Development, Inc., und bei Bedarf angepaßt, beispielsweise an die deutschsprachige Nomenklatur.

### Stöchiometrie von Salzformen:

Wenn bei den im Folgenden beschriebenen Synthese-Intermediaten und
Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.
Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Herstellung der Intermediate

### Intermediat 1.1: 7-Chlor-1-cyclopentyl-2,4-dimethyl-1,4-dihydropyrido[3,4-b]pyrazin-3(2H)-on

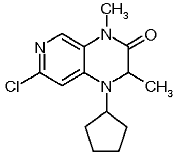

Zu einer Lösung von 3 g 7-Chlor-1-cyclopentyl-2-methyl-1,4-dihydropyrido[3,4-b]pyrazin-3(2H)-on (US20060009457) in 31 ml DMF wurden bei 0°C 700 mg Natriumhydrid (60% in Weissöl) gegeben. Anschließend wurden 1.1 ml Iodmethan zugegeben und 2.5 Stunden gerührt. Es wurde Eiswasser zugegeben und zweimal mit Ethylacetat extrahiert. Die vereinten organischen Phasen wurden mit gesättigter Natriumchlorid-Lösung gewaschen und mit Natriumsulfat getrocknet. Nach Chromatographie an Kieselgel (Hexan / Ethylacetat Gradient) erhielt man 2.53 g 7-Chlor-1-cyclopentyl-2,4-dimethyl-1,4-dihydropyrido[3,4-b]pyrazin-3(2H)-on.
¹H-NMR (300 MHz, RT, DMSO-d6): δ = 1.06 (d, 3H); 1.52-1.74 (m, 6H); 1.86-2.05 (m, 2H); 3.28 (s, 3H); 4.00 (q, 1H); 4.23 (q, 1H); 6.86 (s, 1H); 7.92 (s, 1H);

### Intermediat 1.2: 5-Hydroxy-6-nitro-pyridin-3-carbonsäure

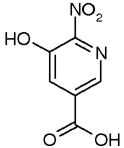

560 ml Schwefelsäure (96%ig) wurden langsam mit 89.6 ml Salpetersäure (65%ig) versetzt, so dass die Innentemperatur nie über 30°C stieg. Dazu wurden bei Raumtemperatur 70g 5-Hydroxynicotinsäure (CAS 5006-66-6) portionsweise zugegeben und 14 Stunden gerührt. Es wurde in reichlich Eiswasser gegossen und der entstandene Niederschlag K1 abfiltriert. Das Filtrat wurde mit Natronlauge auf pH=3 eingestellt und dreimal mit Ethylacetat extrahiert. Die vereinten organischen Phasen wurden mit Wasser gewaschen und mit Natriumsulfat getrocknet. Das Lösungsmittel wurde im Vakuum entfernt. Man erhielt einen weiteren Rückstand K2. Beide Niederschläge wurden vereint zu 43 g 5-Hydroxy-6-nitro-pyridin-3-carbonsäure.
¹H-NMR (300 MHz, RT, DMSO-d6): δ = 5.2 (bs, 1H); 8.03 (d, 1H); 8.43 (d, 1H); 12.06 (bs, 1H);

### Intermediat 1.3: 5-Methoxy-6-nitro-pyridin-3-carbonsäuremethylester

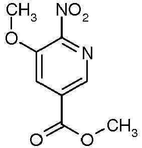

Eine Lösung von 78 g Intermediat 1.2 in 780ml Methanol und 780 ml Toluol wurde bei 0°C langsam mit 847 ml (Diazomethyl)-trimethylsilan versetzt und 14 Stunden gerührt. Es wurden Ethylacetat und gesättigte Natriumhydrogencarbonat-Lösung zugegeben. Die organische Phase wurde abgetrennt und die wässrige Phase zweimal mit Ethylacetat extrahiert. Die vereinten organischen Phasen wurden mit gesättigter Natriumchlorid-Lösung gewaschen und über Natriumsulfat getrocknet. Nach Chromatographie an Kieselgel (Hexan / Ethylacetat 8:2) wurden 27 g 5-Methoxy-6-nitro-pyridin-3-carbonsäuremethylester erhalten.
¹H-NMR (300 MHz, RT, DMSO-d6): δ = 3.95 (s, 3H); 4.06 (s, 3H); 8.27 (d, 1H); 8.59 (d, 1H);

### Intermediat 1.4: 6-Amino-5-methoxy-pyridin-3-carbonsäuremethylester

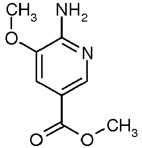

Eine Suspension von 27 g Intermediat 1.3 und 14.2 g Eisenpulver in 250 ml Methanol und 250 ml Essigsäure wurde 14 Stunden bei 85°C Badtemperatur gerührt. Der Ansatz wurde filtriert und im Vakuum eingeengt. Der Rückstand wurde in Ethylacetat aufgenommen und mit gesättigter Natriumhydrogencarbonat-Lösung gewaschen. Dann wurde mit gesättigter Natriumchlorid-Lösung gewaschen und über Natriumsulfat getrocknet. Man erhielt 20 g 6-Amino-5-methoxy-pyridin-3-carbonsäuremethylester.
¹H-NMR (300 MHz, RT, DMSO-d6): δ = 3.77 (s, 3H); 3.82 (s, 3H); 6.71 (bs, 2H); 7.30 (d, 1H); 8.17 (d, 1H);

### Intermediat 1.5: 6-[(1-Cyclopentyl-2,4-dimethyl-3-oxo-1,2,3,4-tetrahydropyrido[3,4-b]pyrazin-7-yl)amino]-5-methoxypyridin-3-carbonsäuremethylester

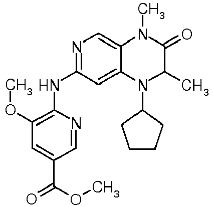

Eine Lösung von 1 g Intermediat 1.1 und 1.3 g Intermediat 1.4 in 38 ml Toluol wurde mit 445 mg BINAP und 161 mg Palladiumacetat versetzt und 5 Minuten gerührt. Dazu wurden 5.8 g Cäsiumcarbonat gegeben und 2.5 Stunden unter Argon bei 110°C gerührt. Der Ansatz wurde mit Ethylacetat verdünnt und zweimal mit Wasser extrahiert. Die vereinten organischen Phasen wurden mit gesättigter Natriumchlorid-Lösung gewaschen, über Natriumsulfat getrocknet und im Vakuum eingeengt. Nach Chromatographie an Kieselgel (Dichlormethan / Methanol Gradient) erhielt man 601 mg 6-[(1-Cyclopentyl-2,4-dimethyl-3-oxo-1,2,3,4-tetrahydropyrido[3,4-b]pyrazin-7-yl)amino]-5-methoxypyridin-3-carbonsäuremethylester.
¹H-NMR (300 MHz, RT, DMSO-d6): δ = 1.08 (d, 3H); 1.57-1.89 (m, 6H); 1.97-2.14 (m, 2H); 3.29 (s, 3H); 3.85 (s, 3H); 3.92 (q, 1H); 3.98 (s, 3H); 4.20 (q, 1H); 7.56 (d, 1H); 7.86 (s, 1H); 8.10 (s, 1H); 8.12 (s, 1H); 8.39 (d, 1H);

### Intermediat 1.6: 6-[(1-Cyclopentyl-2,4-dimethyl-3-oxo-1,2,3,4-tetrahydropyrido[3,4-b]pyrazin-7-yl)amino]-5-methoxypyridin-3-carbonsäure

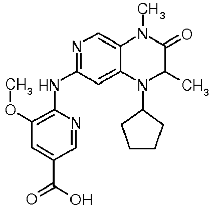

Eine Lösung von 601 mg Intermediat 1.5 in 28 ml Methanol und 9 ml THF wurde mit 14 ml Lithiumhydroxydlösung (1M) versetzt und 14 Stunden bei Raumtemperatur gerührt. Es wurde mit Salzsäure auf pH=5 eingestellt und dreimal mit einer Chloroform / Methanol-Lösung (9:1) extrahiert. Es wurde mit gesättigter Natriumchlorid-Lösung gewaschen, über Natriumsulfat getrocknet und im Vakuum eingeengt. Man erhielt 562 mg 6-[(1-Cyclopentyl-2,4-dimethyl-3-oxo-1,2,3,4-tetrahydropyrido[3,4-b]pyrazin-7-yl)amino]-5-methoxypyridin-3-carbonsäure.
¹H-NMR (300 MHz, RT, DMSO-d6): δ = 1.08 (d, 3H); 1.56-1.93 (m, 6H); 1.96-2.16 (m, 2H); 3.85-4.01 (m+s, 4H); 4.19 (q, 1H); 7.59 (d, 1H); 7.84 (s, 1H); 7.92 (s, 1H); 8.16 (s, 1H); 8.34 (d, 1H);

### Intermediat 2.1: (2R)-2-Azido-N-(4,6-dichlorpyridin-3-yl)butanamid

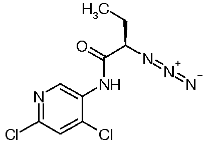

Eine Lösung von 4.75 g (2*R*)-2-Azido-butansäure (Herstellung siehe US20060009457) und 8.05 ml Thionylchlorid in 40 ml Dichlormethan wurde 2 Stunden bei 50°C gerührt. Es wurde vollständig im Vakuum eingeengt und bei 0°C eine Lösung von 3 g 5-Amino-2,4-dichloropyridin (CAS-Nr. 7321-93-9; Herstellung siehe US20060009457), und 6.5 ml Pyridin in 15 ml Dichlormethan zu getropft. Es wurde langsam auf Raumtemperatur, nach ca. 2 Stunden auf 40°C erhitzt. Es wurden weitere 20 ml Pyridin zugegeben und 14 Stunden bei 40°C gerührt. Nach Zugabe von Wasser wurde zweimal mit Dichlormethan extrahiert und über Natriumsulfat getrocknet. Nach Chromatographie an Kieselgel (Hexan / Ethylacetat 80:20) erhielt man 1.37 g (2*R*)-2-Azido-*N-*(4,6-dichlorpyridin-3-yl)butanamid.
¹H-NMR (300 MHz, RT, DMSO-d6): δ = 1.00 (t, 3H); 1.77-1.97 (2m, 2H); 4.09 (dd, 1H); 7.93 (s, 1H); 8.62 (s, 1H); 10.21 (s, 1H);

### Intermediat 2.2: (2R)-2-Amino-N-(4,6-dichlorpyridin-3-yl)butanamid Hydrochlorid

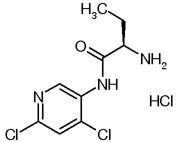

Eine Lösung von 853 mg Intermediat 2.1 in 12 ml THF wurde bei RT unter Argon mit 1.2 äquivalenten einer Trimethylphosphin-Lösung (1M in THF) versetzt. Es wurde 14 Stunden bei RT gerührt. Nach Zugabe von Wasser wurde im Vakuum eingeengt. Der Rückstand wurde in Wasser aufgenommen und mit Dichlormethan extrahiert und die organische Phase über Natriumsulfat getrocknet. Nach entfernen des Lösungsmittels wurde der Rückstand in Aceton/Et₂O aufgenommen und die Zielverbindung mit HCl (Lösung in Diethylether) als Hydrochlorid gefällt. Man erhielt 440 mg (2*R*)-2-Amino-*N*-(4,6-dichlorpyridin-3-yl)butanamid Hydrochlorid
¹H-NMR (300 MHz, RT, DMSO-d6): δ = 1.00 (t, 3H); 1.85-1.98 (m, 2H); 4.13 (bq, 1H); 7.95 (s, 1H); 8.48 (bs, 3H); 8.59 (s, 1H); 10.79 (s, 1H);

### Intermediat 2.3: (2R)-2-(Cyclopentylamino)-N-(4,6-dichlorpyridin-3-yl)butanamid

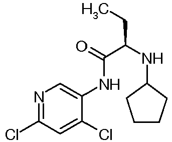

Eine Lösung von 440 mg Intermediat 2.2, 152 mg Cyclopentanon, 254 mg Natriumacetat und 946 mg Natriumtriacetoxyborhydrid in 20 ml Dichlormethan wurde 6 Stunden bei Raumtemperatur gerührt. Es wurde in gesättigte Natriumhydrogencarbonat Lösung gegeben und die organische Phase abgetrennt. Die wässrige Phase wurde mit Dichlormethan extrahiert, die vereinten organischen Phasen über Natriumsulfat getrocknet und im Vakuum vom Lösungsmittel befreit. Der Rückstand wurde durch Chromatographie an Kieselgel (Dichlormethan / Methanol 98:2) gereinigt. Man erhielt 355 mg (2*R*)-2-(Cyclopentylamino)-*N*-(4,6-dichlorpyridin-3-yl)butanamid.
¹H-NMR (300 MHz, RT, DMSO-d6): δ = 0.95 (t, 3H); 1.29-1.4 (m, 2H); 1.4-1.52 (m, 2H); 1.53-1.82 (m, 6H); 3.02 (qi, 1H); 3.14 (dd, 1H); 7.90 (s, 1H); 9.06 (s, 1H);

### Intermediat 2.4: (2R)-7-Chlor-1-cyclopentyl-2-ethyl-1,4-dihydropyrido[3,4-b]pyrazin-3(2H)-on

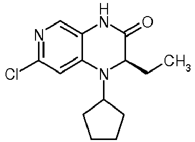

Eine Lösung von 355 mg Intermediat 2.3 und 1.6 ml Di-*iso*-propylethylamin in 2.2 ml DMF wurde 96 Stunden bei 155°C in einem verschlossenen Glasröhrchen gerührt. Es wurde mit Wasser verdünnt und mit Dichlormethan extrahiert. Das Lösungsmittel wurde im Vakuum entfernt und der Rückstand durch Chromatographie an Kieselgel (Hexan / Ethylacetat 75:25) gereinigt. Man erhielt 75 mg (2*R*)-7-Chlor-1-cyclopentyl-2-ethyl-1,4-dihydropyrido[3,4-b]pyrazin-3(2H)-on.
¹H-NMR (300 MHz, RT, DMSO-d6): δ = 0.79 (t, 3H); 1.44-1.53 (m, 1H); 1.54-1.74 (m, 7H); 1.85-1.94 (m, 1H); 1.97-2.05 (m, 1H); 3.95 (dd, 1H); 3.99-4.06 (m, 1H); 6.77 (s, 1H); 7.60 (s, 1H); 10.73 (s, 1H);

### Intermediat 2.5: (2R)-7-Chlor-1-cyclopentyl-2-ethyl-4-methyl-1,4-dihydropyrido[3,4-b]pyrazin-3(2H)-on

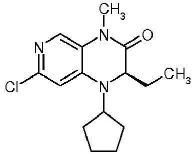

### Variante A:

Eine Lösung von 70 mg Intermediat 2.4 und 0.02 ml Iodmethan in 2 ml DMF wurde bei -5°C mit 20 mg Natriumhydrid (60% in Öl) versetzt. Nach 2 Stunden bei 0°C wurde Wasser zugegeben und 4-mal mit Dichlormethan extrahiert. Die vereinten organischen Phasen wurden mit Natriumsulfat getrocknet und das Lösungsmittel im Vakuum entfernt. Nach Chromatographie an Kieselgel (Hexan / Ethylacetat 1:1) erhielt man 57 mg (2*R*)-7-Chlor-1-cyclopentyl-2-ethyl-4-methyl-1,4-dihydropyrido[3,4-b]pyrazin-3(2H)-on.

### Variante B:

12 g 7-Chlor-1-cyclopentyl-2-ethyl-4-methyl-1,4-dihydropyrido[3,4-b]pyrazin-3(2H)-on (Intermediat 3.4) wurden durch chirale HPLC (Chiralpak IC 20µm 330x51 mm, Hexan / Ethanol 90:10, 250 mL/min) in die Enantiomere getrennt. Man erhielt 5.2 g (2*R*)-7-Chlor-1-cyclopentyl-2-ethyl-4-methyl-1,4-dihydropyrido[3,4-b]pyrazin-3(2H)-on.
¹H-NMR (300 MHz, RT, DMSO-d6): δ = 0.76 (t, 3H); 1.39-1.48 (m, 1H); 1.50-1.74 (m, 7H), 1.87-1.96 (m, 1H); 1.97-2.04 (m, 1H); 3.29 (s, 3H); 4.01-4.09 (m, 2H); 6.84 (s, 1H); 7.88 (s, 1H);

### Intermediat 2.6: 5-Brom-6-{[(dimethylamino)methylidene]amino}pyridin-3-carbonsäureethylester

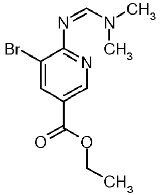

10 g 6-Amino-5-bromnicotinsäureethylester (CAS 850429-51-5) wurden in 53 ml 1,1-Dimethoxy-N,N-dimethylmethanamin 5 Stunden bei Raumtemperatur gerührt. Die Lösung wurde im Vakuum eingeengt und der Rückstand aus Methanol kristallisiert. Man erhielt 10.6 g 5-Brom-6-{[(dimethylamino)methylidene]amino}pyridin-3-carbonsäureethylester.
¹H-NMR (300 MHz, RT, DMSO-d6): δ = 1.30 (t, 3H); 3.10 (s, 3H); 3.17 (s, 3H); 4.28 (q, 2H); 8.22 (d, 1H); 8.61 (s, 1H); 8.64 (d, 1H);

### Intermediat 2.7: 6-{[(Dimethylamino)methylidene]amino}-5-ethenylpyridin-3-carbonsäureethylester

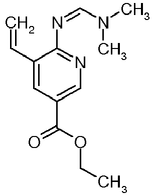

Eine Lösung von 10.6 g Intermediat 2.6, 926 mg Triphenylphosphin, 2.479 g Palladium-dichlor-bis-(triphenylphosphin), 14.2 g Kalium-ethenyltrifluoroborat und 40.3 g Caesiumcarbonat in 109 ml THF und 10.9 ml Wasser wurde für 3.5 Stunden bei 85°C erwärmt. Der Ansatz wurde mit Ethylacetat verdünnt und mit halbgesättigter Natriumhydrogencarbonat Lösung extrahiert. Die organische Phase wurde mit Natriumsulfat getrocknet und im Vakuum eingeengt. Der Rückstand wurde durch Chromatographie an Kieselgel (Hexan / Ethylacetat Gradient) gereinigt. Man erhielt 4.05 g 6-{[(Dimethylamino)methylidene]amino}-5-ethenylpyridin-3-carbonsäureethylester.
¹H-NMR (300 MHz, RT, DMSO-d6): δ = 1.32 (t, 3H); 3.07 (s, 3H); 3.16 (s, 3H); 4.30 (q, 2H); 5.34 (dd, 1H); 5.92 (dd, 1H); 7.20 (dd, 1H); 8.18 (d, 1H); 8.61-8.65 (m, 2H);

### Intermediat 2.8: 6-Amino-5-ethenylpyridin-3-carbonsäureethylester

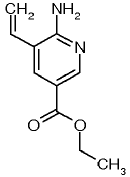

Eine Lösung von 2.5 g Intermediat 2.7, 44.5 ml konzentrierter Salzsäure, 31.2 ml Ethanol und 21.7 ml Wasser wurde 72 Stunden bei Raumtemperatur gerührt. Der Ansatz wurde mit Natronlauge auf pH=7 gebracht und mit Ethylacetat extrahiert. Die organische Phase wurde über Natriumsulfat getrocknet und im Vakuum eingeengt. Der Rückstand wurde durch Chromatographie an Kieselgel (Hexan / Ethylacetat Gradient) gereinigt. Man erhielt 600 mg 6-Amino-5-ethenylpyridin-3-carbonsäureethylester. Dieser Ansatz wurde ein zweites Mal analog mit 1.5 g Intermediat 2.7 durchgeführt und man erhielt dabei 400 mg 6-Amino-5-ethenylpyridin-3-carbonsäureethylester.
¹H-NMR (300 MHz, RT, DMSO-d6): δ = 1.29 (t, 3H); 4.24 (q, 2H); 5.32 (dd, 1H); 5.74 (dd, 1H); 6.82 (dd, 1H); 6.89 (s, 2H); 7.97 (d, 1H); 8.46 (d, 1H);

### Intermediat 2.9: 6-Amino-5-ethylpyridin-3-carbonsäureethylester

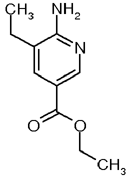

Eine Lösung von 1.0 g 6-Amino-5-ethenylpyridin-3-carbonsäureethylester (hergestellt wie unter Intermediat 2.8 beschrieben) in 161 ml Ethanol wurde mit 587 mg Palladium auf Kohle (10%) versetzt und 2 Stunden unter Wasserstoff-Atmosphäre bei Raumtemperatur gerührt.Danach wurde vom Katalysator abfiltriert und im Vakuum eingeengt. Der Rückstand wurde durch Chromatographie an Kieselgel (Hexan / Ethylacetat Gradient) gereinigt. Man erhielt 984 mg 6-Amino-5-ethylpyridin-3-carbonsäureethylester.
¹H-NMR (300 MHz, RT, DMSO-d6): δ = 1.13 (t, 3H); 1.28 (t, 3H); 2.41 (q, 2H); 4.22 (q, 2H); 6.65 (s, 2H); 7.65 (d, 1H); 8.39 (d, 1H);

### Intermediat 2.10: 6-{[(2R)-1-Cyclopentyl-2-ethyl-4-methyl-3-oxo-1,2,3,4-tetrahydropyrido[3,4-b]pyrazin-7-yl]amino}-5-ethylpyridin-3-carbonsäureethylester

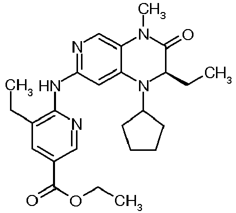

Eine Lösung von 700 mg Intermediat 2.5, 107 mg Palladium(II)acetat, 297 mg (R)-(+)-2,2'-Bis(diphenylphospino)-1,1'-binaphthyl, 3.88 g Caesiumcarbonat und 925 mg Intermediat 2.9 in 25 ml Toluol wurde unter Argon-Atmosphäre 3 Stunden bei 120°C gerührt. Der Ansatz wurde mit Ethylacetat verdünnt, vom Niederschlag abfiltriert und die organische Phase mit gesättigter Natriumchlorid-Lösung gewaschen. Die organische Phase wurde über Natriumsulfat getrocknet und im Vakuum eingeengt. Der Rückstand wurde durch Chromatographie an Kieselgel (Ethylacetat) gereinigt. Man erhielt 300 mg 6-{[(2*R*)-1-Cyclopentyl-2-ethyl-4-methyl-3-oxo-1,2,3,4-tetrahydropyrido[3,4-b]pyrazin-7-yl]amino}-5-ethylpyridin-3-carbonsäureethylester.
¹H-NMR (300 MHz, RT, DMSO-d6): δ = 0.78 (t, 3H); 1.20 (t, 3H); 1.31 (t, 3H); 1.41-1.53 (m, 1H); 1.54-1.89 (2m, 7H); 1.92-2.13 (m, 2H); 2.75 (q, 2H); 3.31 (s, 3H); 3.87-4.00 (m, 1H); 4.03 (dd, 1H); 4.30 (q, 2H); 7.81 (s, 1H); 7.84 (s, 1H); 7.89 (d, 1H); 8.40 (s, 1H); 8.60 (d, 1H);

### Intermediat 2.11: 6-{[(2R)-1-Cyclopentyl-2-ethyl-4-methyl-3-oxo-1,2,3,4-tetrahydropyrido[3,4-b]pyrazin-7-yl]amino}-5-ethylpyridin-3-carbonsäure

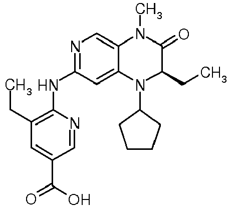

Eine Lösung von 298 mg Intermediat 2.10 in 13.6 ml Methanol, 4.2 ml THF und 6.6 ml Lithiumhydroxid-Lösung (1M) wurde 2 Stunden bei Raumtemperatur gerührt. Mit Salzsäure (1M) wurde auf pH=5 eingestellt und mit Chloroform /Methanol (9:1) extrahiert. Die organische Phase wurde über Natriumsulfat getrocknet und im Vakuum vollständig eingeengt. Man erhielt 274 mg 6-{[(2*R*)-1-Cyclopentyl-2-ethyl-4-methyl-3-oxo-1,2,3,4-tetrahydropyrido[3,4-b]pyrazin-7-yl]amino}-5-ethylpyridin-3-carbonsäure.
¹H-NMR (300 MHz, RT, DMSO-d6): δ = 0.77 (t, 3H); 1.20 (t, 3H); 1.40-1.56 (m, 1H); 1.56-1.91 (2m, 7H); 1.93-2.13 (m, 2H); 2.73 (q, 2H); 3.31 (s, 3H); 3.86-3.98 (m, 1H); 4.03 (dd, 1H); 7.81-7.86 (m, 2H); 7.89 (d, 1H); 8.33 (bs, 1H); 8.58 (d, 1H); 12.81 (bs, 1H);

### Intermediat 3.1: 2-Brom-N-(4,6-dichlor-3-pyridinyl)-butanamid

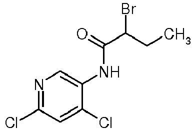

Zu einer Suspension von 194 g 5-Amino-2,4-dichloropyridin (CAS-Nr. 7321-93-9) und 388 g Kaliumcarbonat in 3.88 L Diethylether wurden bei 0°C langsam 260 ml 2-Brombutanoylbromid zugetropft. Der Ansatz wurde filtriert und der Filterkuchen mit Diethylether nachgewaschen. Der Filterkuchen wurde in Dichlormethan gelöst und die entstandene Lösung wurde mit Wasser und gesättigter Natriumchlorid-Lösung gewaschen. Die organische Phase wurde über Natriumsulfat getrocknet und im Vakuum eingeengt. Der Rückstand wurde mit Hexan verrührt, erneut abgesaugt und im Vakuum getrocknet. Man erhielt 150 g 2-Brom-*N*-(4,6-dichlor-3-pyridinyl)-butanamid.
¹H-NMR (300 MHz, RT, DMSO-d6): δ = 0.98 (t, 3H); 1.88-2.01 (m, 1H); 2.03-2.16 (m, 1H); 4.68 (t, 1H); 7.92 (s, 1H); 8.63 (s, 1H); 10.31 (s, 1H);

### Intermediat 3.2: 2-(Cyclopentylamino)-N-(4,6-dichlor-3-pyridinyl)-butanamid

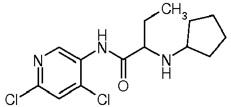

Eine Lösung von 130 g Intermediat 3.1, 119 ml *N,N*-Di-*iso*-propylethylamin und 37.4 ml Cyclopentylamin in 1.3 L Toluol wurde 24 Stunden bei einer Badtemperatur von 150°C gerührt. Der Ansatz wurde filtriert, der Feststoff mit Ethylacetat nachgewaschen und die vereinten Filtrate im Vakuum vollständig eingeengt. Man erhielt 138 g 2-(Cyclopentylamino)-*N*-(4,6-dichlor-3-pyridinyl)-butanamid, welches noch etwas Toluol enthielt.
¹H-NMR (300 MHz, RT, DMSO-d6): δ = 0.95 (t, 3H); 12.9-1.83 (3m, 10H); 3.02 (qi, 1H); 3.15 (t, 1H); 7.90 (s, 1H); 9.05 (s, 1H);

### Intermediat 3.3: 7-Chlor-1-cyclopentyl-2-ethyl-1,4-dihydro-pyrido[3,4-b]pyrazin-3(2H)-on

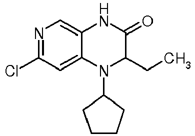

Eine Lösung von 32.5 g Intermediat 3.2 und 53.6 ml *N,N*-Di-*iso*-propylethylamin in 195 ml 1,3-Dimethyl-imidazolidin-2-on wurde bei 210°C Badtemperatur gerührt und dabei das *N,N*-Di-*iso-*propylethylamin langsam destilliert. Dann wurde noch weitere 5 Stunden bei einer Badtemperatur von 220°C gerührt. Nach dem Abkühlen wurde in Wasser aufgenommen und dreimal mit Ethylacetat extrahiert. Die vereinten organischen Phasen wurden mit Wasser gewaschen, über Natriumsulfat getrocknet und im Vakuum eingeengt. Nach Chromatographie an Kieselgel (Hexan / Ethylacetat 8:2) erhielt man 20.3 g 7-Chlor-1-cyclopentyl-2-ethyl-1,4-dihydro-pyrido[3,4-b]pyrazin-3(2H)-on.
¹H-NMR (300 MHz, RT, DMSO-d6): δ = 0.79 (t, 3H); 1.44-1.53 (m, 1H); 1.54-1.74 (m, 7H); 1.85-1.94 (m, 1H); 1.97-2.05 (m, 1H); 3.95 (dd, 1H); 3.99-4.06 (m, 1H); 6.77 (s, 1H); 7.60 (s, 1H); 10.73 (s, 1H);

### Intermediat 3.4: 7-Chlor-1-cyclopentyl-2-ethyl-1,4-dihydro-4-methyl-pyrido[3,4-b]pyrazin-3(2H)-on

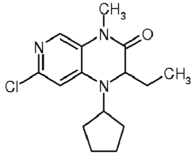

Eine Lösung von 20.3 g Intermediat 3.3 und 7 ml Iodmethan in 203 ml DMF wurde bei -5°C mit 2.69 g Natriumhydrid (60% in Öl) versetzt. Nach 0.5 Stunden bei 0°C wurde Wasser zugegeben und dreimal mit Ethylacetat extrahiert. Die vereinten organischen Phasen wurden mit Natriumsulfat getrocknet und das Lösungsmittel im Vakuum entfernt. Nach Chromatographie an Kieselgel (Hexan / Ethylacetat 8:2) erhielt man 18.1 g 7-Chlor-1-cyclopentyl-2-ethyl-4-methyl-1,4-dihydropyrido[3,4-b]pyrazin-3(2H)-on.
¹H-NMR (300 MHz, RT, DMSO-d6): δ = 0.76 (t, 3H); 1.39-1.48 (m, 1H); 1.50-1.74 (m, 7H), 1.87-1.96 (m, 1H); 1.97-2.04 (m, 1H); 3.29 (s, 3H); 4.01-4.09 (m, 2H); 6.84 (s, 1H); 7.88 (s, 1H);

### Intermediat 3.5: 6-[(1-Cyclopentyl-2-ethyl-1,2,3,4-tetrahydro-4-methyl-3-oxopyrido[3,4-b]pyrazin-7-yl)amino]-5-methoxypyridin-3-carbonsäuremethylester

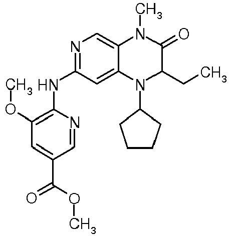

Eine Lösung von 1 g Intermediat 3.4, 1.24 g Intermediat 1.4, 626 mg Palladium(II)acetat, 895 mg (R)-(+)-2,2'-Bis(diphenylphospino)-1,1'-binaphthyl und 658 mg Natrium-*tert*-Butylat in 60 ml Dioxan wurde unter Argon-Atmosphäre 1 Stunden in einem Mikrowellenofen bei 110°C erhitzt. Der Ansatz wurde mit gesättigter Natriumhydrogencarbonat-Lösung versetzt und 3-mal mit Ethylacetat extrahiert. Die organische Phase wurde über Natriumsulfat getrocknet und im Vakuum eingeengt. Der Rückstand wurde durch Chromatographie an Kieselgel (Hexan / Ethylacetat Gradient) gereinigt. Man erhielt 210 mg 6-[(1-Cyclopentyl-2-ethyl-1,2,3,4-tetrahydro-4-methyl-3-oxopyrido[3,4-b]pyrazin-7-yl)amino]-5-methoxypyridin-3-carbonsäuremethylester.
¹H-NMR (300 MHz, RT, DMSO-d6): δ = 0.76 (t, 3H); 1.43-1.55 (m, 1H); 1.56-1.93 (2m, 7H); 1.96-2.12 (m, 2H); 3.30 (s, 3H); 3.85 (s, 3H); 3.91- 4.00 (m+s, 1+3H); 4.06 (dd, 1H); 7.56 (d, 1H); 7.82 (s, 1H); 8.07 (s, 1H); 8.10 (s, 1H); 8.39 (d, 1H);

### Intermediat 3.6: 6-[(1-Cyclopentyl-2-ethyl-4-methyl-3-oxo-1,2,3,4-tetrahydropyrido[3,4-b]pyrazin-7-yl)amino]-5-ethylpyridin-3-carbonsäure

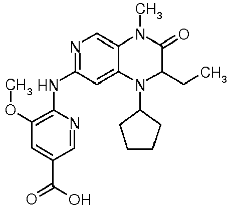

Eine Lösung von 92 mg Intermediat 3.5 in 4.6 ml Methanol und 1.2 ml Wasser wurde mit 0.02 ml Natronlauge (5N) versetzt und 14 Stunden bei Raumtemperatur gerührt. Anschließend wurden 0.01 ml Natronlauge (5N) zugegeben und 2 Stunden bei 55°C gerührt. Der Ansatz wurde mit Salzsäure auf pH = 5-6 eingestellt und im Vakuum stark eingeengt. Es wurde mit Methanol / Chloroform 1:8 aufgenommen und die wässrige Phase abgetrennt. Die organische Phase wurde über Natriumsulfat getrocknet und im Vakuum vollständig eingeengt. Der Rückstand wurde durch Chromatographie an Kieselgel (Dichlormethan / Methanol 8:2) gereinigt. Man erhielt 55 mg 6-[(1-Cyclopentyl-2-ethyl-4-methyl-3-oxo-1,2,3,4-tetrahydropyrido[3,4-b]pyrazin-7-yl)amino]-5-ethylpyridin-3-carbonsäure.
¹H-NMR (300 MHz, RT, DMSO-d6): δ = 0.75 (t, 1H); 1.40-1.55 (m, 1H); 1.55-1.75 (m, 3H); 1.75-2.15 (2m, 6H); 3.29 (s, 1H); 3.86-4.00 (m+s, 1+3H); 4.04 (dd, 1H); 7.61 (bs, 1H); 7.79 (bs, 1H); 7.87 (bs, 1H); 8.12 (bs, 1H); 8.36 (bs, 1H);

### Intermediat 4.1: 6-[[(2R)-1-Cyclopentyl-2-ethyl-1,2,3,4-tetrahydro-4-methyl-3-oxopyrido[3,4-b]pyrazin-7-yl]amino]-5-methoxypyridin-3-carbonsäuremethylester

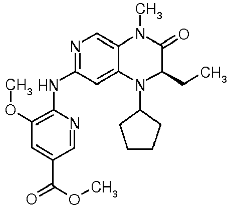

In Analogie zur Herstellung von Intermediat 3.5 wurde Intermediat 4.1 hergestellt aus 5.17 g Intermediat 2.5 und 6.41 g Intermediat 1.4. Man erhielt 3.2 g 6-[[(2*R*)-1-Cyclopentyl-2-ethyl-1,2,3,4-tetrahydro-4-methyl-3-oxopyrido[3,4-b]pyrazin-7-yl]amino]-5-methoxypyridin-3-carbonsäuremethylester.
¹H-NMR (300 MHz, RT, DMSO-d6): δ = 0.76 (t, 3H); 1.42-1.55 (m, 1H); 1.55-1.93 (2m, 7H); 1.95-2.14 (m, 2H); 3.30 (s, 3H); 3.85 (s, 3H); 3.91-4.00 (m+s, 1+3H); 4.06 (dd, 1H); 7.56 (d, 1H); 7.83 (s, 1H); 8.07 (s, 1H); 8.10 (s, 1H); 8.39 (d, 1H);

### Intermediat 4.2: 6-[[(2R)-1-Cyclopentyl-2-ethyl-1,2,3,4-tetrahydro-4-methyl-3-oxopyrido[3,4-b]pyrazin-7-yl]amino]-5-methoxypyridin-3-carbonsäure

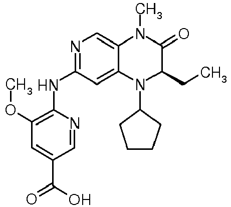

In Analogie zur Herstellung von Intermediat 3.6 wurde Intermediat 4.2 hergestellt aus 2.3 g Intermediat 4.1. Man erhielt 2.2 g 6-[[(2*R*)-1-Cyclopentyl-2-ethyl-1,2,3,4-tetrahydro-4-methyl-3-oxopyrido[3,4-b]pyrazin-7-yl]amino]-5-methoxypyridin-3-carbonsäure.
¹H-NMR (300 MHz, RT, DMSO-d6): δ = 0.76 (t, 3H); 1.41-1.55 (m, 1H); 1.56-1.75 (m, 3H; 1..75-1.94 (m, 4H); 1.96-2.13 (m, 2H); 3.30 (s, 3H); 3.89-4.02 (m+s, 1+3H); 4.06 (dd, 1H); 7.56 (d, 1H); 7.82 (s, 1H); 8.05 (bs, 1H); 8.08 (s, 1H); 8.37 (d, 1H);

### Intermediat 5.1: 6-Amino-5-methyl-pyridin-3-carbonsäuremethylester

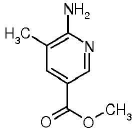

Eine Lösung von 2 g 6-Amino-5-methyl-pyridin-3-carbonitril (CAS 183428-91-3) in 40 ml Methanol und 18 ml konzentrierter Schwefelsäure wurde 3 Stunden auf Rückfluss erhitzt. Der Ansatz wurde auf Eis-Wasser gegeben, mit Natriumhydroxyd basisch gestellt und mit Ethylacetat extrahiert. Die organische Phase wurde mit gesättigter Natriumchlorid-Lösung gewaschen, über Natriumsulfat getrocknet und im Vakuum eingeengt. Man erhielt 1.95 g 6-Amino-5-methyl-pyridin-3-carbonsäuremethylester.
¹H-NMR (300 MHz, RT, DMSO-d6): δ = 2.06 (s, 3H); 3.75 (s, 3H); 6.63 (bs, 2H); 7.68 (d, 1H); 8.39 (d, 1H);

### Intermediat 5.2: 6-[[(2R)-1-Cyclopentyl-2-ethyl-1,2,3,4-tetrahydro-4-methyl-3-oxopyrido[3,4-b]pyrazin-7-yl]amino]-5-methylpyridin-3-carbonsäuremethylester

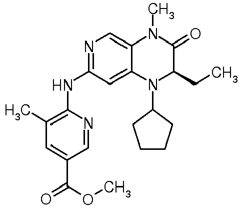

In Analogie zur Herstellung von Intermediat 3.5 wurde Intermediat 5.2 hergestellt aus 700 mg Intermediat 2.5 und 791 mg Intermediat 5.1. Man erhielt 395 mg 6-[[(2*R*)-1-Cyclopentyl-2-ethyl-1,2,3,4-tetrahydro-4-methyl-3-oxopyrido[3,4-b]pyrazin-7-yl]amino]-5-methylpyridin-3-carbonsäuremethylester.
¹H-NMR (300 MHz, RT, DMSO-d6): δ = 0.76 (t, 3H); 1.39-1.55 (m, 1H); 1.55-1.90 (2m, 7H); 1.92-2.13 (m, 2H); 2.32 (s, 3H); 3.30 (s, 3H); 3.82 (s, 3H); 3.82-3.97 (m, 1H); 4.03 (dd, 1H); 7.82 (s, 1H); 7.83 s, 1H); 7.93 (d, 1H); 8.39 (s, 1H), 8.59 (d, 1H);

### Intermediat 5.3: 6-[[(2R)-1-Cyclopentyl-2-ethyl-1,2,3,4-tetrahydro-4-methyl-3-oxopyrido[3,4-b]pyrazin-7-yl]amino]-5-methylpyridin-3-carbonsäure

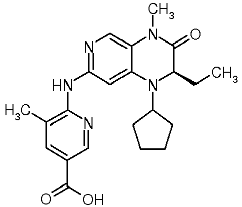

In Analogie zur Herstellung von Intermediat 3.6 wurde Intermediat 5.3 hergestellt aus 385 mg Intermediat 5.2. Man erhielt 226 mg 6-[[(2*R*)-1-Cyclopentyl-2-ethyl-1,2,3,4-tetrahydro-4-methyl-3-oxopyrido[3,4-b]pyrazin-7-yl]amino]-5-methylpyridin-3-carbonsäure.
¹H-NMR (300 MHz, RT, DMSO-d6): δ = 0.77 (t, 1H); 1.45-1.88 (3m, 8H); 1.99-3.13 (m, 2H); 2.36 (s, 3H); 3.31 (s, 3H); 3.95 (qi, 1H); 4.13 (dd, 1H); 7.73 (s, 1H); 7.83 (s, 1H); 7.98 (d, 1H); 8.60 (d, 1H); 8.87 (bs, 1H);

### Herstellung der erfindungsgemässen Verbindungen

### Beispiel 1: 6-{[1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid

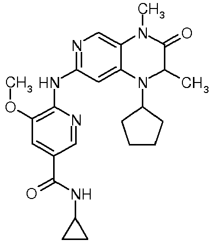

Eine Lösung von 200 mg Intermediat 1.6, 390 mg TBTU, 335 mg Kaliumcarbonat und 0.08 ml Cyclopropylamin in 10 ml DMF wurden bei Raumtemperatur für 14 Stunden gerührt. Der Ansatz wurde mit Ethylacetat verdünnt und je zweimal mit gesättigter Natriumhydrogencarbonat-Lösung und gesättigter Natriumchlorid-Lösung gewaschen, über Natriumsulfat getrocknet und im Vakuum eingeengt. Der Rückstand wurde durch Chromatographie (Kieselgel, Ethylacetat/ Methanol Gradient) gereinigt. Man erhielt 175 mg 6-{[1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid.
¹H-NMR (400 MHz, RT, DMSO-d6): δ = 0.53-0.61 (m, 2H); 0.67-0.75 (m, 2H); 1.08 (d, 3H); 1.58-1.80 (m, 6H); 1.99-2.15 (m, 2H); 2.77-2.88 (m, 1H); 3.29 (s, 3H); 3.68-3.99 m+s, 4H); 4.19 (q, 1H); 7.61 (d, 1H); 7.84 (s, 1H); 7.92 (s, 1H); 8.12 (s, 1H); 8.29 (d, 1H); 8.42 (d, 1H);

### Beispiel 2: 6-{[(2R)-1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid

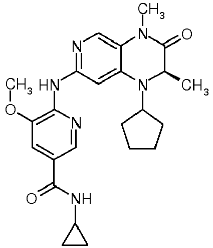

238 mg 6-{[1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid wurden durch chirale HPLC (Chiralcel OD-H 5µm 250x30 mm, Hexan / Ethanol 90:10 + 0.1% Diethylamin (v/v), 25 mL/min) in die Enantiomere getrennt. Man erhielt 49 mg 6-{[(2*R*)-1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid.
Optische Rotation: [α_{D}= -125.7° +/- 0.09° (c=4.2, DMSO)].

### Beispiel 3: 6-{[(2R)-1-Cyclopentyl-2-ethyl-4-methyl-3-oxo-1,2,3,4-tetrahydropyrido[3,4-b]pyrazin-7-yl]amino}-N-cyclopropyl-5-ethylpyridin-3-carboxamid

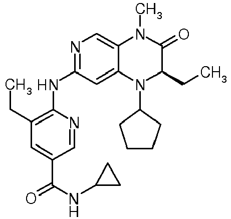

Eine Lösung von 80 mg Intermediat 2.11, 121 mg TBTU, 130 mg Kaliumcarbonat und 0.32 mg Cyclopropylamin in 6.4 ml DMF wurden bei Raumtemperatur für 14 Stunden gerührt. Der Ansatz wurde mit Ethylacetat verdünnt und je zweimal mit gesättigter Natriumhydrogencarbonat-Lösung und gesättigter Natriumchlorid-Lösung gewaschen, über Natriumsulfat getrocknet und im Vakuum eingeengt. Der Rückstand wurde durch Chromatographie (Kieselgel, Ethylacetat/ Methanol Gradient) gereinigt. Man erhielt 62 mg 6-{[(2*R*)-1-Cyclopentyl-2-ethyl-4-methyl-3-oxo-1,2,3,4-tetrahydropyrido[3,4-b]pyrazin-7-yl]amino}-N-cyclopropyl-5-ethylpyridin-3-carboxamid.
¹H-NMR (300 MHz, RT, DMSO-d6): δ = 0.52-0.6 (m, 2H); 0.65-0.73 (m, 2H); 0.78 (t, 3H); 1.21 (t, 3H); 1.40-1.55 (m, 1H); 1.55-1.89 (2m, 7H); 1.93-2.13 (m, 2H); 2.66-2.76 (m, 2H); 2.81 (dq, 1H); 3.30 (s, 1H); 3.85-3.97 (m, 1H); 3.99-4.06 (m, 1H); 7.81 (s, 1H); 7.83 (s, 1H); 1.87 (d, 1H); 8.17 (s, 1H); 8.38 (d, 1H); 8.51 (d, 1H);

### Beispiel 4: 6-[(1-Cyclopentyl-2-ethyl-1,2,3,4-tetrahydro-4-methyl-3-oxopyrido[3,4-b]pyrazin-7-yl)amino]-N-cyclopropyl-5-methoxy-3-pyridincarboxamid

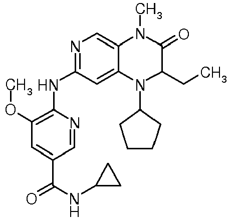

In Analogie zu Beispiel 3 wurden aus 50 mg Intermediat 3.6 und 20 mg Cyclopropylamin 42mg 6-[(1-Cyclopentyl-2-ethyl-1,2,3,4-tetrahydro-4-methyl-3-oxopyrido[3,4-b]pyrazin-7-yl)amino]-N-cyclopropyl-5-methoxy-3-pyridincarboxamid erhalten.
¹H-NMR (300 MHz, RT, DMSO-d6): δ = 0.52-0.6 (m, 2H); 0.66-0.82 (m, 5H); 1.39-1.56 (m, 1H); 1.56-1.93 (2m, 7H); 1.95-2.14 (m, 2H); 2.77-2.88 (m, 1H); 3.30 (s, 3H); 3.87-4.00 (m+s, 1+3H); 4.05 (dd, 1H); 7.61 (s, 1H); 7.81 (s, 1H); 7.90 (s, 1H); 8.09 (s, 1H); 8.29 (s, 1H); 8.43 (d, 1H);

### Beispiel 5: 6-[[(2R)-1-Cyclopentyl-2-ethyl-1,2,3,4-tetrahydro-4-methyl-3-oxopyrido[3,4-b]pyrazin-7-yl)amino]-N-cyclopropyl-5-methoxy-3-pyridincarboxamid

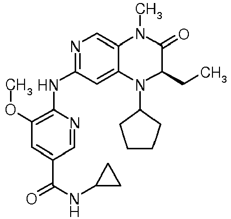

In Analogie zu Beispiel 3 wurden aus 150 mg Intermediat 4.2 und 49 mg Cyclopropylamin 92 mg 6-[[(2*R*)-1-Cyclopentyl-2-ethyl-1,2,3,4-tetrahydro-4-methyl-3-oxopyrido[3,4-b]pyrazin-7-yl)amino]-N-cyclopropyl-5-methoxy-3-pyridincarboxamid erhalten.
¹H-NMR (300 MHz, RT, DMSO-d6): δ = 0.52-0.6 (m, 2H); 0.66-0.82 (m, 5H); 1.39-1.56 (m, 1H); 1.56-1.93 (2m, 7H); 1.95-2.14 (m, 2H); 2.77-2.88 (m, 1H); 3.30 (s, 3H); 3.87-4.00 (m+s, 1+3H); 4.05 (dd, 1H); 7.61 (s, 1H); 7.81 (s, 1H); 7.90 (s, 1H); 8.09 (s, 1H); 8.29 (s, 1H); 8.43 (d, 1H);

### Beispiel 6: 6-[(1-Cyclopentyl-2,4-dimethyl-3-oxo-1,2,3,4-tetrahydropyrido[3,4-b]pyrazin-7-yl)amino]-5-methoxy-N-(1-methylpiperidin-4-yl)pyridin-3-carboxamid

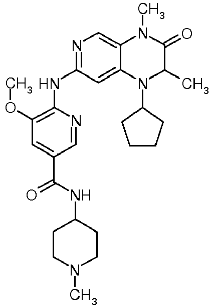

In Analogie zu Beispiel 3 wurden aus 203 mg Intermediat 1.6 und 148 mg 4-Amino-1-methylpiperidin 87 mg 6-[(1-Cyclopentyl-2,4-dimethyl-3-oxo-1,2,3,4-tetrahydropyrido[3,4-b]pyrazin-7-yl)amino]-5-methoxy-N-(1-methylpiperidin-4-yl)pyridin-3-carboxamid erhalten.
¹H-NMR (300 MHz, RT, DMSO-d6): δ = 1.08 (d, 3H); 1.5-2.15 (m, 12H); 2.73-2.84 (m, 2H); 3.29 (s, 3H), 3.64-3.81 (m, 1H); 3.86-4.01 (m+s, 1+3H); 4.19 (q, 1H); 7.63 (d, 1H); 7.85 (s, 1H); 7.92 (s, 1H); 8.13 (s, 1H); 8.20 (d, 1H); 8.32 (d, 1H);

### Beispiel 7: 6-[[(2R)-1-Cyclopentyl-2-ethyl-1,2,3,4-tetrahydro-4-methyl-3-oxopyrido[3,4-b]pyrazin-7-yl]amino]-5-methoxy-N-(2,2,2-trifluoroethyl)-3-pyridincarboxamid

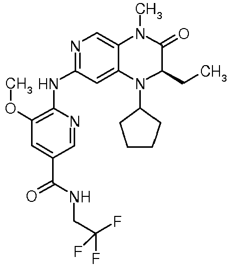

In Analogie zu Beispiel 3 wurden aus 50 mg Intermediat 4.2 und 35 mg 2,2,2-Trifluorethylamin 54 mg 6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(2,2,2-trifluoroethyl)-3-pyridincarboxamid erhalten.
¹H-NMR (300 MHz, RT, DMSO-d6): δ = 0.77 (t, 3H); 1.41-1.57 (m, 1H); 1.57-1.74 (m, 3H); 1.75-1.94 (m, 4H); 1.97-2.14 (m, 2H); 3.30 (s, 3H); 3.90-4.00 (m+2, 1+3H); 4.00-4.19 (m, 3H); 7.58 (d, 1H); 7.82 (s, 1H); 7.98 (s, 1H); 8.10 (s, 1H); 8.40 (d, 1H);

### Beispiel 8: 6-[[(2R)-1-Cyclopentyl-2-ethyl-1,2,3,4-tetrahydro-4-methyl-3-oxopyrido[3,4-b]pyrazin-7-yl]amino]-5-methoxy-N-(2-methoxyethyl)-3-pyridincarboxamid

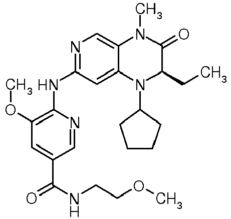

In Analogie zu Beispiel 3 wurden aus 60 mg Intermediat 4.2 und 32 mg 2-Methoxyethylamin 50 mg 6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(2-methoxyethyl)-3-pyridincarboxamid erhalten.
¹H-NMR (300 MHz, RT, DMSO-d6): δ = 0.77 (t, 3H); 1.41-1.57 (m, 1H); 1.57-1.74 (m, 3H); 1.75-1.94 (m, 4H); 1.96-2.15 (m, 2H); 3.27 (s, 3H); 3.30 (s, 3H); 3.40-3.50 (m, 4H); 3.89-4.01 (m+s, 1+3H); 4.05 (dd, 1H); 7.65 (d, 1H); 7.81 (s, 1H); 7.91 (s, 1H); 8.10 (s, 1H); 8.34 (d, 1H); 8.55 (t, 1H);

### Beispiel 9: 6-{[(2R)-1-Cyclopentyl-2-ethyl-4-methyl-3-oxo-1,2,3,4-tetrahydropyrido[3,4-b]pyrazin-7-yl]amino}-5-ethyl-N-[(3R)-2-oxoazepan-3-yl]pyridin-3-carboxamid

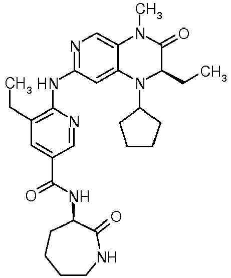

In Analogie zu Beispiel 3 wurden aus 80 mg Intermediat 2.11 und 72 mg (*R*)-3-Amino-azepan-2-on 77 mg 6-{[(2*R*)-1-Cyclopentyl-2-ethyl-4-methyl-3-oxo-1,2,3,4-tetrahydropyrido[3,4-b]pyrazin-7-yl]amino}-5-ethyl-*N*-[(3*R*)-2-oxoazepan-3-yl]pyridin-3-carboxamid erhalten.
¹H-NMR (300 MHz, RT, DMSO-d6): δ = 0.78 (t, 3H); 1.15-1.33 (m, 4H); 1.40-2.15 (m, 15H); 2.74 (q, 2H); 3.03-3.16 (m, 1H); 3.17-3.26 (m, 1H); 3.31 (s, 3H); 3.93 (qi, 1H); 4.03 (dd, 1H); 4.63 (bt, 1H); 7.78-7.87 (m, 3H); 7.93 (s, 1H); 8.23 (s, 1H); 8.29 (d, 1H); 8.56 (d, 1H);

### Beispiel 10: 6-[[(2R)-1-Cyclopentyl-2-ethyl-1,2,3,4-tetrahydro-4-methyl-3-oxopyrido[3,4-b]pyrazin-7-yl]amino]-5-methoxy-N-(tetrahydro-2H-pyran-4-yl)-3-pyridincarboxamid

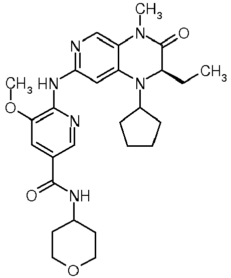

In Analogie zu Beispiel 3 wurden aus 75 mg Intermediat 4.2 und 89 mg 4-Amino-tetrahydro-2H-pyran 56 mg 6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(tetrahydro-2H-pyran-4-yl)-3-pyridincarboxamid erhalten.
¹H-NMR (300 MHz, RT, DMSO-d6): δ = 0.77 (t, 3H); 1.40-1.72 (m, 6H); 1.72-1.92 (m, 6H); 1.96-2.14 (m, 2H); 3.30 (s, 1H); 3.39 (dt, 2H); 3.89 (dd, 2H); 3.94-4.02 (m+s, 1+3H); 4.05 (dd, 1H); 7.63 (d, 1H); 7.81 (s, 1H); 7.91 (s, 1H); 8.09 (s, 1H); 8.28 (d, 1H); 8.33 (d, 1H);

### Beispiel 11: 6-[[(2R)-1-Cyclopentyl-2-ethyl-1,2,3,4-tetrahydro-4-methyl-3-oxopyrido[3,4-b]pyrazin-7-yl]amino]-N-(2-hydroxy-1,1-dimethylethyl)-5-methoxy-3-pyridincarboxamid

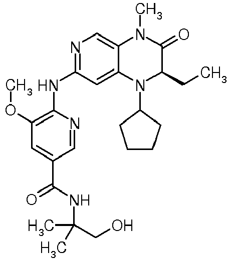

In Analogie zu Beispiel 3 wurden aus 92 mg Intermediat 4.2 und 47 mg 2-Amino-2-methylpropan-1-ol 65 mg 6-[[(2*R*)-1-Cyclopentyl-2-ethyl-1,2,3,4-tetrahydro-4-methyl-3-oxopyrido[3,4-b]pyrazin-7-yl]amino]-*N*-(2-hydroxy-1,1-dimethylethyl)-5-methoxy-3-pyridincarboxamid erhalten.
¹H-NMR (300 MHz, RT, DMSO-d6): δ = 0.77 (t, 3H); 1.32 (s, 6H); 1.41-1.54 (m, 1H); 1.56-1.74 (m, 3H); 1.75-1.91 (m, 4H); 1.98-2.14 (m, 2H); 3.30 (s, 3H); 3.90-3.99 (m+s, 1+3H); 4.00-4.11 (m, 2H); 4.92 (bs, 1H); 7.59 (bs, 2H); 7.81 (s, 1H); 7.89 (s, 1H); 8.10 (s, 1H); 8.29 (d, 1H);

### Beispiel 12: 6-[[(2R)-1-Cyclopentyl-2-ethyl-1,2,3,4-tetrahydro-4-methyl-3-oxopyrido[3,4-b]pyrazin-7-yl]amino]-5-methoxy-N-(3-pyridinylmethyl)-3-pyridincarboxamid

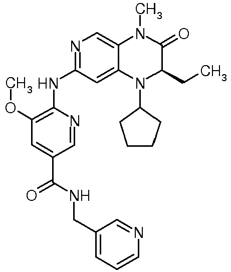

In Analogie zu Beispiel 3 wurden aus 50 mg Intermediat 4.2 und 31 mg 3-Pyridylmethanamin 50 mg 6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(3-pyridinylmethyl)-3-pyridincarboxamid erhalten.
¹H-NMR (300 MHz, RT, DMSO-d6): δ = 0.76 (t, 3H); 1.40-1.53 (m, 1H); 1.53-1.77 (m, 3H); 1.77-1.92 (m, 4H); 1.95-2.13 (m, 2H); 3.30 (s, 3H); 3.88-4.00 (m+s, 1+3H); 4.05 (dd, 1H); 4.51 (d, 2H); 7.36 (dd, 1H); 7.67 (d, 1H); 7.73 (dt, 1H); 7.81 (s, 1H); 7.93 (s, 1H); 8.10 (s, 1H); 8.38 (d, 1H); 8.46 (dd, 1H); 8.56 (d, 1H); 9.09 (t, 1H);

### Beispiel 13: 6-{[(2R)-1-Cyclopentyl-2-ethyl-4-methyl-3-oxo-1,2,3,4-tetrahydropyrido[3,4-b]pyrazin-7-yl]amino}-N-cyclopropyl-5-methylpyridin-3-carboxamid

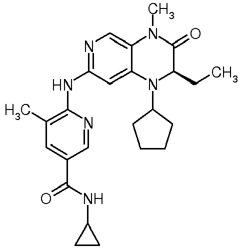

In Analogie zu Beispiel 3 wurden aus 38 mg Intermediat 5.3 und 16 mg Cyclopropylamin 23 mg 6-{[(2*R*)-1-Cyclopentyl-2-ethyl-4-methyl-3-oxo-1,2,3,4-tetrahydropyrido[3,4-b]pyrazin-7-yl]amino}-*N*-cyclopropyl-5-methylpyridin-3-carboxamid erhalten.
¹H-NMR (300 MHz, RT, DMSO-d6): δ = 0.52-0.58 (m, 2H); 0.65-0.72 (m, 2H); 0.77 (t, 3H); 1.40-1.53 (m, 1H); 1.55-1.71 (m, 3H); 1.72-1.88 (m, 4H); 1.94-2.12 (m, 2H); 2.30 (s, 3H); 2.77-2.86 (dqi, 1H); 3.30 (s, 3H); 3.91 (qi, 1H); 4.03 (dd, 1H); 7.81 (s, 1H); 7.83 (s, 1H); 7.88 (d, 1H); 8.19 (s, 1H); 8.35 (d, 1H); 8.50 (d, 1H);

### Beispiel 14: 6-[[(2R)-1-Cyclopentyl-2-ethyl-1,2,3,4-tetrahydro-4-methyl-3-oxopyrido[3,4-b]pyrazin-7-yl]amino]-5-methoxy-N-(1-methyl-4-piperidinyl)-3-pyridincarboxamid

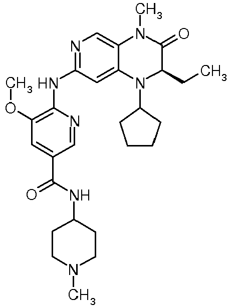

In Analogie zu Beispiel 3 wurden aus 74 mg Intermediat 4.2 und 95 mg 4-Amino-1-methylpiperidin 66 mg 6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(1-methyl-4-piperidinyl)-3-pyridincarboxamid erhalten.
¹H-NMR (300 MHz, RT, DMSO-d6): δ = 0.77 (t, 3H); 1.40-1.74 (m, 5H); 1.74-2.13 (3m, 9H); 2.17 (s, 3H); 2.78 (bs, 2H); 3.67-3.82 (m, 1H); 3.90-4.01 (m+s, 1+3H); 4.04 (dd, 1H); 7.63 (d, 1H); 7.81 (s, 1H); 7.89 (s, 1H); 8.09 (s, 1H); 8.19 (d, 1H); 8.32 (d, 1H);

### Beispiel 15: 6-{[(2R)-1-Cyclopentyl-2-ethyl-4-methyl-3-oxo-1,2,3,4-tetrahydropyrido[3,4-b]pyrazin-7-yl]amino}-5-methylpyridin-3-carboxamid

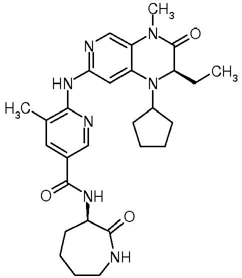

In Analogie zu Beispiel 3 wurden aus 38 mg Intermediat 5.3 und 36 mg (*R*)-3-Amino-azepan-2-on 32 mg 6-{[(2*R*)-1-Cyclopentyl-2-ethyl-4-methyl-3-oxo-1,2,3,4-tetrahydropyrido[3,4-b]pyrazin-7-yl]amino}-5-methylpyridin-3-carboxamid erhalten.
¹H-NMR (300 MHz, RT, DMSO-d6): δ = 0.77 (t, 3H); 1.20-1.30 (m, 1H); 1.40-1.53 (m, 1H); 1.54-1.96 (m, 12H); 1.96-2.13 (m, 2H); 2.33 (s, 3H); 3.04-3.14 (m, 1H); 3.22 (dt, 1H); 3.31 (s, 3H); 3.92 (qi, 1H); 4.03 (dd, 1H); 4.62 (dd, 1H); 7.80-7.87 (m, 3H); 7.93 (d, 1H); 8.23 (s, 1H); 8.24 (s, 1H); 8.55 (d, 1H);

### Beispiel 16: 6-[[(2R)-1-Cyclopentyl-2-ethyl-1,2,3,4-tetrahydro-4-methyl-3-oxopyrido[3,4-b]pyrazin-7-yl]amino]-N-[(3R)-hexahydro-2-oxo-1H-azepin-3-yl]-5-methoxy-3-pyridincarboxamid

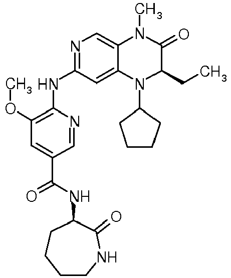

In Analogie zu Beispiel 3 wurden aus 60 mg Intermediat 4.2 und 44 mg (*R*)-3-Amino-azepan-2-on 23 mg 6-[[(2*R*)-1-Cyclopentyl-2-ethyl-1,2,3,4-tetrahydro-4-methyl-3-oxopyrido[3,4-b]pyrazin-7-yl]amino]-*N*-[(3*R*)-hexahydro-2-oxo-1H-azepin-3-yl]-5-methoxy-3-pyridincarboxamid erhalten.
¹H-NMR (300 MHz, RT, DMSO-d6): δ = 0.77 (t, 3H); 1.20-1.32 (m, 2H); 1.40-1.55 (m, 1H); 1.55-1.96 (2m, 11H); 1.96-2.14 (m, 2H); 3.04-3.16 (m, 1H); 3.17-3.27 (m, 1H); 3.30 (s, 3H); 3.90-4.01 (m+s, 1+3H); 4.05 (dd, 1H); 4.65 (dd, 1H); 7.66 (d, 1H); 7.77-7.85 (m, 2H); 7.92 (s, 1H); 8.11 (s, 1H); 8.33-8.40 (m, 2H);

### Beispiel 17: 6-{[(2R)-1-Cyclopentyl-2-ethyl-4-methyl-3-oxo-1,2,3,4-tetrahydropyrido[3,4-b]pyrazin-7-yl]amino}-5-methyl-N-(tetrahydro-2H-pyran-4-yl)pyridin-3-carboxamid

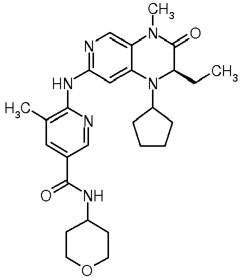

In Analogie zu Beispiel 3 wurden aus 35 mg Intermediat 5.3 und 26 mg 4-Amino-tetrahydro-2H-pyran 18 mg 6-{[(2*R*)-1-Cyclopentyl-2-ethyl-4-methyl-3-oxo-1,2,3,4-tetrahydropyrido[3,4-b]pyrazin-7-yl]amino}-5-methyl-*N*-(tetrahydro-2H-pyran-4-yl)pyridin-3-carboxamid erhalten.
¹H-NMR (400 MHz, RT, DMSO-d6): δ = 0.78 (t, 3H); 1.41-1.53 (m, 1H); 1.53-1.70 (m, 5H); 1.71-1.87 (m, 6H); 1.94-2.12 (m, 2H); 2.32 (s, 3H); 3.31 (s, 3H); 3.38 (dt, 2H); 3.84-4.06 (m, 5H); 7.82 (s, 1H); 7.84 (s, 1H); 7.92 (d, 1H); 8.18 (s, 1H); 8.21 (d, 1H); 8.53 (d, 1H);

### Beispiel 18: 6-[[(2R)-1-Cyclopentyl-2-ethyl-1,2,3,4-tetrahydro-4-methyl-3-oxopyrido[3,4-b]pyrazin-7-yl]amino]-5-methyl-N-(1-methyl-4-piperidinyl)pyridin-3-carboxamid

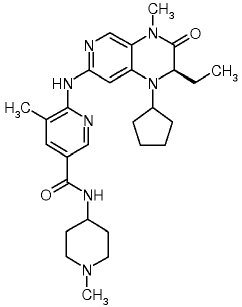

In Analogie zu Beispiel 3 wurden aus 51 mg Intermediat 5.3 und 43 mg 4-Amino-1-methylpiperidin 36 mg 6-[[(2*R*)-1-Cyclopentyl-2-ethyl-1,2,3,4-tetrahydro-4-methyl-3-oxopyrido[3,4-b]pyrazin-7-yl]amino]-5-methyl-*N*-(1-methyl-4-piperidinyl)pyridin-3-carboxamid erhalten.
¹H-NMR (400 MHz, RT, DMSO-d6): δ = 0.77 (t, 3H); 1.40-1.52 (m, 1H); 1.52-1.69 (m, 5H); 1.71-1.86 (m, 6H); 1.92 (dt, 2H); 1.96-2.11 (m, 2H); 2.15 (s, 3H); 2.31 (s, 3H); 2.76 (bd, 2H); 3.30 (s, 3H); 3.65-3.77 (m, 1H); 3.91 (qi, 1H); 4.04 (dd, 1H); 7.81 (s, 1H); 7.83 (s, 1H); 7.91 (d, 1H); 8.15 (d, 1H); 8.19 (s, 1H); 8.52 (d, 1H);

### Beispiel 19: 1N-Cyclopentyl-7-[[5-(4,5-dihydro-4,4-dimethyl-2-oxazolyl)-3-methoxy-2-pyridinyl]amino]-(2R)-ethyl-4N-methyl-1,4-dihydro-pyrido[3,4-b]pyrazin-3(2H)-on

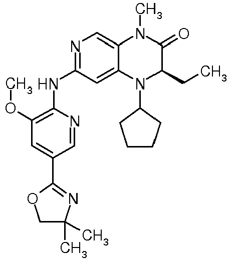

Eine Lösung von 45 mg Beispiel 11 in 4.1 ml THF wurde bei 0°C mit 65 mg Burgess Reagenz und 4.1 ml DMF versetzt. Nach 30 Minuten wurden 66.4 mg Natriumdihydrogenphosphat zugegeben und 14 Stunden bei Raumtemperatur gerührt. Es wurden weitere 2 Stunden bei 40°C und noch einmal 18 Stunden bei Raumtemepratur gerührt. Der Ansatz wurde auf gesättigte Natriumhydrogencarbonat-Lösung gegeben und mit Ethylacetat / Methanol extrahiert. Das Lösungsmittel wurde im Vakuum entfernt und der Rückstand aus Ethanol umkristallisiert. Man erhielt 29 mg 1*N*-Cyclopentyl-7-[[5-(4,5-dihydro-4,4-dimethyl-2-oxazolyl)-3-methoxy-2-pyridinyl]amino]-(2*R*)-ethyl-4*N*-methyl-1,4-dihydro-pyrido[3,4-b]pyrazin-3(2H)-on.
¹H-NMR (400 MHz, RT, DMSO-d6): δ = 0.76 (t, 3H); 1.29 (s, 6H); 1.44-1.56 (m, 1H); 1.57-1.75 (m, 3H); 1.77-1.92 (m, 4H); 1.96-2.11 (m, 2H); 3.30 (s, 1H); 3.90-4.00 (m+s, 1+3H); 4.05 (dd, 1H); 4.10 (s, 2H); 7.49 (d, 1H); 7.81 (s, 1H); 7.97 (s, 1H); 8.08 (s, 1H); 8.21 (d, 1H);

### Beispiel 20: N-Cyclohexyl-6-[[(2R)-1-cyclopentyl-2-ethyl-1,2,3,4-tetrahydro-4-methyl-3-oxopyrido[3,4-b]pyrazin-7-yl]amino]-5-methoxy-pyridin-3-carboxamid

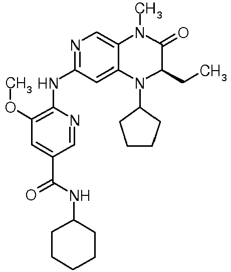

In Analogie zu Beispiel 3 wurden aus 50 mg Intermediat 4.2 und 35 mg Cyclohexanamin 26 mg *N-*Cyclohexyl-6-[[(2*R*)-1-cyclopentyl-2-ethyl-1,2,3,4-tetrahydro-4-methyl-3-oxopyrido[3,4-b]pyrazin-7-yl]amino]-5-methoxy-pyridin-3-carboxamid erhalten.
¹H-NMR (400 MHz, RT, DMSO-d6): δ = 0.77 (t, 3H); 1.07-1.20 (m, 1H); 1.31 (qi, 4H); 1.41-1.54 (m, 1H); 1.57-1.91 (3m, 12H); 1.97-2-13 (m, 2H); 3.30 (s, 3H); 3.78 (m, 1H); 3.91-4.00 (m+s, 1+3H); 4.04 (dd, 1H); 7.63 (d, 1H); 7.81 (s, 1H); 7.89 (s, 1H); 8.10 (s, 1H); 8.18 (d, 1H); 8.32 (d, 1H);

### Biologische Wirksamkeit der erfindungsgemäßen Verbindungen

### Protein-Protein Wechselwirkungsassay: Bindungsassay BRD4 / acetyliertes Peptid H4

### 1. Assay-Beschreibung BRD4-Bromodomäne 1 [BRD4(1)]

Zur Beurteilung der BRD4(1)-Bindungsstärke der in dieser Anmeldung beschriebenen Substanzen wurde deren Fähigkeit quantifiziert, die Wechselwirkung zwischen BRD4(1) und acetyliertem Histon H4 dosisabhängig zu hemmen.

Zu diesem Zweck wurde ein zeitaufgelöster Fluoreszenz-Resonanz-Energie-Transfer (TR-FRET) Assay verwendet, der die Bindung zwischen N-terminal His6-getaggtem BRD4(1) (Aminosäuren 67-152) und einem synthetischen acetylierten Histon H4 (Ac-H4) Peptid mit Sequenz GRGK(Ac)GGK(Ac)GLGK(Ac)GGAK(Ac)RHGSGSK-Biotin misst. Das nach Filippakopoulos et al., Cell, 2012, 149:214-231 im Haus produzierte rekombinante BRD4(1) Protein wurde in E. coli exprimiert und mittels (Ni-NTA) Affinitäts- und (Sephadex G-75) Größenausschlusschromatografie gereinigt. Das Ac-H4 Peptid kann von z.B. Biosyntan (Berlin, Deutschland) gekauft werden.

Im Assay wurden typischerweise 11 verschiedene Konzentrationen von jeder Substanz (0,1 nM, 0,33 nM, 1,1 nM, 3,8 nM, 13 nM, 44 nM, 0,15 µM, 0,51 µM, 1,7 µM, 5,9 µM und 20 µM) als Duplikate auf derselben Mikrotiter-Platte gemessen. Dafür wurden 100-fach konzentrierte Lösungen in DMSO vorbereitet durch serielle Verdünnungen (1:3,4) einer 2 mM Stammlösung in eine klare, 384-Well Mikrotiter-Platte (Greiner Bio-One, Frickenhausen, Germany). Daraus wurden 50 nl in eine schwarze Testplatte (Greiner Bio-One, Frickenhausen, Germany) überführt. Der Test wurde gestartet durch die Zufuhr von 2 µl einer 2,5-fach konzentrierten BRD4(1)-Lösung (üblicherweise 10 nM Endkonzentration in den 5 µl des Reaktionsvolums) in wässrigem Assaypuffer [50 mM HEPES pH 7.5, 50 mM Natriumchlorid (NaCl), 0,25 mM CHAPS und 0,05% Serumalbumin (BSA)] zu den Substanzen in der Testplatte. Darauf folgte ein 10-minütiger Inkubationsschritt bei 22°C für die Voräquilibrierung von putativen Komplexen zwischen BRD4(1) und den Substanzen. Anschließend wurden 3 µl einer 1,67-fach konzentrierten Lösung (im Assaypuffer) bestehend aus Ac-H4 Peptid (83,5 nM) und TR-FRET Detektionsreagenzien [16,7 nM Anti-6His-XL665 und 3,34 nM Streptavidin-Kryptat (beide von Cisbio Bioassays, Codolet, France), so wie 668 mM Kaliumfluorid (KF)] zugegeben.

Die Mischung wurde dann im Dunkeln für eine Stunde bei 22°C und anschließend für mindestens 3 Stunden und maximal über Nacht bei 4°C inkubiert. Die Bildung von BRD4(1) /Ac-H4 Komplexen wurde bestimmt durch die Messung des Resonanzenergietransfers von dem Streptavidin-Eu-Kryptat zum anti-6His-XL665 Antikörper der sich in der Reaktion befindet. Dafür wurden die Fluoreszenzemission bei 620 nm und 665 nm nach Anregung bei 330-350 nm in einem TR-FRET Messgerät, z.B. ein Rubystar oder Pherastar (beide von BMG Lab Technologies, Offenburg, Germany) oder ein Viewlux (Perkin-Elmer), gemessen. Das Verhältnis der Emission bei 665 nm und bei 622 nm (Ratio) wurde als Indikator für die Menge der gebildeten BRD4(1)/Ac-H4 Komplexe genommen.

Die erhaltenen Daten (Ratio) wurden normalisiert, wobei 0% Inhibition dem Mittelwert aus den Messwerten eines Satzes von Kontrollen (üblicherweise 32 Datenpunkte) entsprach, bei denen alle Reagenzien enthalten waren. Dabei wurden anstatt von Testsubstanzen 50 nl DMSO (100%) eingesetzt. Inhibition von 100% entsprach dem Mittelwert aus den Messwerten eines Satzes von Kontrollen (üblicherweise 32 Datenpunkte), bei denen alle Reagenzien außer BRD4(1) enthalten waren. Die Bestimmung des IC₅₀ Wertes erfolgte durch Regressionsanalyse auf Basis einer 4-Parameter Gleichung (Minimum, Maximum, IC₅₀, Hill; Y = Max + (Min - Max) / (1 + (X/IC₅₀)Hill).

### 2. Plk-1 Enzym-Assay

Für den Kinaseassay wird aus Insektenzellen (Hi5) exprimiertes, durch Glutathion-Sepharose-Affinitätschromatographie und anschließender Gel-filtration (Superdex 75) gereinigtes rekombinantes Fusionsprotein bestehend aus GST und Plk (Kinase Domäne 33-345; MW 36 kDa, conc 0,8 µg/µl) verwendet. Aliquots hiervon werden in flüssigem Stickstoff weggefroren und bei - 80°C gelagert und nach dem Auftauen nur einmal verwendet.

Bei dem verwendeten Assay handelt es sich um einen indirekten HTRF-Assay, bei dem folgende Materialien und Verfahrensweisen zum Einsatz kamen.
Als Substrat für die Kinasereaktion wird das biotinylierte Peptid Btn-Ahx-KKLNRTLSFAEPG-Amid x TFA, von Biosyntan, Probennr.: 6178.1 (C-Terminus in Amidform) verwendet. Hierbei handelt es sich um eine artifizielle Sequenz, die von keinem bekannten Protein abgeleitet wurde.50 nl der in 100% Dimethylsulfoxid (DMSO) gelösten Testverbindungen (Endkonzentrationen : 0 µM und Konzentrationen im Bereich von 0,001 - 20 µM) werden mit 2 µl Plk-1 Enzym Arbeitslösung im Arbeitspuffer [25 mM MgCl₂; 1mM DTT; 50 mM Hepes pH 7,0; 0,01% NP40; 1x Complete; 0,05% BSA] für 30 min vorinkubiert. Anschließend wird die Kinase-Reaktion durch Zugabe von 3 µl Substratlösung [Adenosintriphosphate (ATP) und 1,4 µM Substratpeptid (Biotin-Ttds-KKLNRTLSFAEPG -NH2)] in Arbeitspuffer gestartet, und nach 30 min durch Zugabe einer

Abstopp-Lösung (100 mM EDTA, 100 mM Hepes pH 7,5, 800 mM Kaliumfluorid, 0,12% BSA, 0,4 µM SA-XLent (0,05 µM, von CIS bio international, Marcoule, Frankreich), Eu³⁺ Cryptate-conjugated Rabbit anti-Mouse IgG (1,5 nM; ein mit Europiumkryptat markierter Anti-Maus-IgG-Antikörper von CIS bio international, Marcoule, Frankreich), 1 nM Anti-phospho-Serine Kinase (ein phospho-spezifischer Antikörper von Upstate Biotechnology, Dundee, Schottland), gestoppt, und bei 4°C über Nacht inkubiert.
Für den Versuch mit niedriger ATP Konzentration werden eine 1,25 ng/µl Plk-1 Arbeitslösung und 16,7 µM ATP verwendet, für den Versuch mit hoher ATP Konzentration werden eine 0,039 ng/µl Plk-1 Arbeitslösung und 16,7 mM ATP verwendet.

Danach wird die Menge des phosphorylierten Substratpeptids durch Messung des Resonanzenergietransfers vom Europium-markierten Antikörperkomplex zum Streptavidine-XLent bestimmt. Zu diesem Zweck wird die Fluoreszenzemission bei 620 nm und 665 nm nach Anregung bei 350 nm in einem HTRF-Messgerät, z. B. Rubystar (BMG Labtechnologies, Offenburg, Deutschland) oder Viewlux (Perkin-Elmer), bestimmt. Das Verhältnis der Emissionen bei 665 nm und bei 620 nm wird als Maß für die Menge des phosphorylierten Substratpeptids verwendet. Die Daten werden normalisiert (Enzymreaktion ohne Inhibitor = 0 % Inhibition, alle anderen Assaykomponenten, aber kein Enzym = 100 % Inhibition), und IC₅₀-Werte werden mit einer 4 Parameter-Gleichung berechnet (Minimum, Maximum, IC₅₀, Hill; Y = Max + (Min - Max) / (1 + (X/IC50)Hill)).

### 4. Zell-Assay

### Zellproliferationsassay

In Übereinstimmung mit der Erfindung, wurde die Fähigkeit der Substanzen die Proliferation der MOLM-13 Zelllinie (Deutsche Sammlung für Mikroorganismen und Zellkulturen, ACC 554) zu hemmen bestimmt. Die Zellviabilität wurde mittels des alamarBlue® Reagenz (Invitrogen) in einem Victor X3 Multilabel Reader (Perkin Elmer) bestimmt. Die Anregungswellenlänge war 530 nm und die Emissionswellenlänge 590 nM.

Die MOLM-13 Zellen wurden in einer Dichte von 4000 Zellen/Well in 100µl Wachstumsmedium auf 96well Microtiterplatten ausgesät. Nach einer Übernachtinkubation bei 37°C wurden die Fluoreszenzwerte bestimmt (CI Werte). Dann wurden die Platten mit verschiedenen Substanzverdünnungen behandelt und während 96 Stunden bei 37°C inkubiert. Anschließend wurden die Fluoreszenzwerte bestimmt (CO Werte). Für die Datenanalyse wurden die CI Werte von den CO Werten abgezogen und die Ergebnisse verglichen zwischen Zellen, die mit verschiedenen Verdünnungen der Substanz oder nur mit Pufferlösung behandelt wurden. Die IC₅₀-Werte (Substanzkonzentration, die für eine 50%ige Hemmung der Zellproliferation notwendig ist) wurden daraus berechnet.

### 5. Ergebnisse:

### 5.1 Bindungsassay

Die Tabelle 1 zeigt die Ergebnisse aus dem BRD4(1) Bindungsassay.

**Tabelle 1**

| Beispiel | IC₅₀ [BRD4(1)] (nmol/l) |
|---|---|
| 1 | 204 |
| 2 | 126 |
| 3 | 199 |
| 4 | 450 |
| 5 | 225 |
| 6 | 243 |
| 7 | 276 |
| 8 | 333 |
| 9 | 343 |
| 10 | 376 |
| 11 | 393 |
| 12 | 424 |
| 13 | 434 |
| 14 | 458 |
| 15 | 496 |
| 16 | 525 |
| 17 | 539 |
| 18 | 649 |
| 19 | 732 |
| 20 | 878 |

### 5.2 Kinase Aktivitätsassay

Die Tabelle 2 zeigt die Ergebnisse des Plk-1-Assays bei 10 µM ATP.

**Tabelle 2**

| Beispiel | IC₅₀ [Plk-1] (nmol/l, 10 µM ATP) |
|---|---|
| 1 | 15 |
| 2 | 17 |
| 3 | 8 |
| 5 | 7 |
| 7 | 11 |
| 8 | 9 |
| 13 | 7 |
| 14 | 11 |
| 15 | 13 |
| 17 | 8 |

### 5.3 Kinase Aktivitätsassay

Die Tabelle 3 zeigt die Ergebnisse des Plk-1-Assays bei 10 mM ATP.

**Tabelle 3**

| Beispiel | IC₅₀ [Plk-1] (nmol/l, 10mM ATP) |
|---|---|
| 3 | 17 |
| 4 | 31 |
| 5 | 21 |
| 6 | 19 |
| 7 | 30 |
| 8 | 22 |
| 9 | 17 |
| 10 | 31 |
| 11 | 57 |
| 12 | 23 |
| 13 | 15 |
| 14 | 29 |
| 15 | 22 |
| 16 | 22 |
| 17 | 14 |
| 18 | 7 |
| 19 | 99 |
| 20 | 35 |

### 5.4 Zell-Proliferationsassay

Die Tabelle 4 zeigt die Ergebnisse aus dem MOLM-13 Zellproliferationsassays.

**Tabelle 4**

| Es wurde die Fähigkeit der erfindungsgemäßen Verbindungen die Proliferation der MOLM-13 Zelllinie zu hemmen bestimmt. | |
|---|---|
| Beispiel | IC₅₀ (MOLM-13) (nmol/l) |
| 1 | 209 |
| 2 | 93 |
| 3 | 61 |
| 4 | 68 |
| 5 | 29 |
| 6 | 114 |
| 7 | 80 |
| 8 | 72 |
| 9 | 76 |
| 10 | 43 |
| 11 | 74 |
| 12 | 52 |
| 13 | 56 |
| 14 | 32 |
| 15 | 77 |
| 16 | 77 |
| 17 | 55 |
| 18 | 42 |
| 19 | 362 |
| 20 | 101 |

## Patentansprüche

1. Verbindungen der allgemeinen Formel (I) 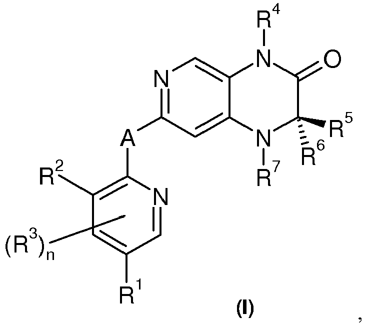 in der
A für -NH- oder -O- steht,
R¹ für eine Gruppe -C(=O)NR⁸R⁹ oder -S(=O)₂NR⁸R⁹ steht,
oder
für Oxazolin-2-yl-, das gegebenenfalls ein- oder zweifach substituiert sein kann mit C₁-C₃-Alkyl-,
oder
für 5-gliedriges monocyclisches Heteroaryl- steht, das gegebenenfalls ein-, zwei- oder dreifach, gleich oder verschieden substituiert sein kann mit Halogen, Cyano, C₁-C₄-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio-, Halogen-C₁-C₄-alkylthio-, -NR¹⁰R¹¹, -C(=O)OR¹², -C(=O)N¹⁰R¹¹, -C(=O)R¹², -S(=O)₂R¹²,-S(=O)₂NR¹⁰R¹¹,
R² für Wasserstoff, Halogen, Cyano, C₁-C₄-Alkyl-, C₂-C₄-Alkenyl-, C₂-C₄-Alkinyl-, Halogen-C₁-C₄-alkyl-, C₁-C₄-Alkoxy-, Halogen-C₁-C₄-alkoxy-, C₁-C₄-Alkylthio- oder Halogen-C₁-C₄-alkylthio- steht,
R³ für Halogen, C₁-C₃-Alkyl-, C₁-C₃-Alkoxy- oder Cyano steht,
R⁴ für Methyl- oder Ethyl- steht,
R⁵ für Wasserstoff oder C₁-C₃-Alkyl- steht,
R⁶ für Wasserstoff oder C₁-C₃-Alkyl steht,
oder
R⁵ und R⁶ gemeinsam mit dem Kohlenstoffatom, an das sie gebunden sind, für C₃-C₆-Cycloalkyl stehen,
R⁷ für C₁-C₆-Alkyl- steht, das gegebenenfalls einfach substituiert sein kann mit Phenyl-, C₃-C₈-Cycloalkyl-, oder 4-bis 8-gliedrigem Heterocycloalkyl-, worin Phenyl- seinerseits gegebenenfalls ein-, zwei- oder dreifach, gleich oder verschieden substituiert sein kann mit: Halogen, Cyano, C₁-C₄-Alkyl-, C₂-C₄-Alkenyl-, C₂-C₄-Alkinyl-, C₁-C₄-Alkoxy-, Halogen-C₁-C₄-Alkyl-, Halogen-C₁-C₄-Alkoxy-,
und
worin C₃-C₈-Cycloalkyl- und 4-bis 8-gliedriges Heterocycloalkyl-ihrerseits gegebenenfalls ein- oder zweifachsubstituiert sein können mit C₁-C₃-Alkyl-,
oder
für C₃-C₈-Cycloalkyl- oder 4-bis 8-gliedriges Heterocycloalkyl- steht, die gegebenenfalls ein- oder zweifach, substituiert sein können mit C₁-C₃-Alkyl-,
R⁸ für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₄-Alkoxy-, Halogen-C₁-C₄-alkoxy-, -NR¹⁰R¹¹, C₃-C₈-Cycloalkyl-, C₄-C₈-Cycloalkenyl-, 4- bis 8-gliedrigem Heterocycloalkyl, 4- bis 8-gliedrigem Heterocycloalkenyl-, C₅-C₁₁-Spirocycloalkyl-, C₅-C₁₁-Heterospirocycloalkyl-, verbrücktem C₆-C₁₂-Cycloalkyl-, verbrücktem C₆-C₁₂-Heterocycloalkyl-, C₆-C₁₂-Bicycloalkyl-, C₆-C₁₂-Heterobicycloalkyl-, Phenyl- oder 5- bis 6-gliedrigem Heteroaryl-, worin C₃-C₈-Cycloalkyl-, C₄-C₈-Cycloalkenyl-, 4- bis 8-gliedriges Heterocycloalkyl-, 4- bis 8-gliedriges Heterocycloalkenyl-, C₅-C₁₁-Spirocycloalkyl-, C₅-C₁₁-Heterospirocycloalkyl-, verbrücktes C₆-C₁₂-Cycloalkyl-, verbrücktes C₆-C₁₂-Heterocycloalkyl-, C₆-C₁₂-Bicycloalkyl-, C₆-C₁₂-Heterobicycloalkyl- gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR¹⁰R¹¹,
und
worin Phenyl- und 5- bis 6-gliedriges Heteroaryl- gegebenenfalls ein- oder zweifach, gleich oder verschieden substituiert sein können mit: Halogen, Cyano, Trifluormethyl-, C₁-C₃-Alkyl-, C₁-C₃-Alkoxy-, oder für C₃-C₆-Alkenyl- oder C₃-C₆-Alkinyl- steht,
oder für Fluor-C₁-C₃-Alkyl- steht, das gegebenenfalls einfach substituiert sein kann durch Cyano oder Hydroxy,
oder für C₃-C₈-Cycloalkyl- C₄-C₈-Cycloalkenyl-, C₅-C₁₁-Spirocycloalkyl-, verbrücktes C₆-C₁₂-Cycloalkyl- oder C₆-C₁₂-Bicycloalkyl- steht, die gegebenenfalls ein- oder zweifach, gleich oder verschieden substituiert sein können mit: Hydroxy, Oxo, Cyano, Fluor, C₁-C₃-Alkyl, C₁-C₃-Alkoxy, Trifluormethyl, -NR¹⁰R¹¹,
oder für 4- bis 8-gliedriges Heterocycloalkyl-, 4- bis 8-gliedriges Heterocycloalkenyl-, C₅-C₁₁-Heterospirocycloalkyl-, verbrücktem C₆-C₁₂-Heterocycloalkyl- oder C₆-C₁₂-Heterobicycloalkyl- steht, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl-, C₁-C₄-Alkoxycarbonyl- oder -NR¹⁰R¹¹,
R⁹ für Wasserstoff oder für gegebenenfalls ein- oder zweifach, gleich oder verschieden mit Hydroxy, Oxo, C₁-C₃-Alkoxy- substituiertes C₁-C₃-Alkyl-, oder für Fluor-C₁-C₃-Alkyl steht,
oder
R⁸ und R⁹ gemeinsam mit dem Stickstoffatom, an welches sie gebunden sind, für 4-bis 8-gliedriges Heterocycloalkyl, 4-bis 8-gliedriges Heterocycloalkenyl-, C₅-C₁₁-Heterospirocycloalkyl-, verbrücktes C₆-C₁₂-Heterocycloalkyl- oder C₆-C₁₂-Heterobicycloalkyl- stehen, die gegebenenfalls ein- oder zweifach, gleich oder verschieden substituiert sein können mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₅-Alkylcarbonyl-, C₁-C₄-Alkoxycarbonyl-oder -NR¹⁰R¹¹,
R¹⁰ und R¹¹ unabhängig voneinander für Wasserstoff oder für gegebenenfalls ein- oder zweifach, gleich oder verschieden mit Hydroxy, Oxo, C₁-C₃-Alkoxy-substituiertes C₁-C₃-Alkyl, oder für Fluor-C₁-C₃-Alkyl stehen,
oder
R¹⁰ und R¹¹ gemeinsam mit dem Stickstoffatom, an das sie gebunden sind, für 4- bis 8-gliedriges Heterocycloalkyl- stehen, das gegebenenfalls ein- oder zweifach, gleich oder verschieden substituiert sein kann mit: Hydroxy, Fluor, Oxo, Cyano, C₁-C₃-Alkyl-, Fluor-C₁-C₃-Alkyl-, C₃-C₆-Cycloalkyl-, Cyclopropylmethyl-, C₁-C₃-Alkylcarbonyl- oder C₁-C₄-Alkoxycarbonyl-,
R¹² für C₁-C₆-Alkyl- oder Phenyl-C₁-C₃-Alkyl- steht, und
n für 0 oder 1 steht,
sowie deren Diastereomere, Racemate, Polymorphe und physiologisch verträglichen Salze.

2. Verbindungen der allgemeinen Formel (I) gemäß Anspruch 1, in der
A für -NH- steht,
R¹ für eine Gruppe -C(=O)NR⁸R⁹ oder -S(=O)₂NR⁸R⁹ steht,
oder
für Oxazolin-2-yl- steht, das gegebenenfalls ein- oder zweifach substituiert sein kann mit C₁-C₃-Alkyl-,
oder
für Oxazolyl-, Thiazolyl-, Oxadiazolyl- oder Thiadiazolyl- steht, die gegebenenfalls ein- oder zweifach, gleich oder verschieden substituiert sein können mit Halogen, Cyano, C₁-C₃-Alkyl-, Trifluormethyl-, C₁-C₃-Alkoxy-, Trifluormethoxy- oder -NR¹⁰R¹¹,
R² für Wasserstoff, Fluor, Chlor, Cyano, Methyl-, Ethyl-, Methoxy- oder Ethoxy- steht,
R³ für Fluor, Chlor oder Methyl- steht,
R⁴ für Methyl- steht,
R⁵ für Wasserstoff, Methyl- oder Ethyl- steht,
R⁶ für Wasserstoff, Methyl- oder Ethyl- steht,
R⁷ für C₃-C₅-Alkyl- steht,
oder
für Methyl- oder Ethyl- steht, die einfach substituiert sein können mit Phenyl- oder 4-bis 8-gliedrigem Heterocycloalkyl-, worin Phenyl- seinerseits gegebenenfalls ein- oder zweifach, gleich oder verschieden substituiert sein kann mit: Fluor, Chlor, Brom, Cyano, C₁-C₃-Alkyl-, C₁-C₃-Alkoxy-, Trifluoromethyl-,
und
worin 4-bis 8-gliedriges Heterocycloalkyl- seinerseits gegebenenfalls ein- oder zweifach substituiert sein kann mit Methyl-,
oder
für C₃-C₆-Cycloalkyl- oder 4-bis 8-gliedriges Heterocycloalkyl- steht, die gegebenenfalls ein- oder zweifach substituiert sein können mit Methyl-,
R⁸ für C₁-C₆-Alkyl- steht, das gegebenenfalls ein-, zwei-, oder dreifach, gleich oder verschieden substituiert sein kann mit: Hydroxy, Oxo, Fluor, Cyano, C₁-C₃-Alkoxy-, Fluor-C₁-C₃-alkoxy-, -NR¹⁰R¹¹, 4- bis 8-gliedrigem
Heterocycloalkyl-, Phenyl- oder 5- bis 6-gliedrigem Heteroaryl-, worin das 4- bis 8-gliedrige Heterocycloalkyl- gegebenenfalls einfach substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert-*Butoxycarbonyl-,
und worin Phenyl- und 5- bis 6-gliedriges Heteroaryl- gegebenenfalls ein- oder zweifach, gleich oder verschieden substituiert sein können mit: Fluor, Chlor, Cyano, Trifluormethyl-, Methyl-, Methoxy-, oder für Fluor-C₁-C₃-Alkyl- steht,
oder für C₃-C₆-Cycloalkyl- steht, das gegebenenfalls ein- oder zweifach, gleich oder verschieden substituiert sein kann mit: Hydroxy, Oxo, Cyano, Fluor, -NR¹⁰R¹¹,
oder für 4- bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkyl- steht, die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
R⁹ für Wasserstoff oder C₁-C₃-Alkyl- steht, oder
R⁸ und R⁹ gemeinsam mit dem Stickstoffatom, an welches sie gebunden sind, für 4-bis 8-gliedriges Heterocycloalkyl-, C₆-C₈-Heterospirocycloalkyl-, verbrücktes C₆-C₁₀-Heterocycloalkyl- oder C₆-C₁₀-Heterobicycloalkylstehen,
die gegebenenfalls ein- oder zweifach, gleich oder verschieden substituiert sein können mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-,
R¹⁰ und R¹¹ unabhängig voneinander für Wasserstoff oder für gegebenenfalls einfach mit Hydroxy oder Oxo substituiertes C₁-C₃-Alkyl- oder für Trifluormethylstehen,
oder
R¹⁰ und R¹¹ gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Hydroxy, Oxo, C₁-C₃-Alkyl-, Fluor-C₁-C₃-Alkyl-, Cyclopropyl-, Cyclopropylmethyl-, Acetyl- oder *tert*-Butoxycarbonyl-, und
n für 0 oder 1 steht
sowie deren Diastereomere, Racemate, Polymorphe und physiologisch verträglichen Salze.

3. Verbindungen der allgemeinen Formel I gemäß den Ansprüchen 1 und 2, in der
A für -NH- steht,
R¹ für eine Gruppe -C(=O)NR⁸R⁹ oder -S(=O)₂NR⁸R⁹ steht,
oder
für Oxazolin-2-yl- steht, das gegebenenfalls ein- oder zweifach substituiert sein kann mit C₁-C₃-Alkyl-,
R² für Wasserstoff, Methyl-, Ethyl- oder Methoxy- steht,
R⁴ für Methyl- steht,
R⁵ für Methyl- oder Ethyl- steht,
R⁶ für Wasserstoff steht,
R⁷ für C₃-C₅-Alkyl- steht,
oder
für Methyl- steht, das einfach substituiert ist mit Phenyl- oder 4-bis 6-gliedrigem Heterocycloalkyl-,
worin Phenyl- seinerseits gegebenenfalls ein-, oder zweifach, gleich oder verschieden substituiert sein kann mit: Fluor, Chlor, Cyano, Methyl-, Methoxy-,
und
worin 4-bis 6-gliedriges Heterocycloalkyl- seinerseits gegebenenfalls einfach substituiert sein kann mit Methyl-,
oder
für C₃-C₆-Cycloalkyl- oder für 4-bis 6-gliedriges Heterocycloalkyl- steht,
R⁸ für C₁-C₄-Alkyl- steht, das gegebenenfalls ein- oder zweifach substituiert sein kann mit Hydroxy, C₁-C₃-Alkoxy-, -NR¹⁰R¹¹, 4- bis 8-gliedrigem Heterocycloalkyl-, Phenyl- oder 5- bis 6-gliedrigem Heteroaryl-, worin das 4- bis 8-gliedrige Heterocycloalkyl- gegebenenfalls einfach substituiert sein kann mit: Oxo, C₁-C₃-Alkyl-, Fluor-C₁-C₃-Alkyl-, Cyclopropyl- oder Cyclopropylmethyl-,
und worin Phenyl- und 5- bis 6-gliedriges Heteroaryl- gegebenenfalls ein- oder zweifach, gleich oder verschieden substituiert sein können mit: Fluor, Chlor, Cyano, Trifluormethyl-, Methyl- oder Methoxy-, oder für Fluor-C₁-C₃-Alkyl- steht,
oder für C₃-C₆-Cycloalkyl- steht, das gegebenenfalls einfach substituiert sein kann mit Hydroxy, Fluor oder -NR¹⁰R¹¹,
oder für 4- bis 8-gliedriges Heterocycloalkyl-, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
R⁹ für Wasserstoff oder Methyl- steht,
oder
R⁸ und R⁹ gemeinsam mit dem Stickstoffatom, an welches sie gebunden sind, für 5-bis 6-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-,
R¹⁰ und R¹¹ unabhängig voneinander für Wasserstoff oder für C₁-C₃-Alkyl- stehen,
oder
R¹⁰ und R¹¹ gemeinsam mit dem Stickstoffatom, an das sie gebunden sind, für 4- bis 7-gliedriges Heterocycloalkyl- stehen, das gegebenenfalls ein- oder zweifach, gleich oder verschieden substituiert sein kann mit: Oxo, C₁-C₃-Alkyl-, Fluor-C₁-C₃-Alkyl-, Cyclopropyl- oder Cyclopropylmethyl-, und
n für 0 steht,
sowie deren Diastereomere, Racemate, Polymorphe und physiologisch verträglichen Salze.

4. Verbindungen der allgemeinen Formel I gemäß den Ansprüchen 1 bis 3, in der
A für -NH- steht,
R¹ für eine Gruppe -C(=O)NR⁸R⁹ steht,
oder
für Oxazolin-2-yl- steht, das gegebenenfalls ein- oder zweifach substituiert sein kann mit C₁-C₃-Alkyl-,
R² für Methyl-, Ethyl- oder Methoxy- steht,
R⁴ für Methyl- steht,
R⁵ für Methyl- oder Ethyl- steht,
R⁶ für Wasserstoff steht,
R⁷ für C₃-C₅-Alkyl- steht,
oder
für C₃-C₆-Cycloalkyl- steht,
R⁸ für C₁-C₃-Alkyl- steht, das gegebenenfalls einfach substituiert sein kann mit Hydroxy, C₁-C₃-Alkoxy-, Phenyl- oder 5- bis 6-gliedrigem Heteroaryl-, worin Phenyl- und 5- bis 6-gliedriges Heteroaryl- gegebenenfalls ein- oder zweifach, gleich oder verschieden substituiert sein können mit: Fluor,
Chlor, Methyl- oder Methoxy-,
oder für Fluor-C₁-C₃-Alkyl- steht,
oder für C₃-C₆-Cycloalkyl- steht,
oder für 4- bis 8-gliedriges Heterocycloalkyl-, das gegebenenfalls ein- oder zweifach, gleich oder verschieden substituiert sein kann mit: Oxo oder C₁-C₃-Alkyl-,
R⁹ für Wasserstoff steht,
n für 0 steht,
sowie deren Diastereomere, Racemate, Polymorphe und physiologisch verträglichen Salze.

5. Verbindungen der allgemeinen Formel I gemäß den Ansprüchen 1 bis 4, in der
A für -NH- steht,
R¹ für eine Gruppe -C(=O)NR⁸R⁹ steht,
oder
für Oxazolin-2-yl- steht, das gegebenenfalls ein- oder zweifach substituiert sein kann mit Methyl-,
R² für Methyl-, Ethyl- oder Methoxy- steht,
R⁴ für Methyl- steht,
R⁵ für Methyl- oder Ethyl- steht,
R⁶ für Wasserstoff steht,
R⁷ für Cyclopentyl- steht,
R⁸ für C₁-C₄-Alkyl- steht, das gegebenenfalls einfach substituiert sein kann mit Hydroxy, Methoxy- oder Pyridinyl-,
oder für Fluor-C₁-C₂-Alkyl- steht,
oder für C₃-C₆-Cycloalkyl- steht,
oder für 4- bis 8-gliedriges Heterocycloalkyl-, das gegebenenfalls ein- oder zweifach, gleich oder verschieden substituiert sein kann mit: Oxo oder C₁-C₃-Alkyl-,
R⁹ für Wasserstoff steht,
n für 0 steht,
sowie deren Diastereomere, Racemate, Polymorphe und physiologisch verträglichen Salze.

6. Verbindungen der allgemeinen Formel I gemäß den Ansprüchen 1 bis 5, in der
A für -NH- steht,
R¹ für eine Gruppe -C(=O)NR⁸R⁹ steht,
oder
für Oxazolin-2-yl- steht, das zweifach substituiert ist mit Methyl-,
R² für Methyl-, Ethyl- oder Methoxy- steht,
R⁴ für Methyl- steht,
R⁵ für Methyl- oder Ethyl- steht,
R⁶ für Wasserstoff steht,
R⁷ für Cyclopentyl- steht,
R⁸ für C₁-C₄-Alkyl- steht, das gegebenenfalls einfach substituiert sein kann mit Hydroxy, Methoxy- oder Pyridinyl-,
oder für 2,2,2-Trifluorethyl- steht,
oder für Cyclopropyl- oder Cyclohexyl- steht,
oder für Piperidinyl, Azepanyl oder Tetrahydropyranyl, die gegebenenfalls ein- oder zweifach, gleich oder verschieden substituiert sein können mit:
Oxo oder Methyl,
R⁹ für Wasserstoff steht,
n für 0 steht,
sowie deren Diastereomere, Racemate, Polymorphe und physiologisch verträglichen Salze.

7. Verbindungen der allgemeinen Formel I gemäß den Ansprüchen 1 bis 6
6-{[1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid,
6-{[(2*R*)-1-Cyclopentyl-2,4-dimethyl-3-oxo-1,2,3,4-tetrahydropyrido[3,4-b]pyrazin-7-yl]amino}-N-cyclopropyl-5-methoxypyridin-3-carboxamid,
6-{[(2*R*)-1-Cyclopentyl-2-ethyl-4-methyl-3-oxo-1,2,3,4-tetrahydropyrido[3,4-b]pyrazin-7-yl]amino}-N-cyclopropyl-5-ethylpyridin-3-carboxamid,
6-[(1-Cyclopentyl-2-ethyl-1,2,3,4-tetrahydro-4-methyl-3-oxopyrido[3,4-b]pyrazin-7-yl)amino]-N-cyclopropyl-5-methoxy-3-pyridincarboxamid,
6-[[(2*R*)-1-Cyclopentyl-2-ethyl-1,2,3,4-tetrahydro-4-methyl-3-oxopyrido[3,4-b]pyrazin-7-yl)amino]-N-cyclopropyl-5-methoxy-3-pyridincarboxamid,
6-[(1-Cyclopentyl-2,4-dimethyl-3-oxo-1,2,3,4-tetrahydropyrido[3,4-b]pyrazin-7-yl)amino]-5-methoxy-N-(1-methylpiperidin-4-yl)pyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(2,2,2-trifluoroethyl)-3-pyridincarboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(2-methoxyethyl)-3-pyridincarboxamid,
6-{[(2*R*)-1-Cyclopentyl-2-ethyl-4-methyl-3-oxo-1,2,3,4-tetrahydropyrido[3,4-b]pyrazin-7-yl]amino}-5-ethyl-*N*-[(3*R*)-2-oxoazepan-3-yl]pyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(tetrahydro-2H-pyran-4-yl)-3-pyridincarboxamid,
6-[[(2*R*)-1-Cyclopentyl-2-ethyl-1,2,3,4-tetrahydro-4-methyl-3-oxopyrido[3,4-b]pyrazin-7-yl]amino]-*N*-(2-hydroxy-1,1-dimethylethyl)-5-methoxy-3-pyridincarboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(3-pyridinylmethyl)-3-pyridincarboxamid,
6-{[(2*R*)-1-Cyclopentyl-2-ethyl-4-methyl-3-oxo-1,2,3,4-tetrahydropyrido[3,4-b]pyrazin-7-yl]amino}-*N*-cyclopropyl-5-methylpyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-5-methoxy-*N*-(1-methyl-4-piperidinyl)-3-pyridincarboxamid,
6-{[(2*R*)-1-Cyclopentyl-2-ethyl-4-methyl-3-oxo-1,2,3,4-tetrahydropyrido[3,4-b]pyrazin-7-yl]amino}-5-methylpyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-*N*-[(3*R*)-hexahydro-2-oxo-1H-azepin-3-yl]-5-methoxy-3-pyridincarboxamid,
6-{[(2*R*)-1-Cyclopentyl-2-ethyl-4-methyl-3-oxo-1,2,3,4-tetrahydropyrido[3,4-b]pyrazin-7-yl]amino}-5-methyl-*N*-(tetrahydro-2H-pyran-4-yl)pyridin-3-carboxamid,
6-[[(2*R*)-1-Cyclopentyl-2-ethyl-1,2,3,4-tetrahydro-4-methyl-3-oxopyrido[3,4-b]pyrazin-7-yl]amino]-5-methyl-*N*-(1-methyl-4-piperidinyl)pyridin-3-carboxamid,
1*N*-Cyclopentyl-7-[[5-(4,5-dihydro-4,4-dimethyl-2-oxazolyl)-3-methoxy-2-pyridinyl]amino]-(2*R*)-ethyl-4*N*-methyl-1,4-dihydro-pyrido[3,4-b]pyrazin-3(2H)-on,
*N*-Cyclohexyl-6-[[(2*R*)-1-cyclopentyl-2-ethyl-1,2,3,4-tetrahydro-4-methyl-3-oxopyrido[3,4-b]pyrazin-7-yl]amino]-5-methoxy-pyridin-3-carboxamid.

8. Verbindungen gemäß den Ansprüchen 1 bis 7 zur Verwendung als Arzneimittel.

9. Verbindungen gemäß den Ansprüchen 1 bis 7, zur Verwendung in der Prophylaxe und/oder Therapie von Tumorerkrankungen.

10. Verwendung der Verbindungen gemäß den Ansprüchen 1 bis 7, zur Herstellung eines Arzneimittels.

11. Verwendung der Verbindungen gemäß den Ansprüchen 1 bis 7 zur Herstellung eines Arzneimittels zur Prophylaxe und/oder Therapie von Tumorerkrankungen.

12. Verbindungen gemäß den Ansprüchen 1 bis 7, zur Verwendung in der Prophylaxe und/oder Therapie von hyperproliferativen Erkrankungen.

13. Verbindungen gemäß den Ansprüchen 1 bis 7 zur Verwendung in der Prophylaxe und/ oder Therapie von viralen Infektionen, neurodegenerativen Erkrankungen, inflammatorischen Erkrankungen, atherosklerotischen Erkrankungen und in der männlichen Fertilitätskontrolle.

14. Verwendung der Verbindungen gemäß den Ansprüchen 1 bis 7 zur Herstellung eines Arzneimittels zur Prophylaxe und/ oder Therapie von viralen Infektionen, neurodegenerativen Erkrankungen, inflammatorischen Erkrankungen, atherosklerotischen Erkrankungen und in der männlichen Fertilitätskontrolle.

15. Verbindungen gemäß den Ansprüchen 1 bis 7 in Kombination mit ein oder mehreren weiteren pharmakologisch wirksamen Substanzen.

16. Verbindungen gemäß den Ansprüchen 1 bis 7 in Kombination mit ein oder mehreren weiteren pharmakologisch wirksamen Substanzen, zur Verwendung in der Prophylaxe und/oder Therapie von hyperproliferativen Erkrankungen.

17. Verbindungen gemäß den Ansprüchen 1 bis 7 in Kombination mit ein oder mehreren weiteren pharmakologisch wirksamen Substanzen, zur Verwendung in der Prophylaxe und/oder Therapie von Tumorerkrankungen.

18. Verbindungen gemäß den Ansprüchen 1 bis 7 in Kombination mit ein oder mehreren weiteren pharmakologisch wirksamen Substanzen, zur Verwendung in der Prophylaxe und/oder Therapie von viralen Infektionen, neurodegenerativen Erkrankungen, inflammatorischen Erkrankungen, atherosklerotischen Erkrankungen und in der männlichen Fertilitätskontrolle.

19. Verbindungen der allgemeinen Formel (VIII), 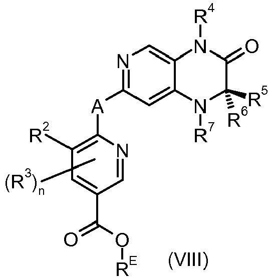 in der A, R², R³, R⁴, R⁵, R⁶, R⁷ und n die in der allgemeinen Formel (I) angegebenen Bedeutungen haben und R^{E} für C₁-C₆-Alkyl steht.

20. Verbindungen der allgemeinen Formel (IX) 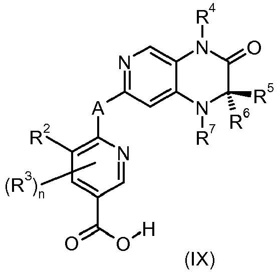 in der A, R², R³, R⁴, R⁵, R⁶, R⁷ und n die in der allgemeinen Formel (I) angegebenen Bedeutungen haben.

21. Verwendung der Verbindungen der allgemeinen Formel (VIII), 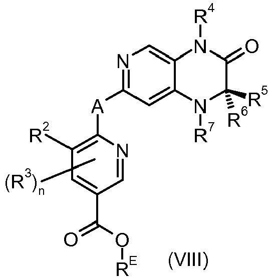 in der A, R², R³, R⁴, R⁵, R⁶, R⁷ und n die in der allgemeinen Formel (I) angegebenen Bedeutungen haben und R^{E} für C₁-C₆-Alkyl steht, zur Herstellung der erfindungsgemäßen Verbindungen der allgemeinen Formel (I).

22. Verwendung der Verbindungen der allgemeinen Formel (IX) 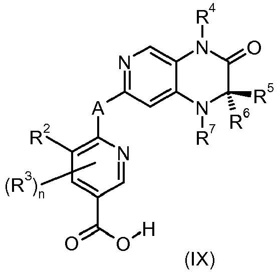 in der A, R², R³, R⁴, R⁵, R⁶, R⁷ und n die in der allgemeinen Formel (I) angegebenen Bedeutungen haben, zur Herstellung der erfindungsgemäßen Verbindungen der allgemeinen Formel (I).

## Claims

1. Compounds of the general formula (I) 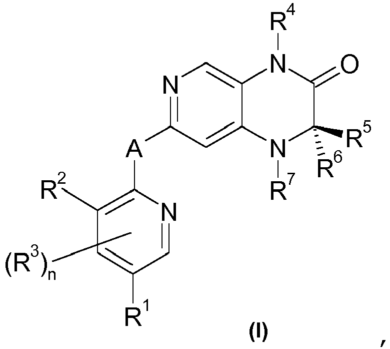 in which
A represents -NH- or -O-,
R¹ represents a -C(=O)NR⁸R⁹ or -S(=O)₂NR⁸R⁹ group,
or
represents oxazolin-2-yl which may optionally be mono- or disubstituted by C₁-C₃-alkyl-,
or
represents 5-membered monocyclic heteroaryl- which may optionally be mono-, di- or trisubstituted by identical or different substituents from the group consisting of halogen, cyano, C₁-C₄-alkyl-, C₂-C₄-alkenyl-, C₂-C₄-alkynyl-, halo-C₁-C₄-alkyl-, C₁-C₄-alkoxy-, halo-C₁-C₄-alkoxy-, C₁-C₄-alkylthio-, halo-C₁-C₄-alkylthio-,
-NR¹⁰R¹¹, -C(=O)OR¹², -C(=O)N¹⁰R¹¹, - C(=O)R¹², -S(=O)₂R¹², -S(=O)₂NR¹⁰R¹¹,
R² represents hydrogen, halogen, cyano, C₁-C₄-alkyl-, C₂-C₄-alkenyl-, C₂-C₄-alkynyl-, halo-C₁-C₄-alkyl-, C₁-C₄-alkoxy-, halo-C₁-C₄-alkoxy-, C₁-C₄-alkylthio- or halo-C₁-C₄-alkylthio-,
R³ represents halogen, C₁-C₃-alkyl-, C₁-C₃-alkoxy- or cyano,
R⁴ represents methyl- or ethyl-,
R⁵ represents hydrogen or C₁-C₃-alkyl-,
R⁶ represents hydrogen or C₁-C₃-alkyl,
or
R⁵ and R⁶ together with the carbon atom to which they are attached represent C₃-C₆-cycloalkyl,
R⁷ represents C₁-C₆-alkyl- which may optionally be monosubstituted by phenyl-, C₃-C₈-cycloalkyl-, or 4- to 8-membered heterocycloalkyl-,
in which phenyl- for its part may optionally be mono-, di- or trisubstituted by identical or different substituents from the group consisting of: halogen, cyano, C₁-C₄-alkyl-, C₂-C₄-alkenyl-, C₂-C₄-alkynyl-, C₁-C₄-alkoxy-, halo-C₁-C₄-alkyl-, halo-C₁-C₄-alkoxy-,
and
in which C₃-C₈-cycloalkyl- and 4- to 8-membered heterocycloalkyl- for their part may optionally be mono- or disubstituted by C₁-C₃-alkyl-,
or
represents C₃-C₈-cycloalkyl- or 4- to 8-membered heterocycloalkyl- which may optionally be mono- or disubstituted by C₁-C₃-alkyl-,
R⁸ represents C₁-C₆-alkyl- which may optionally be mono-, di- or trisubstituted by identical or different substituents from the group consisting of: hydroxy, oxo, fluorine, cyano, C₁-C₄-alkoxy-, halo-C₁-C₄-alkoxy-, -NR¹⁰R¹¹, C₃-C₈-cycloalkyl-, C₄-C₈-cycloalkenyl-, 4-to 8-membered heterocycloalkyl-, 4- to 8-membered heterocycloalkenyl-, C₅-C₁₁-spirocycloalkyl-, C₅-C₁₁-heterospirocycloalkyl-, bridged C₆-C₁₂-cycloalkyl-, bridged C₆-C₁₂-heterocycloalkyl-, C₆-C₁₂-bicycloalkyl-, C₆-C₁₂-heterobicycloalkyl-, phenyl- or 5-to 6-membered heteroaryl-, in which C₃-C₈-cycloalkyl-, C₄-C₈-cycloalkenyl-, 4- to 8-membered heterocycloalkyl-, 4- to 8-membered heterocycloalkenyl-, C₅-C₁₁-spirocycloalkyl-, C₅-C₁₁-heterospirocycloalkyl-, bridged C₆-C₁₂-cycloalkyl-, bridged C₆-C₁₂-heterocycloalkyl-, C₆-C₁₂-bicycloalkyl-, C₆-C₁₂-heterobicycloalkyl- may optionally be mono- or disubstituted by identical or different substituents from the group consisting of: hydroxy, fluorine, oxo, cyano, C₁-C₃-alkyl-, fluoro-C₁-C₃-alkyl-, C₃-C₆-cycloalkyl-, cyclopropylmethyl-, C₁-C₃-alkylcarbonyl-, C₁-C₄-alkoxycarbonyl- and -NR¹⁰R¹¹,
and
in which phenyl and 5- to 6-membered heteroaryl may optionally be mono- or disubstituted by identical or different substituents from the group consisting of: halogen, cyano, trifluoromethyl-, C₁-C₃-alkyl-, C₁-C₃-alkoxy-,
or represents C₃-C₆-alkenyl or C₃-C₆-alkynyl,
or represents fluoro-C₁-C₃-alkyl- which may optionally be monosubstituted by cyano or hydroxy,
or represents C₃-C₈-cycloalkyl-, C₄-C₈-cycloalkenyl-, C₅-C₁₁-spirocycloalkyl-, bridged C₆-C₁₂-cycloalkyl- or C₆-C₁₂-bicycloalkyl- which may optionally be mono- or disubstituted by identical or different substituents from the group consisting of: hydroxy, oxo, cyano, fluorine, C₁-C₃-alkyl, C₁-C₃-alkoxy, trifluoromethyl, -NR¹⁰R¹¹,
or represents 4- to 8-membered heterocycloalkyl-, 4- to 8-membered heterocycloalkenyl-, C₅-C₁₁-heterospirocycloalkyl-, bridged C₆-C₁₂-heterocycloalkyl- or C₆-C₁₂-heterobicycloalkyl- which may optionally be mono- or disubstituted by identical or different substituents from the group consisting of: hydroxy, fluorine, oxo, cyano, C₁-C₃-alkyl-, fluoro-C₁-C₃-alkyl-, C₃-C₆-cycloalkyl-, cyclopropylmethyl-, C₁-C₃-alkylcarbonyl-, C₁-C₄-alkoxycarbonyl- and -NR¹⁰R¹¹,
R⁹ represents hydrogen or represents C₁-C₃-alkyl- which is optionally mono- or disubstituted by identical or different substituents from the group consisting of hydroxy, oxo, C₁-C₃-alkoxy-, or represents fluoro-C₁-C₃-alkyl,
or
R⁸ and R⁹ together with the nitrogen atom to which they are attached represent 4- to 8-membered heterocycloalkyl, 4- to 8-membered heterocycloalkenyl-, C₅-C₁₁-heterospirocycloalkyl-, bridged C₆-C₁₂-heterocycloalkyl- or C₆-C₁₂-heterobicycloalkyl- which may optionally be mono- or disubstituted by identical or different substituents from the group consisting of: hydroxy, fluorine, oxo, cyano, C₁-C₃-alkyl-, fluoro-C₁-C₃-alkyl-, C₃-C₆-cycloalkyl-, cyclopropylmethyl-, C₁-C₃-alkylcarbonyl-, C₁-C₄-alkoxycarbonyl- and -NR¹⁰R¹¹,
R¹⁰ and R¹¹ independently of one another represent hydrogen or represent C₁-C₃-alkyl which is optionally mono- or disubstituted by identical or different substituents from the group consisting of hydroxy, oxo, C₁-C₃-alkoxy-, or represent fluoro-C₁-C₃-alkyl,
or
R¹⁰ and R¹¹ together with the nitrogen atom to which they are bonded represent 4- to 8-membered heterocycloalkyl- which may optionally be mono- or disubstituted by identical or different substituents from the group consisting of: hydroxy, fluorine, oxo, cyano, C₁-C₃-alkyl-, fluoro-C₁-C₃-alkyl-, C₃-C₆-cycloalkyl-, cyclopropylmethyl-, C₁-C₃-alkylcarbonyl-and C₁-C₄-alkoxycarbonyl-,
R¹² represents C₁-C₆-alkyl- or phenyl-C₁-C₃-alkyl-, and
n represents 0 or 1,
and diastereomers, racemates, polymorphs and physio-logically acceptable salts thereof.

2. Compounds of the general formula (I) according to Claim 1, in which
A represents -NH-,
R¹ represents a -C(=O)NR⁸R⁹ or -S(=O)₂NR⁸R⁹ group,
or
represents oxazolin-2-yl which may optionally be mono- or disubstituted by C₁-C₃-alkyl-,
or
represents oxazolyl-, thiazolyl-, oxadiazolyl- or thiadiazolyl- which may optionally be mono- or disubstituted by identical or different substituents from the group consisting of halogen, cyano, C₁-C₃-alkyl-, trifluoromethyl-, C₁-C₃-alkoxy-, trifluoromethoxy- and -NR¹⁰R¹¹,
R² represents hydrogen, fluorine, chlorine, cyano, methyl-, ethyl-, methoxy- or ethoxy-,
R³ represents fluorine, chlorine or methyl-,
R⁴ represents methyl-,
R⁵ represents hydrogen, methyl- or ethyl-,
R⁶ represents hydrogen, methyl- or ethyl-,
R⁷ represents C₃-C₅-alkyl-,
or
represents methyl- or ethyl- which may be monosubstituted by phenyl- or 4- to 8-membered heterocycloalkyl-,
in which phenyl- for its part may optionally be mono- or disubstituted by identical or different substituents from the group consisting of: fluorine, chlorine, bromine, cyano, C₁-C₃-alkyl-, C₁-C₃-alkoxy-, trifluoromethyl-,
and
in which 4- to 8-membered heterocycloalkyl- for its part may optionally be mono- or disubstituted by methyl-,
or
represents C₃-C₆-cycloalkyl- or 4- to 8-membered heterocycloalkyl- which may optionally be mono- or disubstituted by methyl-,
R⁸ represents C₁-C₆-alkyl- which may optionally be mono-, di- or trisubstituted by identical or different substituents from the group consisting of: hydroxy, oxo, fluorine, cyano, C₁-C₃-alkoxy-, fluoro-C₁-C₃-alkoxy-, -NR¹⁰R¹¹, 4- to 8-membered heterocycloalkyl-, phenyl- and 5- to 6-membered heteroaryl-,
in which the 4- to 8-membered heterocycloalkyl- may optionally be monosubstituted by: hydroxy, oxo, C₁-C₃-alkyl-, fluoro-C₁-C₃-alkyl-, cyclopropyl-, cyclopropylmethyl-, acetyl- or *tert-*butoxycarbonyl-,
and in which phenyl and 5- to 6-membered heteroaryl may optionally be mono- or disubstituted by identical or different substituents from the group consisting of: fluorine, chlorine, cyano, trifluoromethyl-, methyl-, methoxy-,
or represents fluoro-C₁-C₃-alkyl-,
or represents C₃-C₆-cycloalkyl- which may optionally be mono- or disubstituted by identical or different substituents from the group consisting of: hydroxy, oxo, cyano, fluorine, -NR¹⁰R¹¹,
or represents 4- to 8-membered heterocycloalkyl-, C₆-C₈-heterospirocycloalkyl-, bridged C₆-C₁₀-heterocycloalkyl- or C₆-C₁₀-heterobicycloalkyl- which may optionally be mono- or disubstituted by identical or different substituents from the group consisting of: hydroxy, oxo, C₁-C₃-alkyl-, fluoro-C₁-C₃-alkyl-, cyclopropyl-, cyclopropylmethyl-, acetyl- and *tert-*butoxycarbonyl-,
R⁹ represents hydrogen or C₁-C₃-alkyl,
or
R⁸ and R⁹ together with the nitrogen atom to which they are attached represent 4- to 8-membered heterocycloalkyl-, C₆-C₈-heterospirocycloalkyl-, bridged C₆-C₁₀-heterocycloalkyl- or C₆-C₁₀-heterobicycloalkyl-,
which may optionally be mono- or disubstituted by identical or different substituents from the group consisting of: hydroxy, oxo, C₁-C₃-alkyl-, fluoro-C₁-C₃-alkyl-, cyclopropyl-, cyclopropylmethyl-, acetyl- and *tert-*butoxycarbonyl-,
R¹⁰ and R¹¹ independently of one another represent hydrogen or represent C₁-C₃-alkyl which is optionally monosubstituted by hydroxy or oxo or represent trifluoromethyl-,
or
R¹⁰ and R¹¹ together with the nitrogen atom to which they are attached represent 4- to 7-membered heterocycloalkyl- which may optionally be mono- or disubstituted by identical or different substituents from the group consisting of: hydroxy, oxo, C₁-C₃-alkyl-, fluoro-C₁-C₃-alkyl-, cyclopropyl-, cyclopropylmethyl-, acetyl- and *tert*-butoxycarbonyl-, and
n represents 0 or 1
and diastereomers, racemates, polymorphs and physiologically acceptable salts thereof.

3. Compounds of the general formula (I) according to Claims 1 and 2 in which
A represents -NH-,
R¹ represents a -C(=O)NR⁸R⁹ or -S(=O)₂NR⁸R⁹ group,
or
represents oxazolin-2-yl which may optionally be mono- or disubstituted by C₁-C₃-alkyl-,
R² represents hydrogen, methyl-, ethyl- or methoxy-,
R⁴ represents methyl-,
R⁵ represents methyl- or ethyl-,
R⁶ represents hydrogen,
R⁷ represents C₃-C₅-alkyl-,
or
represents methyl- monosubstituted by phenyl- or 4- to 6-membered heterocycloalkyl-,
in which phenyl- for its part may optionally be mono- or disubstituted by identical or different substituents from the group consisting of: fluorine, chlorine, cyano, methyl-, methoxy-,
and
in which 4- to 6-membered heterocycloalkyl- for its part may optionally be monosubstituted by methyl-,
or
represents C₃-C₆-cycloalkyl- or represents 4- to 6-membered heterocycloalkyl-,
R⁸ represents C₁-C₄-alkyl- which may optionally be mono- or disubstituted by hydroxy, C₁-C₃-alkoxy-, -NR¹⁰R¹¹, 4- to 8-membered heterocycloalkyl, phenyl or 5-to 6-membered heteroaryl,
in which the 4- to 8-membered heterocycloalkyl- may optionally be monosubstituted by: oxo, C₁-C₃-alkyl, fluoro-C₁-C₃-alkyl-, cyclopropyl- or cyclopropylmethyl-,
and in which phenyl and 5- to 6-membered heteroaryl may optionally be mono- or disubstituted by identical or different substituents from the group consisting of: fluorine, chlorine, cyano, trifluoromethyl-, methyl- and methoxy-,
or represents fluoro-C₁-C₃-alkyl-,
or represents C₃-C₆-cycloalkyl- which may optionally be monosubstituted by hydroxy, fluorine or -NR¹⁰R¹¹,
or represents 4- to 8-membered heterocycloalkyl- which may optionally be mono- or disubstituted by identical or different substituents from the group consisting of: oxo, C₁-C₃-alkyl, fluoro-C₁-C₃-alkyl-, cyclopropyl- and cyclopropylmethyl-,
R⁹ represents hydrogen or methyl-,
or
R⁸ and R⁹ together with the nitrogen atom to which they are attached represent 5- to 6-membered heterocycloalkyl- which may optionally be mono- or disubstituted by identical or different substituents from the group consisting of: oxo, C₁-C₃-alkyl, fluoro-C₁-C₃-alkyl-, cyclopropyl-and cyclopropylmethyl-,
R¹⁰ and R¹¹ independently of one another represent hydrogen or represent C₁-C₃-alkyl-,
or
R¹⁰ and R¹¹ together with the nitrogen atom to which they are attached represent 4- to 7-membered heterocycloalkyl- which may optionally be mono- or disubstituted by identical or different substituents from the group consisting of: oxo, C₁-C₃-alkyl-, fluoro-C₁-C₃-alkyl-, cyclopropyl-and cyclopropylmethyl-, and
n represents 0,
and diastereomers, racemates, polymorphs and physiologically acceptable salts thereof.

4. Compounds of the general formula I according to any of Claims 1 to 3 in which
A represents -NH-,
R¹ represents a -C(=O)NR⁸R⁹ group,
or
represents oxazolin-2-yl which may optionally be mono- or disubstituted by C₁-C₃-alkyl-,
R² represents methyl-, ethyl- or methoxy-,
R⁴ represents methyl-,
R⁵ represents methyl- or ethyl-,
R⁶ represents hydrogen,
R⁷ represents C₃-C₅-alkyl-,
or
represents C₃-C₆-cycloalkyl,
R⁸ represents C₁-C₃-alkyl- which may optionally be monosubstituted by hydroxy, C₁-C₃-alkoxy-, phenyl- or 5- to 6-membered heteroaryl-,
in which phenyl and 5- to 6-membered heteroaryl may optionally be mono- or disubstituted by identical or different substituents from the group consisting of: fluorine, chlorine, methyl- and methoxy-,
or represents fluoro-C₁-C₃-alkyl-,
or represents C₃-C₆-cycloalkyl,
or represents 4- to 8-membered heterocycloalkyl- which may optionally be mono- or disubstituted by identical or different substituents from the group consisting of: oxo and C₁-C₃-alkyl-,
R⁹ represents hydrogen,
n represents 0,
and diastereomers, racemates, polymorphs and physiologically acceptable salts thereof.

5. Compounds of the general formula I according to any of Claims 1 to 4 in which
A represents -NH-,
R¹ represents a -C(=O)NR⁸R⁹ group,
or
represents oxazolin-2-yl which may optionally be mono- or disubstituted by methyl-,
R² represents methyl-, ethyl- or methoxy-,
R⁴ represents methyl-,
R⁵ represents methyl- or ethyl-,
R⁶ represents hydrogen,
R⁷ represents cyclopentyl-,
R⁸ represents C₁-C₄-alkyl- which may optionally by monosubstituted by hydroxy, methoxy- or pyridinyl-,
or represents fluoro-C₁-C₂-alkyl-,
or represents C₃-C₆-cycloalkyl,
or represents 4- to 8-membered heterocycloalkyl- which may optionally be mono- or disubstituted by identical or different substituents from the group consisting of: oxo and C₁-C₃-alkyl-,
R⁹ represents hydrogen,
n represents 0,
and diastereomers, racemates, polymorphs and physiologically acceptable salts thereof.

6. Compounds of the general formula I according to any of Claims 1 to 5 in which
A represents -NH-,
R¹ represents a -C(=O)NR⁸R⁹ group,
or
represents oxazolin-2-yl- which is disubstituted by methyl-,
R² represents methyl-, ethyl- or methoxy-,
R⁴ represents methyl-,
R⁵ represents methyl- or ethyl-,
R⁶ represents hydrogen,
R⁷ represents cyclopentyl-,
R⁸ represents C₁-C₄-alkyl- which may optionally by monosubstituted by hydroxy, methoxy- or pyridinyl-,
or represents 2,2,2-trifluoroethyl-, or represents cyclopropyl- or cyclohexyl-,
or represents piperidinyl, azepanyl or tetrahydropyranyl which may optionally be mono- or disubstituted by identical or different substituents from the group consisting of: oxo and methyl,
R⁹ represents hydrogen,
n represents 0,
and diastereomers, racemates, polymorphs and physiologically acceptable salts thereof.

7. Compounds of the general formula I according to any of Claims 1 to 6
6-{[1-cyclopentyl-2,4-dimethyl-3-oxo-1,2,3,4-tetrahydropyrido[3,4-b]pyrazin-7-yl]amino}-N-cyclopropyl-5-methoxypyridine-3-carboxamide,
6-{[(2*R*)-1-cyclopentyl-2,4-dimethyl-3-oxo-1,2,3,4-tetrahydropyrido[3,4-b]pyrazin-7-yl]amino}-N-cyclopropyl-5-methoxypyridine-3-carboxamide,
6-{[(2*R*)-1-cyclopentyl-2-ethyl-4-methyl-3-oxo-1,2,3,4-tetrahydropyrido[3,4-b]pyrazin-7-yl]amino}-N-cyclopropyl-5-ethylpyridine-3-carboxamide,
6-[(1-cyclopentyl-2-ethyl-1,2,3,4-tetrahydro-4-methyl-3-oxopyrido[3,4-b]pyrazin-7-yl)amino]-N-cyclopropyl-5-methoxy-3-pyridinecarboxamide,
6-[[(2*R*)-1-cyclopentyl-2-ethyl-1,2,3,4-tetrahydro-4-methyl-3-oxopyrido[3,4-b]pyrazin-7-yl)amino]-N-cyclopropyl-5-methoxy-3-pyridinecarboxamide,
6-[(1-cyclopentyl-2,4-dimethyl-3-oxo-1,2,3,4-tetrahydropyrido[3,4-b]pyrazin-7-yl)amino]-5-methoxy-N-(1-methylpiperidin-4-yl)pyridine-3-carboxamide,
6-[[(2*R*)-1-cyclopentyl-2-ethyl-1,2,3,4-tetrahydro-4-methyl-3-oxopyrido[3,4-b]pyrazin-7-yl]amino]-5-methoxy-N-(2,2,2-trifluoroethyl)-3-pyridinecarboxamide,
6-[[(2*R*)-1-cyclopentyl-2-ethyl-1,2,3,4-tetrahydro-4-methyl-3-oxopyrido[3,4-b]pyrazin-7-yl]amino]-5-methoxy-N-(2-methoxyethyl)-3-pyridinecarboxamide,
6-{[(2*R*)-1-cyclopentyl-2-ethyl-4-methyl-3-oxo-1,2,3,4-tetrahydropyrido[3,4-b]pyrazin-7-yl]amino}-5-ethyl-N-[(3R)-2-oxoazepan-3-yl]pyridine-3-carboxamide,
6-[[(2*R*)-1-cyclopentyl-2-ethyl-1,2,3,4-tetrahydro-4-methyl-3-oxopyrido[3,4-b]pyrazin-7-yl]amino]-5-methoxy-N-(tetrahydro-2H-pyran-4-yl)-3-pyridinecarboxamide,
6-[[(2*R*)-1-cyclopentyl-2-ethyl-1,2,3,4-tetrahydro-4-methyl-3-oxopyrido[3,4-b]pyrazin-7-yl]amino]-N-(2-hydroxy-1,1-dimethylethyl)-5-methoxy-3-pyridinecarboxamide,
6-[[(2*R*)-1-cyclopentyl-2-ethyl-1,2,3,4-tetrahydro-4-methyl-3-oxopyrido[3,4-b]pyrazin-7-yl]amino]-5-methoxy-*N*-(3-pyridinylmethyl)-3-pyridinecarboxamide,
6-{[(2*R*)-1-cyclopentyl-2-ethyl-4-methyl-3-oxo-1,2,3,4-tetrahydropyrido[3,4-b]pyrazin-7-yl]amino}-*N*-cyclopropyl-5-methylpyridine-3-carboxamide,
6-[[(2*R*)-1-cyclopentyl-2-ethyl-1,2,3,4-tetrahydro-4-methyl-3-oxopyrido[3,4-b]pyrazin-7-yl]amino]-5-methoxy-N-(1-methyl-4-piperidinyl)-3-pyridinecarboxamide,
6-{[(2*R*)-1-cyclopentyl-2-ethyl-4-methyl-3-oxo-1,2,3,4-tetrahydropyrido[3,4-b]pyrazin-7-yl]amino}-5-methylpyridine-3-carboxamide,
6-[[(2*R*)-1-cyclopentyl-2-ethyl-1,2,3,4-tetrahydro-4-methyl-3-oxopyrido[3,4-b]pyrazin-7-yl]amino]-N-[(3*R*)-hexahydro-2-oxo-1H-azepin-3-yl]-5-methoxy-3-pyridinecarboxamide,
6-{[(2*R*)-1-cyclopentyl-2-ethyl-4-methyl-3-oxo-1,2,3,4-tetrahydropyrido[3,4-b]pyrazin-7-yl]amino}-5-methyl-*N*-(tetrahydro-2H-pyran-4-yl)pyridine-3-carboxamide,
6-[[(2*R*)-1-cyclopentyl-2-ethyl-1,2,3,4-tetrahydro-4-methyl-3-oxopyrido[3,4-b]pyrazin-7-yl]amino]-5-methyl-*N*-(1-methyl-4-piperidinyl)pyridine-3-carboxamide,
1*N*-cyclopentyl-7-[[5-(4,5-dihydro-4,4-dimethyl-2-oxazolyl)-3-methoxy-2-pyridinyl]amino]-(2*R*)-ethyl-4*N*-methyl-1,4-dihydropyrido[3,4-b]pyrazin-3(2H)-one,
*N*-cyclohexyl-6-[[(2*R*)-1-cyclopentyl-2-ethyl-1,2,3,4-tetrahydro-4-methyl-3-oxopyrido[3,4-b]pyrazin-7-yl]amino]-5-methoxypyridine-3-carboxamide.

8. Compounds according to any of Claims 1 to 7 for use as medicaments.

9. Compounds according to any of Claims 1 to 7 for use in the prophylaxis and/or therapy of tumour diseases.

10. Use of the compounds according to any of Claims 1 to 7 for preparing a medicament.

11. Use of the compounds according to any of Claims 1 to 7 for preparing a medicament for the prophylaxis and/or therapy of tumour diseases.

12. Compounds according to any of Claims 1 to 7 for use in the prophylaxis and/or therapy of hyperproliferative disorders.

13. Compounds according to any of Claims 1 to 7 for use in the prophylaxis and/or therapy of viral infections, neurodegenerative disorders, inflammatory disorders, atherosclerotic disorders and in male fertility control.

14. Use of the compounds according to any of Claims 1 to 7 for preparing a medicament for the prophylaxis and/or therapy of viral infections, neurodegenerative disorders, inflammatory disorders, atherosclerotic disorders and in male fertility control.

15. Compounds according to any of Claims 1 to 7 in combination with one or more further pharmacologically active substances.

16. Compounds according to any of Claims 1 to 7 in combination with one or more further pharmacologically active substances, for use in the prophylaxis and/or treatment of hyperproliferative disorders.

17. Compounds according to any of Claims 1 to 7 in combination with one or more further pharmacologically active substances, for use in the prophylaxis and/or treatment of tumour diseases.

18. Compounds according to any of Claims 1 to 7 in combination with one or more further pharmacologically active substances, for use in the prophylaxis and/or treatment of viral infections, neurodegenerative disorders, inflammatory disorders, atherosclerotic disorders and in male fertility control.

19. Compounds of the general formula (VIII) 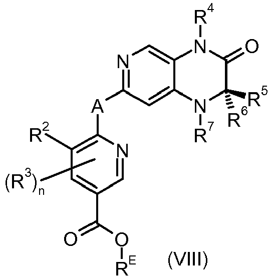 in which A, R², R³, R⁴, R⁵, R⁶, R⁷and n have the meanings given in the general formula (I) and R^{E} represents C₁-C₆-alkyl.

20. Compounds of the general formula (IX) 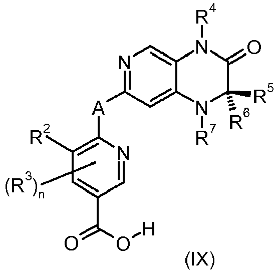 in which A, R², R³, R⁴, R⁵, R⁶, R⁷ and n have the meanings given in the general formula (I).

21. Use of the compounds of the general formula (VIII) 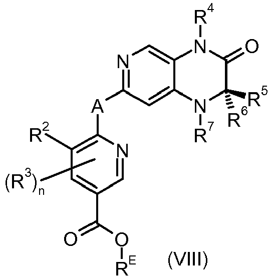 in which A, R², R³, R⁴, R⁵, R⁶, R⁷and n have the meanings given in the general formula (I) and R^{E} represents C₁-C₆-alkyl, for preparation of the compounds of the general formula (I) according to the invention.

22. Use of the compounds of the general formula (IX) 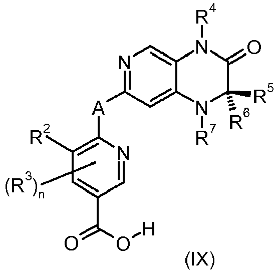 in which A, R², R³, R⁴, R⁵, R⁶, R⁷ and n have the meanings given in the general formula (I), for preparation of the compounds of the general formula (I) according to the invention.

## Revendications

1. Composés de formule générale (I) 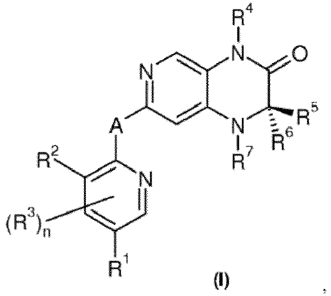 dans laquelle
A représente -NH- ou -O-,
R¹ représente un groupe -C(=O)NR⁸R⁹ ou - S(=O)₂NR⁸R⁹,
ou
représente oxazolin-2-yle, qui peut éventuellement être substitué une ou deux fois avec alkyle en C₁-C₃,
ou
représente hétéroaryle monocyclique à 5 éléments, qui peut éventuellement être substitué une, deux ou trois fois, de manière identique ou différente, avec halogène, cyano, alkyle en C₁-C₄, alcényle en C₂-C₄, alcynyle en C₂-C₄, halogénoalkyle en C₁-C₄, alcoxy en C₁-C₄, halogénoalcoxy en C₁-C₄, alkylthio en C₁-C₄, halogénoalkylthio en C₁-C₄, -NR¹⁰R¹¹, -C(=O)OR¹², -C(=O)N¹⁰R¹¹, - C(=O)R¹², -S(=O)₂R¹², -S(=O)₂NR¹⁰R¹¹,
R² représente hydrogène, halogène, cyano, alkyle en C₁-C₄, alcényle en C₂-C₄, alcynyle en C₂-C₄, halogénoalkyle en C₁-C₄, alcoxy en C₁-C₄, halogénoalcoxy en C₁-C₄, alkylthio en C₁-C₄ ou halogénoalkylthio en C₁-C₄,
R³ représente halogène, alkyle en C₁-C₃, alcoxy en C₁-C₃ ou cyano,
R⁴ représente méthyle ou éthyle,
R⁵ représente hydrogène ou alkyle en C₁-C₃,
R⁶ représente hydrogène ou alkyle en C₁-C₃, ou
R⁵ et R⁶ représentent ensemble avec l'atome de carbone auquel ils sont reliés cycloalkyle en C₃-C₆,
R⁷ représente alkyle en C₁-C₆, qui peut éventuellement être substitué une fois avec phényle, cycloalkyle en C₃-C₈ ou hétérocycloalkyle de 4 à 8 éléments, le phényle pouvant de son côté éventuellement être substitué une, deux ou trois fois, de manière identique ou différente, avec : halogène, cyano, alkyle en C₁-C₄, alcényle en C₂-C₄, alcynyle en C₂-C₄, alcoxy en C₁-C₄, halogénoalkyle en C₁-C₄, halogénoalcoxy en C₁-C₄,
et
le cycloalkyle en C₃-C₈ et l'hétérocycloalkyle de 4 à 8 éléments pouvant de leur côté éventuellement être substitués une ou deux fois avec alkyle en C₁-C₃, ou
représente cycloalkyle en C₃-C₈ ou hétérocycloalkyle de 4 à 8 éléments, qui peuvent éventuellement être substitués une ou deux fois avec alkyle en C₁-C₃,
R⁸ représente alkyle en C₁-C₆, qui peut éventuellement être substitué une, deux ou trois fois, de manière identique ou différente, avec : hydroxy, oxo, fluor, cyano, alcoxy en C₁-C₄, halogénoalcoxy en C₁-C₄, -NR¹⁰R¹¹, cycloalkyle en C₃-C₈, cycloalcényle en C₄-C₈, hétérocycloalkyle de 4 à 8 éléments, hétérocycloalcényle de 4 à 8 éléments, spirocycloalkyle en C₅-C₁₁, hétérospirocycloalkyle en C₅-C₁₁, cycloalkyle en C₆-C₁₂ ponté, hétérocycloalkyle en C₆-C₁₂ ponté, bicycloalkyle en C₆-C₁₂, hétérobicycloalkyle en C₆-C₁₂, phényle ou hétéroaryle de 5 à 6 éléments,
le cycloalkyle en C₃-C₈, le cycloalcényle en C₄-C₈, l'hétérocycloalkyle de 4 à 8 éléments, l'hétérocycloalcényle de 4 à 8 éléments, le spirocycloalkyle en C₅-C₁₁, l'hétérospirocycloalkyle en C₅-C₁₁, le cycloalkyle en C₆-C₁₂ ponté, l'hétérocycloalkyle en C₆-C₁₂ ponté, le bicycloalkyle en C₆-C₁₂, l'hétérobicycloalkyle en C₆-C₁₂ pouvant éventuellement être substitués une ou deux fois, de manière identique ou différente, avec : hydroxy, fluor, oxo, cyano, alkyle en C₁-C₃, fluoroalkyle en C₁-C₃, cycloalkyle en C₃-C₆, cyclopropylméthyle, alkylcarbonyle en C₁-C₃, alcoxycarbonyle en C₁-C₄ ou -NR¹⁰R¹¹,
et
le phényle et l'hétéroaryle de 5 à 6 éléments pouvant éventuellement être substitués une ou deux fois, de manière identique ou différente, avec : halogène, cyano, trifluorométhyle, alkyle en C₁-C₃, alcoxy en C₁-C₃, ou représente alcényle en C₃-C₆ ou alcynyle en C₃-C₆,
ou représente fluoroalkyle en C₁-C₃, qui peut éventuellement être substitué une fois par cyano ou hydroxy,
ou représente cycloalkyle en C₃-C₈, cycloalcényle en C₄-C₈, spirocycloalkyle en C₅-C₁₁, cycloalkyle en C₆-C₁₂ ponté ou bicycloalkyle en C₆-C₁₂, qui peuvent éventuellement être substitués une ou deux fois, de manière identique ou différente, avec : hydroxy, oxo, cyano, fluor, alkyle en C₁-C₃, alcoxy en C₁-C₃, trifluorométhyle, -NR¹⁰R¹¹,
ou représente hétérocycloalkyle de 4 à 8 éléments, hétérocycloalcényle de 4 à 8 éléments, hétérospirocycloalkyle en C₅-C₁₁, hétérocycloalkyle en C₆-C₁₂ ponté ou hétérobicycloalkyle en C₆-C₁₂, qui peuvent éventuellement être substitués une ou deux fois, de manière identique ou différente, avec : hydroxy, fluor, oxo, cyano, alkyle en C₁-C₃, fluoroalkyle en C₁-C₃, cycloalkyle en C₃-C₆, cyclopropylméthyle, alkylcarbonyle en C₁-C₃, alcoxycarbonyle en C₁-C₄ ou -NR¹⁰R¹¹,
R⁹ représente hydrogène ou alkyle en C₁-C₃ éventuellement substitué une ou deux fois, de manière identique ou différente, avec hydroxy, oxo, alcoxy en C₁-C₃, ou représente fluoroalkyle en C₁-C₃, ou
R⁸ et R⁹ représentent ensemble avec l'atome d'azote auquel ils sont reliés hétérocycloalkyle de 4 à 8 éléments, hétérocycloalcényle de 4 à 8 éléments, hétérospirocycloalkyle en C₅-C₁₁, hétérocycloalkyle en C₆-C₁₂ ponté ou hétérobicycloalkyle en C₆-C₁₂, qui peuvent éventuellement être substitués une ou deux fois, de manière identique ou différente, avec : hydroxy, fluor, oxo, cyano, alkyle en C₁-C₃, fluoroalkyle en C₁-C₃, cycloalkyle en C₃-C₆, cyclopropylméthyle, alkylcarbonyle en C₁-C₃, alcoxycarbonyle en C₁-C₄ ou -NR¹⁰R¹¹,
R¹⁰ et R¹¹ représentent indépendamment l'un de l'autre hydrogène ou alkyle en C₁-C₃ éventuellement substitué une ou deux fois, de manière identique ou différente, avec hydroxy, oxo, alcoxy en C₁-C₃, ou représentent fluoroalkyle en C₁-C₃, ou
R¹⁰ et R¹¹ représentent ensemble avec l'atome d'azote auquel ils sont reliés hétérocycloalkyle de 4 à 8 éléments, qui peut éventuellement être substitué une ou deux fois, de manière identique ou différente, avec : hydroxy, fluor, oxo, cyano, alkyle en C₁-C₃, fluoroalkyle en C₁-C₃, cycloalkyle en C₃-C₆, cyclopropylméthyle, alkylcarbonyle en C₁-C₃ ou alcoxycarbonyle en C₁-C₄,
R¹² représente alkyle en C₁-C₆ ou phényl-alkyle en C₁-C₃, et
n représente 0 ou 1, ainsi que leurs diastéréomères, racémats, polymorphes et sels physiologiquement acceptables.

2. Composés de formule générale (I) selon la revendication 1, dans laquelle
A représente -NH-,
R¹ représente un groupe -C(=O)NR⁸R⁹ ou - S(=O)₂NR⁸R⁹,
ou
représente oxazolin-2-yle, qui peut éventuellement être substitué une ou deux fois avec alkyle en C₁-C₃,
ou
représente oxazolyle, thiazolyle, oxadiazolyle ou thiadiazolyle, qui peuvent éventuellement être substitués une ou deux fois, de manière identique ou différente, avec halogène, cyano, alkyle en C₁-C₃, trifluorométhyle, alcoxy en C₁-C₃, trifluorométhoxy ou -NR¹⁰R¹¹,
R² représente hydrogène, fluor, chlore, cyano, méthyle, éthyle, méthoxy ou éthoxy,
R³ représente fluor, chlore ou méthyle,
R⁴ représente méthyle,
R⁵ représente hydrogène, méthyle ou éthyle,
R⁶ représente hydrogène, méthyle ou éthyle,
R⁷ représente alkyle en C₃-C₅,
ou
représente méthyle ou éthyle, qui peuvent être substitués une fois avec phényle ou hétérocycloalkyle de 4 à 8 éléments,
le phényle pouvant de son côté éventuellement être substitué une ou deux fois, de manière identique ou différente, avec : fluor, chlore, brome, cyano, alkyle en C₁-C₃, alcoxy en C₁-C₃, trifluorométhyle,
et
l'hétérocycloalkyle de 4 à 8 éléments pouvant de son côté éventuellement être substitué une ou deux fois avec méthyle, ou représente cycloalkyle en C₃-C₆ ou hétérocycloalkyle de 4 à 8 éléments, qui peuvent éventuellement être substitués une ou deux fois avec méthyle,
R⁸ représente alkyle en C₁-C₆, qui peut éventuellement être substitué une, deux ou trois fois, de manière identique ou différente, avec : hydroxy, oxo, fluor, cyano, alcoxy en C₁-C₃, fluoroalcoxy en C₁-C₃, -NR¹⁰R¹¹, hétérocycloalkyle de 4 à 8 éléments, phényle ou hétéroaryle de 5 à 6 éléments,
l'hétérocycloalkyle de 4 à 8 éléments pouvant éventuellement être substitué une fois avec : hydroxy, oxo, alkyle en C₁-C₃, fluoroalkyle en C₁-C₃, cyclopropyle, cyclopropylméthyle, acétyle ou tert-butoxycarbonyle,
et
le phényle et l'hétéroaryle de 5 à 6 éléments pouvant éventuellement être substitués une ou deux fois, de manière identique ou différente, avec : fluor, chlore, cyano, trifluorométhyle, méthyle, méthoxy,
ou représente fluoroalkyle en C₁-C₃, ou représente cycloalkyle en C₃-C₆ qui peut éventuellement être substitué une ou deux fois, de manière identique ou différente, avec : hydroxy, oxo, cyano, fluor, -NR¹⁰R¹¹,
ou représente hétérocycloalkyle de 4 à 8 éléments, hétérospirocycloalkyle en C₆-C₈, hétérocycloalkyle en C₆-C₁₀ ponté ou hétérobicycloalkyle en C₆-C₁₀, qui peuvent éventuellement être substitués une ou deux fois, de manière identique ou différente, avec : hydroxy, oxo, alkyle en C₁-C₃, fluoroalkyle en C₁-C₃, cyclopropyle, cyclopropylméthyle, acétyl- ou tert-butoxycarbonyle,
R⁹ représente hydrogène ou alkyle en C₁-C₃,
ou
R⁸ et R⁹ représentent ensemble avec l'atome d'azote auquel ils sont reliés hétérocycloalkyle de 4 à 8 éléments, hétérospirocycloalkyle en C₆-C₈, hétérocycloalkyle en C₆-C₁₀ ponté ou hétérobicycloalkyle en C₆-C₁₀, qui peuvent éventuellement être substitués une ou deux fois, de manière identique ou différente, avec : hydroxy, oxo, alkyle en C₁-C₃, fluoroalkyle en C₁-C₃, cyclopropyle, cyclopropylméthyle, acétyl- ou tert-butoxycarbonyle,
R¹⁰ et R¹¹ représentent indépendamment l'un de l'autre hydrogène ou alkyle en C₁-C₃ éventuellement substitué une fois avec hydroxy ou oxo, ou représentent trifluorométhyle,
ou
R¹⁰ et R¹¹ représentent ensemble avec l'atome d'azote auquel ils sont reliés hétérocycloalkyle de 4 à 7 éléments, qui peut éventuellement être substitué une ou deux fois, de manière identique ou différente, avec : hydroxy, oxo, alkyle en C₁-C₃, fluoroalkyle en C₁-C₃, cyclopropyle, cyclopropylméthyle, acétyl- ou tert-butoxycarbonyle, et
n représente 0 ou 1,
ainsi que leurs diastéréomères, racémats, polymorphes et sels physiologiquement acceptables.

3. Composés de formule générale (I) selon les revendications 1 et 2, dans laquelle
A représente -NH-,
R¹ représente un groupe -C(=O)NR⁸R⁹ ou - S(=O)₂NR⁸R⁹,
ou
représente oxazolin-2-yle, qui peut éventuellement être substitué une ou deux fois avec alkyle en C₁-C₃,
R² représente hydrogène, méthyle, éthyle ou méthoxy,
R⁴ représente méthyle,
R⁵ représente méthyle ou éthyle,
R⁶ représente hydrogène,
R⁷ représente alkyle en C₃-C₅,
ou
représente méthyle, qui peut être substitué une fois avec phényle ou hétérocycloalkyle de 4 à 6 éléments, le phényle pouvant de son côté éventuellement être substitué une ou deux fois, de manière identique ou différente, avec : fluor, chlore, cyano, méthyle, méthoxy,
et
l'hétérocycloalkyle de 4 à 6 éléments pouvant de son côté éventuellement être substitué une fois avec méthyle,
ou
représente cycloalkyle en C₃-C₆ ou hétérocycloalkyle de 4 à 6 éléments,
R⁸ représente alkyle en C₁-C₄, qui peut éventuellement être substitué une ou deux fois avec hydroxy, alcoxy en C₁-C₃, -NR¹⁰R¹¹, hétérocycloalkyle de 4 à 8 éléments, phényle ou hétéroaryle de 5 à 6 éléments,
l'hétérocycloalkyle de 4 à 8 éléments pouvant éventuellement être substitué une fois avec : oxo, alkyle en C₁-C₃, fluoroalkyle en C₁-C₃, cyclopropyle ou cyclopropylméthyle, et le phényle et l'hétéroaryle de 5 à 6 éléments pouvant éventuellement être substitués une ou deux fois, de manière identique ou différente, avec : fluor, chlore, cyano, trifluorométhyle, méthyle ou méthoxy,
ou représente fluoroalkyle en C₁-C₃, ou représente cycloalkyle en C₃-C₆ qui peut éventuellement être substitué une fois avec hydroxy, fluor ou -NR¹⁰R¹¹, ou représente hétérocycloalkyle de 4 à 8 éléments, qui peut éventuellement être substitué une ou deux fois, de manière identique ou différente, avec : oxo, alkyle en C₁-C₃, fluoroalkyle en C₁-C₃, cyclopropyle ou cyclopropylméthyle,
R⁹ représente hydrogène ou méthyle,
ou
R⁸ et R⁹ représentent ensemble avec l'atome d'azote auquel ils sont reliés hétérocycloalkyle de 5 à 6 éléments, qui peut éventuellement être substitué une ou deux fois, de manière identique ou différente, avec : oxo, alkyle en C₁-C₃, fluoroalkyle en C₁-C₃, cyclopropyle ou cyclopropylméthyle,
R¹⁰ et R¹¹ représentent indépendamment l'un de l'autre hydrogène ou alkyle en C₁-C₃, ou
R¹⁰ et R¹¹ représentent ensemble avec l'atome d'azote auquel ils sont reliés hétérocycloalkyle de 4 à 7 éléments, qui peut éventuellement être substitué une ou deux fois, de manière identique ou différente, avec : oxo, alkyle en C₁-C₃, fluoroalkyle en C₁-C₃, cyclopropyle ou cyclopropylméthyle, et
n représente 0,
ainsi que leurs diastéréomères, racémats, polymorphes et sels physiologiquement acceptables.

4. Composés de formule générale (I) selon les revendications 1 à 3, dans laquelle
A représente -NH-,
R¹ représente un groupe -C(=O)NR⁸R⁹,
ou
représente oxazolin-2-yle, qui peut éventuellement être substitué une ou deux fois avec alkyle en C₁-C₃,
R² représente méthyle, éthyle ou méthoxy,
R⁴ représente méthyle,
R⁵ représente méthyle ou éthyle,
R⁶ représente hydrogène,
R⁷ représente alkyle en C₃-C₅,
ou
représente cycloalkyle en C₃-C₆,
R⁸ représente alkyle en C₁-C₃, qui peut éventuellement être substitué une fois avec hydroxy, alcoxy en C₁-C₃, -phényle ou hétéroaryle de 5 à 6 éléments, le phényle et l'hétéroaryle de 5 à 6 éléments pouvant éventuellement être substitués une ou deux fois, de manière identique ou différente, avec : fluor, chlore, méthyle ou méthoxy, ou représente fluoroalkyle en C₁-C₃, ou représente cycloalkyle en C₃-C₆,
ou représente hétérocycloalkyle de 4 à 8 éléments, qui peut éventuellement être substitué une ou deux fois, de manière identique ou différente, avec : oxo ou alkyle en C₁-C₃,
R⁹ représente hydrogène,
n représente 0,
ainsi que leurs diastéréomères, racémats, polymorphes et sels physiologiquement acceptables.

5. Composés de formule générale (I) selon les revendications 1 à 4, dans laquelle
A représente -NH-,
R¹ représente un groupe -C(=O)NR⁸R⁹,
ou
représente oxazolin-2-yle, qui peut éventuellement être substitué une ou deux fois avec méthyle,
R² représente méthyle, éthyle ou méthoxy,
R⁴ représente méthyle,
R⁵ représente méthyle ou éthyle,
R⁶ représente hydrogène,
R⁷ représente cyclopentyle,
R⁸ représente alkyle en C₁-C₄, qui peut éventuellement être substitué une fois avec hydroxy, méthoxy ou pyridinyle, ou représente fluoroalkyle en C₁-C₂, ou représente cycloalkyle en C₃-C₆, ou représente hétérocycloalkyle de 4 à 8 éléments, qui peut éventuellement être substitué une ou deux fois, de manière identique ou différente, avec : oxo ou alkyle en C₁-C₃,
R⁹ représente hydrogène,
n représente 0,
ainsi que leurs diastéréomères, racémats, polymorphes et sels physiologiquement acceptables.

6. Composés de formule générale (I) selon les revendications 1 à 5, dans laquelle
A représente -NH-,
R¹ représente un groupe -C(=O)NR⁸R⁹,
ou
représente oxazolin-2-yle, qui est substitué deux fois avec méthyle,
R² représente méthyle, éthyle ou méthoxy,
R⁴ représente méthyle,
R⁵ représente méthyle ou éthyle,
R⁶ représente hydrogène,
R⁷ représente cyclopentyle,
R⁸ représente alkyle en C₁-C₄, qui peut éventuellement être substitué une fois avec hydroxy, méthoxy ou pyridinyle,
ou représente 2,2,2-trifluoroéthyle,
ou représente cyclopropyle ou cyclohexyle,
ou représente pipéridinyle, azépanyle ou tétrahydropyranyle, qui peuvent éventuellement être substitués une ou deux fois, de manière identique ou différente, avec : oxo ou méthyle,
R⁹ représente hydrogène,
n représente 0,
ainsi que leurs diastéréomères, racémats, polymorphes et sels physiologiquement acceptables.

7. Composés de formule générale I selon les revendications 1 à 6
6-{[1-cyclopentyl-2,4-diméthyl-3-oxo-1,2,3,4-tétrahydropyrido[3,4-b]pyrazin-7-yl]amino}-N-cyclopropyl-5-méthoxypyridine-3-carboxamide,
6-{[(2R)-1-cyclopentyl-2,4-diméthyl-3-oxo-1,2,3,4-tétrahydropyrido[3,4-b]pyrazin-7-yl]amino}-N-cyclopropyl-5-méthoxypyridine-3-carboxamide,
6-{[(2R)-1-cyclopentyl-2-éthyl-4-méthyl-3-oxo-1,2,3,4-tétrahydropyrido[3,4-b]pyrazin-7-yl]amino}-N-cyclopropyl-5-éthylpyridine-3-carboxamide,
6-[(1-cyclopentyl-2-éthyl-1,2,3,4-tétrahydro-4-méthyl-3-oxopyrido[3,4-b]pyrazin-7-yl)amino]-N-cyclopropyl-5-méthoxy-3-pyridine-carboxamide,
6-[[(2R)-1-cyclopentyl-2-éthyl-1,2,3,4-tétrahydro-4-méthyl-3-oxopyrido[3,4-b]pyrazin-7-yl)amino]-N-cyclopropyl-5-méthoxy-3-pyridine-carboxamide,
6-[(1-cyclopentyl-2,4-diméthyl-3-oxo-1,2,3,4-tétrahydropyrido[3,4-b]pyrazin-7-yl)amino]-5-méthoxy-N-(1-méthylpipéridin-4-yl)pyridin-3-carboxamide,
6-[[(2R)-1-cyclopentyl-2-éthyl-1,2,3,4-tétrahydro-4-méthyl-3-oxopyrido[3,4-b]pyrazin-7-yl]amino]-5-méthoxy-N-(2,2,2-trifluoroéthyl)-3-pyridine-carboxamide,
6-[[(2R)-1-cyclopentyl-2-éthyl-1,2,3,4-tétrahydro-4-méthyl-3-oxopyrido[3,4-b]pyrazin-7-yl]amino]-5-méthoxy-N-(2-méthoxyéthyl)-3-pyridine-carboxamide,
6-{[(2R)-1-cyclopentyl-2-éthyl-4-méthyl-3-oxo-1,2,3,4-tétrahydropyrido[3,4-b]pyrazin-7-yl]amino}-5-éthyl-N-[(3R)-2-oxoazépan-3-yl]pyridine-3-carboxamide,
6-[[(2R)-1-cyclopentyl-2-éthyl-1,2,3,4-tétrahydro-4-méthyl-3-oxopyrido[3,4-b]pyrazin-7-yl]amino]-5-méthoxy-N-(tétrahydro-2H-pyran-4-yl)-3-pyridine-carboxamide,
6-[[(2R)-1-cyclopentyl-2-éthyl-1,2,3,4-tétrahydro-4-méthyl-3-oxopyrido[3,4-b]pyrazin-7-yl]amino]-N-(2-hydroxy-1,1-diméthyléthyl)-5-méthoxy-3-pyridine-carboxamide,
6-[[(2R)-1-cyclopentyl-2-éthyl-1,2,3,4-tétrahydro-4-méthyl-3-oxopyrido[3,4-b]pyrazin-7-yl]amino]-5-méthoxy-N-(3-pyridinylméthyl)-3-pyridine-carboxamide,
6-{[(2R)-1-cyclopentyl-2-éthyl-4-méthyl-3-oxo-1,2,3,4-tétrahydropyrido[3,4-b]pyrazin-7-yl]amino}-N-cyclopropyl-5-méthylpyridine-3-carboxamide,
6-[[(2R)-1-cyclopentyl-2-éthyl-1,2,3,4-tétrahydro-4-méthyl-3-oxopyrido[3,4-b]pyrazin-7-yl]amino]-5-méthoxy-N-(1-méthyl-4-pipéridinyl)-3-pyridine-carboxamide,
6-{[(2R)-1-cyclopentyl-2-éthyl-4-méthyl-3-oxo-1,2,3,4-tétrahydropyrido[3,4-b]pyrazin-7-yl]amino}-5-méthylpyridine-3-carboxamide,
6-[[(2R)-1-cyclopentyl-2-éthyl-1,2,3,4-tétrahydro-4-méthyl-3-oxopyrido[3,4-b]pyrazin-7-yl]amino]-N-[(3R)-hexahydro-2-oxo-1H-azépin-3-yl]-5-méthoxy-3-pyridine-carboxamide,
6-{[(2R)-1-cyclopentyl-2-éthyl-4-méthyl-3-oxo-1,2,3,4-tétrahydropyrido[3,4-b]pyrazin-7-yl]amino}-5-méthyl-N-(tétrahydro-2H-pyran-4-yl)pyridine-3-carboxamide,
6-[[(2R)-1-cyclopentyl-2-éthyl-1,2,3,4-tétrahydro-4-méthyl-3-oxopyrido[3,4-b]pyrazin-7-yl]amino]-5-méthyl-N-(1-méthyl-4-pipéridinyl)pyridine-3-carboxamide,
1N-cyclopentyl-7-[[5-(4,5-dihydro-4,4-diméthyl-2-oxazolyl)-3-méthoxy-2-pyridinyl]amino]-(2R)-éthyl-4N-méthyl-1,4-dihydro-pyrido[3,4-b]pyrazin-3(2H)-one,
N-cyclohexyl-6-[[(2R)-1-cyclopentyl-2-éthyl-1,2,3,4-tétrahydro-4-méthyl-3-oxopyrido[3,4-b]pyrazin-7-yl]amino]-5-méthoxy-pyridine-3-carboxamide.

8. Composés selon les revendications 1 à 7, destinés à une utilisation en tant que médicament.

9. Composés selon les revendications 1 à 7, destinés à une utilisation dans la prophylaxie et/ou le traitement de maladies tumorales.

10. Utilisation des composés selon les revendications 1 à 7 pour la fabrication d'un médicament.

11. Utilisation des composés selon les revendications 1 à 7 pour la fabrication d'un médicament pour la prophylaxie et/ou le traitement de maladies tumorales.

12. Composés selon les revendications 1 à 7, destinés à une utilisation dans la prophylaxie et/ou le traitement de maladies hyperprolifératives.

13. Composés selon les revendications 1 à 7, destinés à une utilisation dans la prophylaxie et/ou le traitement d'infections virales, de maladies neurodégénératives, de maladies inflammatoires, de maladies athérosclérotiques et dans le contrôle de la fertilité masculine.

14. Utilisation des composés selon les revendications 1 à 7 pour la fabrication d'un médicament pour la prophylaxie et/ou le traitement d'infections virales, de maladies neurodégénératives, de maladies inflammatoires, de maladies athérosclérotiques et dans le contrôle de la fertilité masculine.

15. Composés selon les revendications 1 à 7, en combinaison avec une ou plusieurs autres substances pharmacologiquement actives.

16. Composés selon les revendications 1 à 7, en combinaison avec une ou plusieurs autres substances pharmacologiquement actives, destinés à une utilisation dans la prophylaxie et/ou le traitement de maladies hyperprolifératives.

17. Composés selon les revendications 1 à 7, en combinaison avec une ou plusieurs autres substances pharmacologiquement actives, destinés à une utilisation dans la prophylaxie et/ou le traitement de maladies tumorales.

18. Composés selon les revendications 1 à 7, en combinaison avec une ou plusieurs autres substances pharmacologiquement actives, destinés à une utilisation dans la prophylaxie et/ou le traitement d'infections virales, de maladies neurodégénératives, de maladies inflammatoires, de maladies athérosclérotiques et dans le contrôle de la fertilité masculine.

19. Composés de formule générale (VIII) 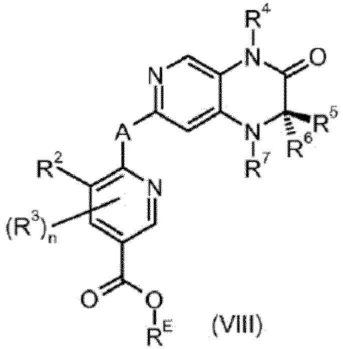 dans laquelle A, R², R³, R⁴, R⁵, R⁶, R⁷ et n ont les significations indiquées pour la formule générale (I) et R^{E} représente alkyle en C₁-C₆.

20. Composés de formule générale (IX) 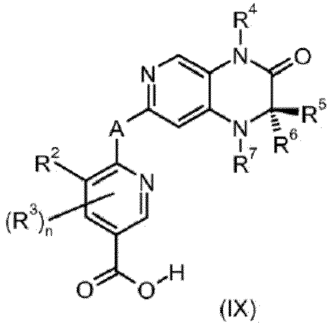 dans laquelle A, R², R³, R⁴, R⁵, R⁶, R⁷ et n ont les significations indiquées pour la formule générale (I).

21. Utilisation des composés de formule générale (VIII) 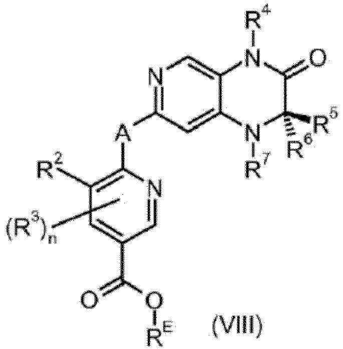 dans laquelle A, R², R³, R⁴, R⁵, R⁶, R⁷ et n ont les significations indiquées pour la formule générale (I) et R^{E} représente alkyle en C₁-C₆, pour la fabrication des composés de formule générale (I) selon l'invention.

22. Utilisation des composés de formule générale (IX) 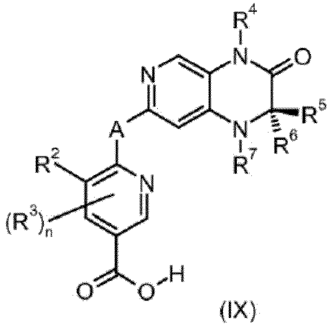 dans laquelle A, R², R³, R⁴, R⁵, R⁶, R⁷ et n ont les significations indiquées pour la formule générale (I), pour la fabrication des composés de formule générale (I) selon l'invention.


### SEQUENCE LISTING

<110> Bayer Pharma Aktiengesellschaft
<120> Substituierte Dihydropyrido[3,4-b]pyrazinone als duale Inhibitoren von BET-Proteinen und Polo-like Kinasen
<130> BHC123072-Sequence Listing
<150> EP13177539.7
   <151> 2013-07-23
<160> 3
<170> BiSSAP 1.3
<210> 1
   <211> 22
   <212> PRT
   <213> synthetic organisms
<220>
   <223> Synthetisches acetyliertes Histon H4 (Ac-H4) Peptid; acetyliert am Lysin in Position 4, 7, 11 und 15
<400> 1
   Gly Arg Gly Lys Gly Gly Lys Gly Leu Gly Lys Gly Gly Ala Lys Arg
   1               5                   10                  15
   His Gly Ser Gly Ser Lys
               20
<210> 2
   <211> 13
   <212> PRT
   <213> synthetic organisms
<220>
   <223> Biotinyliertes Peptid Btn-Ahx
<400> 2
   Lys Lys Leu Asn Arg Thr Leu Ser Phe Ala Glu Pro Gly
   1               5                   10
<210> 3
   <211> 13
   <212> PRT
   <213> synthetic organisms
<220>
   <223> Biotinyliertes Peptid Ttds
<400> 3
   Lys Lys Leu Asn Arg Thr Leu Ser Phe Ala Glu Pro Gly
   1               5                   10